# EUROPEAN PATENT APPLICATION

(11) **EP 4 431 102 A1**
(43) Date of publication of application: **18.09.2024**
(21) Application number: 22893206.7
(22) Date of filing: 09.11.2022
(51) Int. Cl.: A61K 31/714, A61K 31/095, A61K 31/28, A61K 31/5025, A61P 43/00, A61P 35/00, A61P 17/00, A61K 38/44, G01N 33/50

(54) **COMPOSITIONS FOR REMOVING SENESCENT CELLS AND USES THEREOF**

(30) Priority: 10.11.2021 KR 20210154244; 30.06.2022 KR 20220080942
(71) Applicant: Lifeceutix Co., Ltd., Suwon-si, Gyeonggi-do 16229 (KR)
(72) Inventor: KIM, Tae Kook, Suwon-si, Gyeonggi-do 16229 (KR); JANG, Hyung Ju, Suwon-si, Gyeonggi-do 16229 (KR); CHOO, Kang Sik, Suwon-si, Gyeonggi-do 16229 (KR)
(74) Representative: Høiberg P/S
(86) International application number: PCT/KR2022/017594
(87) International publication number: WO 2023/085787

(57) **Abstract**

The present disclosure provides a composition for removing senescent cells that is useful in senescent cell removal. The composition of the present invention induces the death of senescent cells and thus can be usefully applied to the amelioration, treatment, or prevention of various aging-associated diseases and disorders caused by senescent cells. In addition, the composition of the present invention can be usefully applied as pharmaceuticals, cosmetics, health functional foods, animal products, etc. in relation to aging-associated diseases and disorders caused by senescent cells.

## Description

### [Technical Field]

This application claims priorities based on Korean Patent Application No. 10-2021-0154244 filed on November 10, 2021 and Korean Patent Application No. 10-2022-0080942 filed on June 30, 2022. All contents disclosed in the specifications and drawings of the applications are incorporated into this application.

The present invention relates to a composition that has the effect of removing senescent cells. The composition of the present invention comprises as an active agent a compound having excellent ability of removing senescent cells or its pharmaceutically acceptable salt; secreted fluid from cells killed by treatment with such a compound or its pharmaceutically acceptable salt; or cells killed by treatment with such a compound or its pharmaceutically acceptable salt. The invention also relates to various uses, in particular medical uses of the compositions of the invention.

### [Background Art]

Aging refers to the process of gradual physical and cognitive decline as one ages, leading to death after humans are born and grow for a certain period of time. The World Health Organization (WHO) assigned a disease code (MG2A) to old age itself in 2018, and several research and development and clinical trials are being conducted to treat (e.g., slow down) aging.

In particular, the target of aging is being narrowed from 'individual' to 'cell' through various research and development results. Accordingly, it was revealed that `senescent cells' are the fundamental cause of various diseases and disorders related to aging. Senescent cells occur in the human body due to various factors such as aging, drug side effects, stress, obesity, diabetes, wounds, transplants, and infections. These senescent cells are removed by the body's immune cells. However, as immune cells do not function properly due to various reasons due to aging, or more fundamentally, immune cells also age together, they do not function properly and eventually senescent cells accumulate in the body.

Accumulated senescent cells secret a large number of senescence-promoting factors as well as senescence-associated pathogenic factors such as senescence-associated inflammation and senescence-associated fibrosis. The role of these senescence-associated secretory phenotypes (SASP) is known (i) to gradually spread senescent cells by changing surrounding normal cells into senescent cells, (ii) to change the structure and function in all organs and tissues, (iii) to cause various underlying pathological phenotypes such as chronic inflammation (immuno-aging) and fibro-aging due to aging, and (iv) accordingly, to ultimately cause various aging-related diseases and disorders throughout the body.

Accordingly, recently, just as cancer is treated by removing cancer cells, various research and development efforts are underway to treat aging-related diseases and disorders by removing senescent cells (Robbins PD et al., Annual Review of Pharmacological Toxicology 61, 779-803, 2021). For example, ABT-263/737 has been recently reported as an innovative anti-aging treatment, but many side effects such as toxicity exist. Therefore, it is important to develop new compounds and/or compositions that eliminate senescent cells through a mechanism different from ABT-263/737.

### [Disclosure]

### [Technical Problem]

Therefore, the problem to be solved by the present invention is to provide compositions useful for removing senescent cells and uses of such compositions for removing senescent cells.

Another problem to be solved by the present invention is to provide compositions that can improve, treat or prevent various diseases and disorders related to senescent cells by removing senescent cells via comprising the active agent according to the present invention. That is, another problem to be solved by the present invention is to provide uses, preferably medical uses, of the composition according to the present disclosure for improving, treating or preventing diseases and disorders related to senescent cells.

### [Technical Solution]

In order to achieve the above object, one aspect of the present invention provides compositions for removing senescent cells, comprising as active agent (a) the compound of Table 1 or 2 below or its pharmaceutically acceptable salt; (b) secreted fluid from cells killed by treatment with such compound or pharmaceutically acceptable salt thereof; or (c) cells killed by treatment with such compound or pharmaceutically acceptable salt thereof. That is, one aspect of the present invention provides uses, preferably medical uses, for removing senescent cells by treatment with the compound of Table 1 or 2 below or its pharmaceutically acceptable salt; secreted fluid from cells killed by treatment with such compound or pharmaceutically acceptable salt thereof; or cells killed by treatment with such compound or pharmaceutically acceptable salt thereof.

In one embodiment of the present invention, the compounds in Table 1 or 2 below have the effect of increasing the secretion of HMGN1 (High Mobility Group Nucleosome Binding Domain 1) protein (HMG14) in senescent cells.

**[Table 1]**

| Compound Name Compound No. | Structure, CAS Number, or IUPAC name | Compound Name Compound No. | Structure, CAS Number, or IUPAC name |
|---|---|---|---|
| Auranofin LX-201 | 34031-32-8 | Aurothiomalate LX-202 | 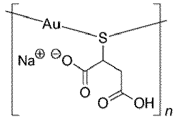 (e.g., Sodium aurothiomalate (12244-57-4)) |
| | 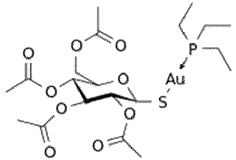 | | |
| TVB-2640 LX-305 | 1399177-37-7 | PX-12 LX-307 | 141400-58-0 |
| | 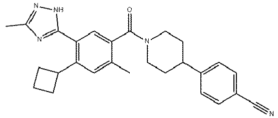 | | 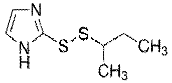 |
| MK-8245 LX-309 | 1030612-90-8 | | |
| | 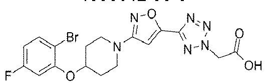 | | |
| ML210 (DPI10) LX-401 | 1360705-96-9 | ML162 (DPI7) LX-402 | 1035072-16-2 |
| | 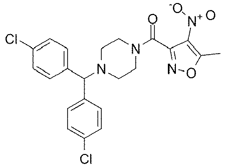 | | 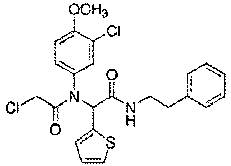 |
| JKE1674 LX-403 | 2421119-60-8 | RSL3 LX-405 | 1219810-16-8 |
| | 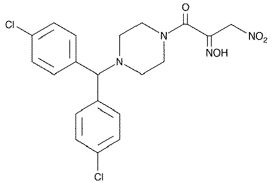 | | 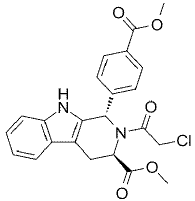 |
| LOC880 LX-406 | 139257-24-2 | QW-017 LX-407 | 1000510-69-9 |
| | 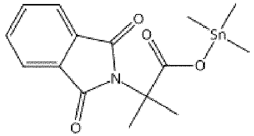 | | 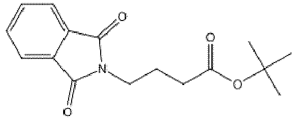 |
| Compound102 LX-411 | 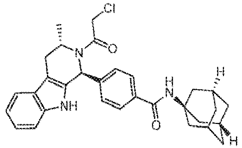 | Compound106 LX-412 | 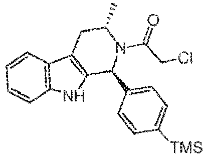 |
| Compound166 LX-413 | 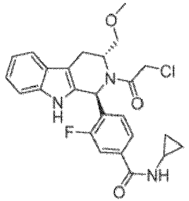 | Compound211 LX-415 | 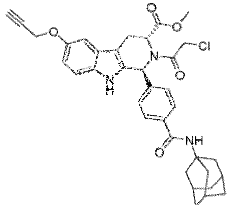 |
| Compound40 LX-416 | 2485004-42-8 | Compound41 LX-417 | 2485004-43-9 |
| | 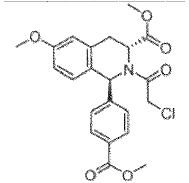 | | 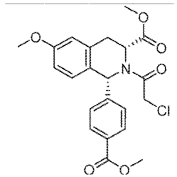 |
| CompoundA-13 LX-418 | 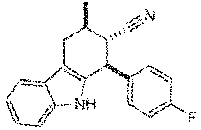 | | |
| CompoundA-3 LX-419 | 2617522-81-1 | CompoundB-1 LX-420 | 2617522-97-9 |
| | 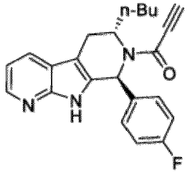 | | 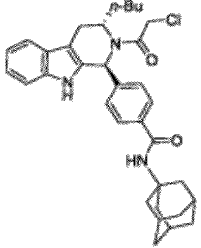 |
| CompoundB-62 LX-421 | 2762601-44-3 | CompoundA-2 LX-422 | 2617524-47-5 |
| | 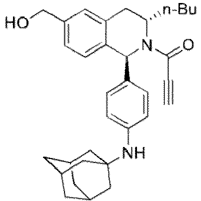 | | 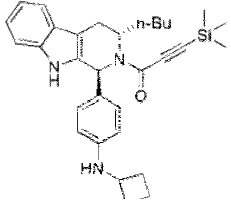 |
| Compound 1 LX-431 | 4-((2-(2-(4-((4-((4-chlorophenyl)(4-(5-methyl-4-nitroisooxazol-3-carbonyl)piperazin-1-yl)methyl)phenoxy)methyl)-1 H-1,2,3-triazol-1-yl)ethoxy)ethyl)amino)-2-(2,6-dioxopiperidin-3-yl)isoindolin-1,3-dione | Compound 2 LX-432 | 4-((2-(2-(2-(4-((4-((4-chlorophenyl)(4-(5-methyl-4-nitroisooxazol-3-carbonyl)piperazin-1-yl)methyl)phenoxy)methyl)-1H-1,2,3-triazol-1-yl)ethoxy)ethoxy)ethyl)amino)-2-(2,6-dioxopiperidin-3-yl)isoindolin-1,3-dione |
| Compound 3 LX-435 | 4-((2-(2-(2-(4-((4-((4-chlorophenyl)(4-(5-methyl-4-nitroisooxazol-3-carbonyl)piperazin-1-yl)methyl)phenoxy)methyl)-1H-1,2,3-triazol-1-yl)ethoxy)ethoxy)ethyl)amino)-2-(2,6-dioxopiperidin-3-yl)isoindolin-1,3-dione | Compound 4 LX-436 | 4-((14-(4-((4-((4-chlorophenyl)(4-(5-methyl-4-nitroisooxazol-3-carbonyl)piperazin-1-yl)methyl)phenoxy)methyl)-1H-1,2,3-triazol-1-yl)-3,6,9,12-tetraoxatetradecyl)amino)-2-(2,6-dioxopiperidin-3-yl)isoindolin-1,3-dione |
| Compound 5 LX-437 | 4-((2-(4-((4-((4-chlorophenyl)(4-(5-methyl-4-nitroisooxazol-3-carbonyl)piperazin-1-yl)methyl)phenoxy)methyl)-1H-1,2,3-triazol-1-yl)ethyl)amino)-2-(2,6-dioxopiperidin-3-yl)isoindolin-1,3-dione | | |
| Altretamine LX-501 | 645-05-6 | BP-1007 LX-502 | 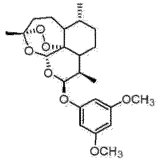 |
| | 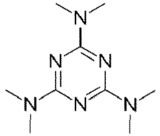 | | |
| BP-10131 LX-503 | 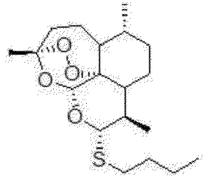 | BP-1001 LX-505 | 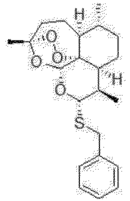 |
| Artemisinin LX-506 | 63968-64-9 | DMOCPTL LX-601 | 2289585-58-4 |
| | | | 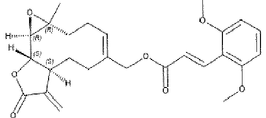 |
| FIN56 LX-602 | 1083162-61-1 | FINO2 LX-603 | 869998-31-7 |
| | 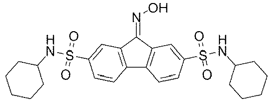 | | 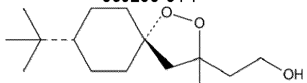 |
| Eprenetapopt (APR-246) LX-701 | 5291-32-7 | 2-Methylene-3-quinuclidinone LX-702 | 207556-03-4 |
| | 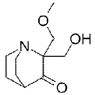 | | 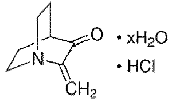 |
| COTI-2 LX-801 | 1039455-84-9 | COTI-NMe2 LX-802 | 2-(6,7-Dihydroquinolin-8(5H)-ylidene)-*N*⁴,*N*⁴-dimethylhydrazine-1-carbothioamide |
| | | | 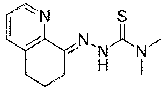 |
| PhiKan083 LX-901 | 880813-36-5 (free base) | PhiKan059 LX-902 | 1009675-01-7 |
| | 1050480-30-2 (HCl salt) | | |
| | 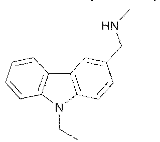 | | 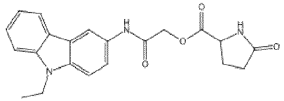 |
| PhiKan9318 LX-903 | 2413985-38-1 | PC14586 LX-905 | 2649340-85-0 |
| | 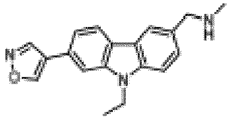 | | 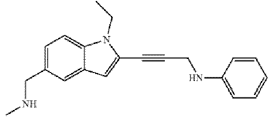 |
| PK7088 (Compound2) LX-907 | 1446352-67-5 | PK7242 LX-909 | 1446352-68-6 |
| | 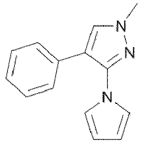 | | 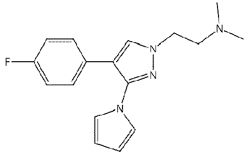 |
| PK5196 (Compounds) LX-910 | 1370554-91-8 | MB710 (Compound70) LX-911 | 2230044-57-0 |
| | 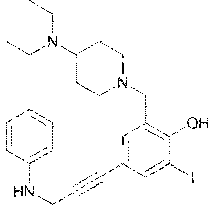 | | 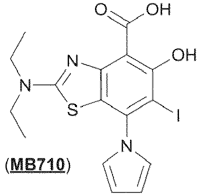 |
| MB725 (Compound72) LX-912 | 2230058-99-6 | PhiKan7242 (Compound7) LX-913 | 1446352-68-6 |
| | 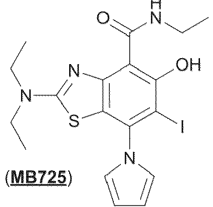 | | 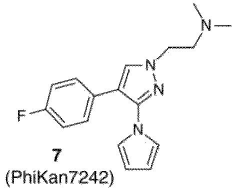 |
| Compound 231B LX-915 | 2746371-35-5 | | |
| PK11007 LX-916 | 874146-69-7 | PK11000 LX-917 | 38275-34-2 |
| NSC 350629 LX-918 | [3,4,5-triacetyloxy-6-[4-[2-[(dimethylamino)methyl]prop-2-enoyl]phenoxy]oxan-2-yl]methyl acetate | NSC 382006 LX-919 | 116218-47-4 |
| | | | 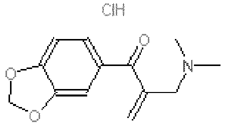 |
| NSC 708045 LX-920 | 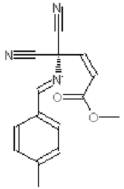 | NSC 382001 LX-921 | 116218-50-9 |
| | | | 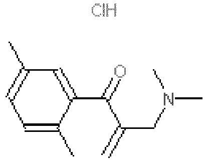 |
| NSC 382000 LX-923 | 116218-49-6 | | |
| | 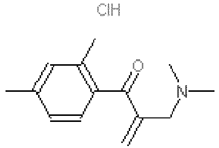 | | |
| Erastin2 LX-1001 | 1695533-44-8 | Erastin | 571203-78-6 |
| | 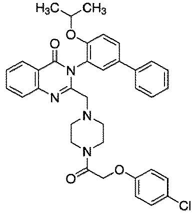 | | |
| Piperazine erastin | 1538593-71-3 | Imidazole ketone erastin | 1801530-11-9 |
| PRLX 93936 LX-1002 | 903499-49-0 | Erastin B | 340144-42-5 |
| | 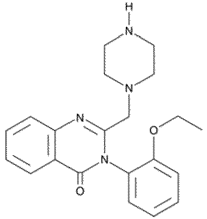 | | 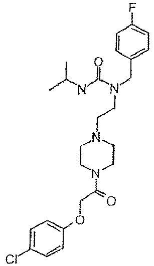 |
| iFSP1 LX-1101 | 150651-39-1 | Siramesine LX-1102 | 147817-50-3 |
| | 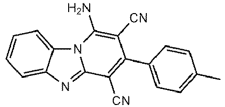 | | 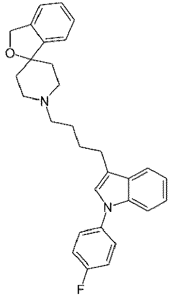 |
| 5-Aminolevulinic acid LX-1103 | 106-60-5 | | |
| Hexadecyltriphenylp hosphonium LX-1201 | 14866-43-4 | Decyltriphenylpho sphonium LX-1202 | 32339-43-8 |
| | 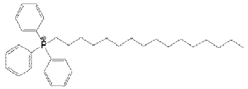 | | 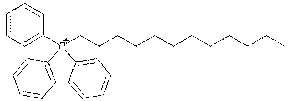 |
| Mitoquinone (MitoQ) LX-1203 | 845959-50-4 | SkQ1 LX-1205 | 934826-68-3 |
| MitoCDNB LX-1206 | [4-(5'-Chloro-2',4'-dinitrophenylamino)but-1-yl]triphenylphosphonium methanosulfonate | Mito-LND (Mito-Lonidamine) LX-1207 | 2361564-49-8 |
| | 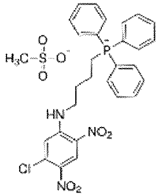 | | |
| Mitotane LX-1301 | 53-19-0 | Sulfasalazine LX-1302 | 599-79-1 |
| | 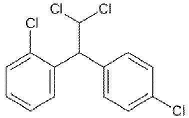 | | |
| Eperisone LX-1401 | 64840-90-0 | Lanperisone LX-1402 | 116287-14-0 |
| Tolperisone LX-1403 | 3644-61-9 | Inaperisone LX-1405 | 99323-21-4 |
| Silperisone LX-1407 | 140944-30-5 | Disulfiram LX-1601 | 97-77-8 |
| Brequinar LX-1602 | 96187-53-0 | Leflunomide LX-1603 | 75706-12-6 |
| ME-344 LX-1605 | 1374524-55-6 | Lapatinib LX-1607 | 231277-92-2 |
| | 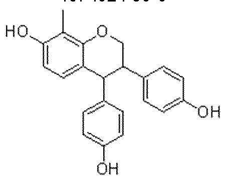 | | 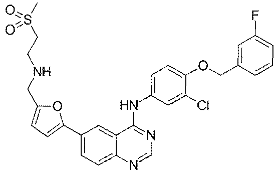 |
| Neratinib LX-1608 | 698387-09-6 | | |
| Ferumoxytol (Iron oxide(II,III)) LX-1701 | Nanoparticles synthesized according to https://www.nature.com/articles/s41565 -019-0406-1 | Cornell dot (C/C' dot) LX-1703 | Nanoparticles synthesized according to https://www.nature.com/articles/nnan o.2016.164 |
| Ferroptocide LX-1801 | (2*S*,3a*S*,4*S*,5*R*,7*R*,8*S*,8a*S*,11*R*)-7-(1,3-Dioxo-2-phenyl-2,3,5,8-tetrahydro-1*H-*[1,2,4]triazolo[1,2-a]pyridazin-6-yl)-2,3a-dihydroxy-4,8,11-trimethyl-3-oxooctahydro-1*H*-4,8a-propanoazulen-5-yl 2-Chloroacetate | VLX600 LX-1901 | 327031-55-0 |
| | 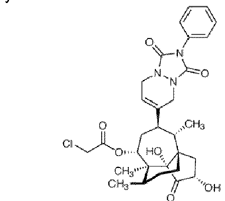 | | 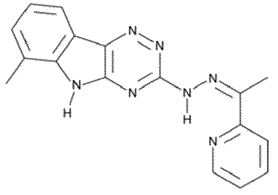 |
| Pralsetinib LX-1902 | 2097132-94-8 | Erdafitinib LX-1903 | 1346242-81-6 |
| Nelfinavir LX-1905 | 159989-64-7 | | |
| Ursolic acid LX-2001 | 77-52-1 | Corosolic acid LX-2002 | 4547-24-4 |
| Oleanolic acid LX-2003 | 508-02-1 | Betulinic acid LX-2005 | 472-15-1 |
| Moronic acid LX-2007 | 6713-27-5 | Momordin | 96990-18-0 |
| Omaveloxolone (RTA-408) LX-2101 | 1474034-05-3 | Bardoxolone methyl (RTA-402, CDDO-Me) LX-2102 | 218600-53-4 |
| TX63682 LX-2201 | 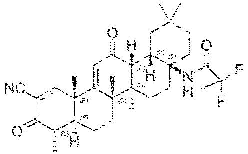 | | |
| 63101 (dh402, 402-02) LX-2301 | 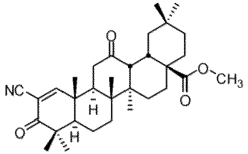 | 63102 (dh404, 404-02) LX-2302 | 1191265-33-4 Dihydro-CDDO-Trifluoroethyl Amide |
| | | | 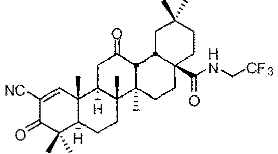 |
| 63167 (402-12) LX-2305 | 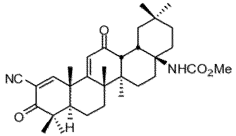 | 63175 (402-19) LX-2306 | 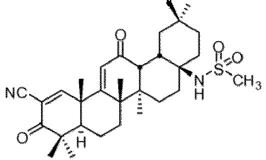 |
| 63194 (402-50) LX-2307 | 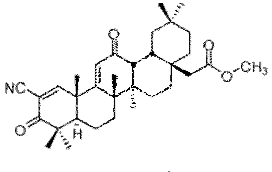 | 63311 LX-2308 | 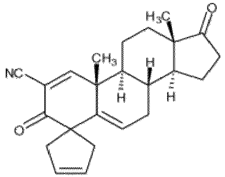 |
| 63165 (402-09) LX-2309 | 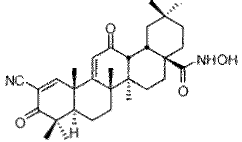 | 63166 (402-10) LX-2310 | 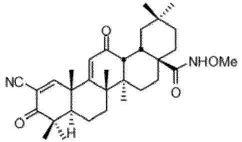 |
| TP-321 LX-2311 | 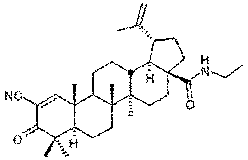 | | |
| TX63541 LX-2312 | 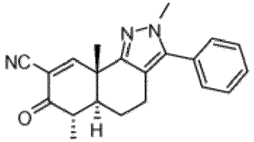 | TX63724 LX-2315 | 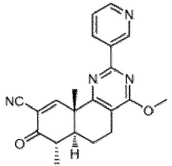 |
| TX63435 LX-2316 | 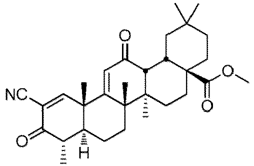 | TX63545 LX-2317 | 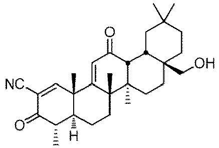 |
| TX63537 LX-2318 | 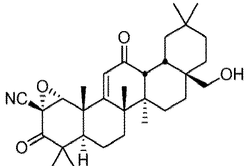 | TX63946 LX-2319 | 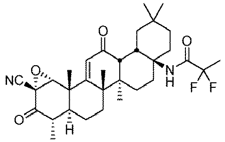 |
| TX63501 LX-2401 | 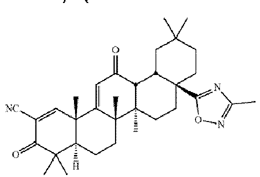 | | |
| TX63768 LX-2402 | 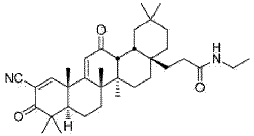 | TX63770 LX-2403 | 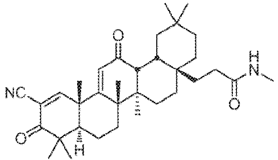 |
| TX63490 LX-2405 | | TX63530 LX-2406 | |
| LX-2407 | (5aS,6S,9aR)-1,6-dimethyl-7-oxo-9a-phenyl-3-(pyridin-3-yl)-4,5,5a,6,7,9a-hexahydro-1H-benzo[g]indazole-8-carbonitrile | LX-2408 | (5aS,6S,9aR)-1,6-dimethyl-7-oxo-9a-phenyl-3-(pyrimidin-5-yl)-4,5,5a,6,7,9a-hexahydro-H-benzo[g]indazole-8-carbonitrile) |
| T12 LX-2409 | 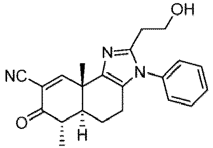 | T48 LX-2501 | 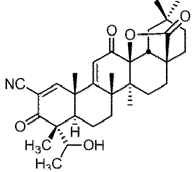 |
| T158 LX-2502 | 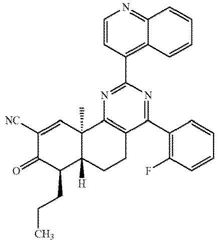 | T159 LX-2503 | 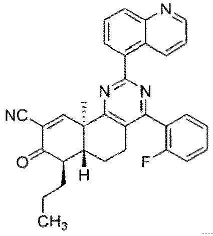 |
| T45 LX-2505 | 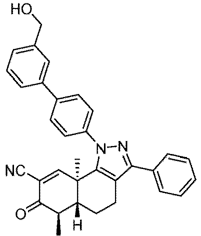 | T83 LX-2506 | 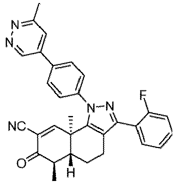 |
| T38 LX-2507 | 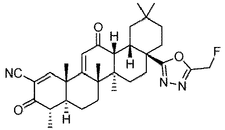 | T42 LX-2508 | 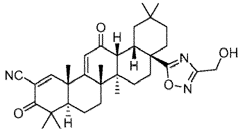 |
| T16 LX-2509 | 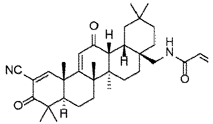 | T53 LX-2510 | 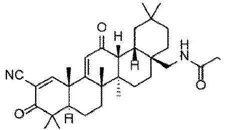 |
| T5 LX-2511 | 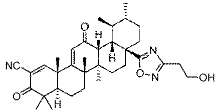 | T13 LX-2512 | 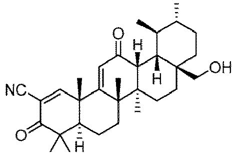 |
| Soloxolone methyl LX-2513 | 1032131-00-2 | Soloxolone tryptamide LX-2517 | 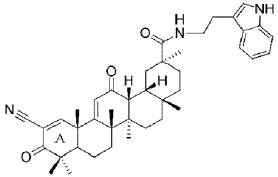 |
| | 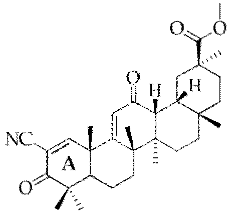 | | |
| ATN-224 LX-2601 | 649749-10-0 | Ezatiostat LX-2602 | 168682-53-9 |
| Ethacrynic acid LX-2603 | 58-54-8 | Polyunsaturated fatty acid | e.g. Arachidonic acid (LX-2701) (506-32-1), Docosahexaenoic acid (LX-2702) (6217-54-5), Linoleic acid (LX-2703) (60-33-3), Dihomogamma-linolenic acid (LX-2705) (1783-84-2), Punicic acid (LX-2707) (544-72-9), Eicosapentaenoic acid (10417-94-4) |
| Lercanidipine LX-2801 | 100427-26-7 | Atovaquone LX-2802 | 95233-18-4 |
| Lenvatinib LX-2803 | 417716-92-8 | Buthionine sulfoximine LX-2805 | 83730-53-4 |
| PACMA 31 LX-2807 | 1401089-31-3 | NOV-002 LX-2809 | 27025-41-8 |
| Methotrexate LX-2901 | 59-05-2 | Poseltinib LX-2902 | 1353552-97-2 |
| Tocofersolan (TPGS) LX-3001 | 9002-96-4 | APATONE^{®} (Vit C+Vit K3) LX-3002 | 50-81-7 + 58-27-5 |
| Clomipramine LX-3101 | 303-49-1 | Cyst(e)inase LX-3102 | Protein synthesized according to https://pubmed.ncbi.nlm.nih.gov/2786 9804/ |
| Asparaginase LX-3103 | 9015-68-3 | Methylseleninic acid LX-3105 | 28274-57-9 |
| Opaganib (ABC294640) LX-3106 | 915385-81-8 | INCB01158 (CB-1158) LX-3107 | 2095732-06-0 |
| CPI-613 (Devimistat) LX-3109 | 95809-78-2 | IACS-010759 LX-3200 | 1570496-34-2 |
| Sirpiglenastat (DRP-104) LX-3201 | 2079939-05-0 | AZD3965 LX-3203 | 1448671-31-5 |
| SM-88 (Racemetyrosine) LX-3206 | 658-48-0 | Lenalidomide LX-3207 | 191732-72-6 |
| Ibrutinib LX-3209 | 936563-96-1 | Pazopanib LX-3301 | 444731-52-6 |
| Vandetanib LX-3302 | 443913-73-3 | Gilteritinib (ASP2215) LX-3303 | 1254053-43-4 |
| Midostaurin LX-3307 | 120685-11-2 | Zanubrutinib LX-3309 | 1691249-45-2 |
| IFNgamma (Interferon gamma) LX-3401 | 98059-61-1 (IFNγ-1b) | CTLA4 inhibitor | |
| | 82115-62-6 (IFNγ) | | |
| PD-1 inhibitor LX-3403 | | PD-L1/2 inhibitor | |

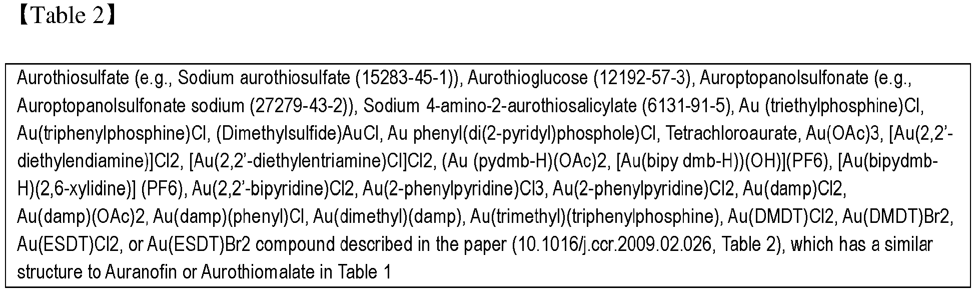

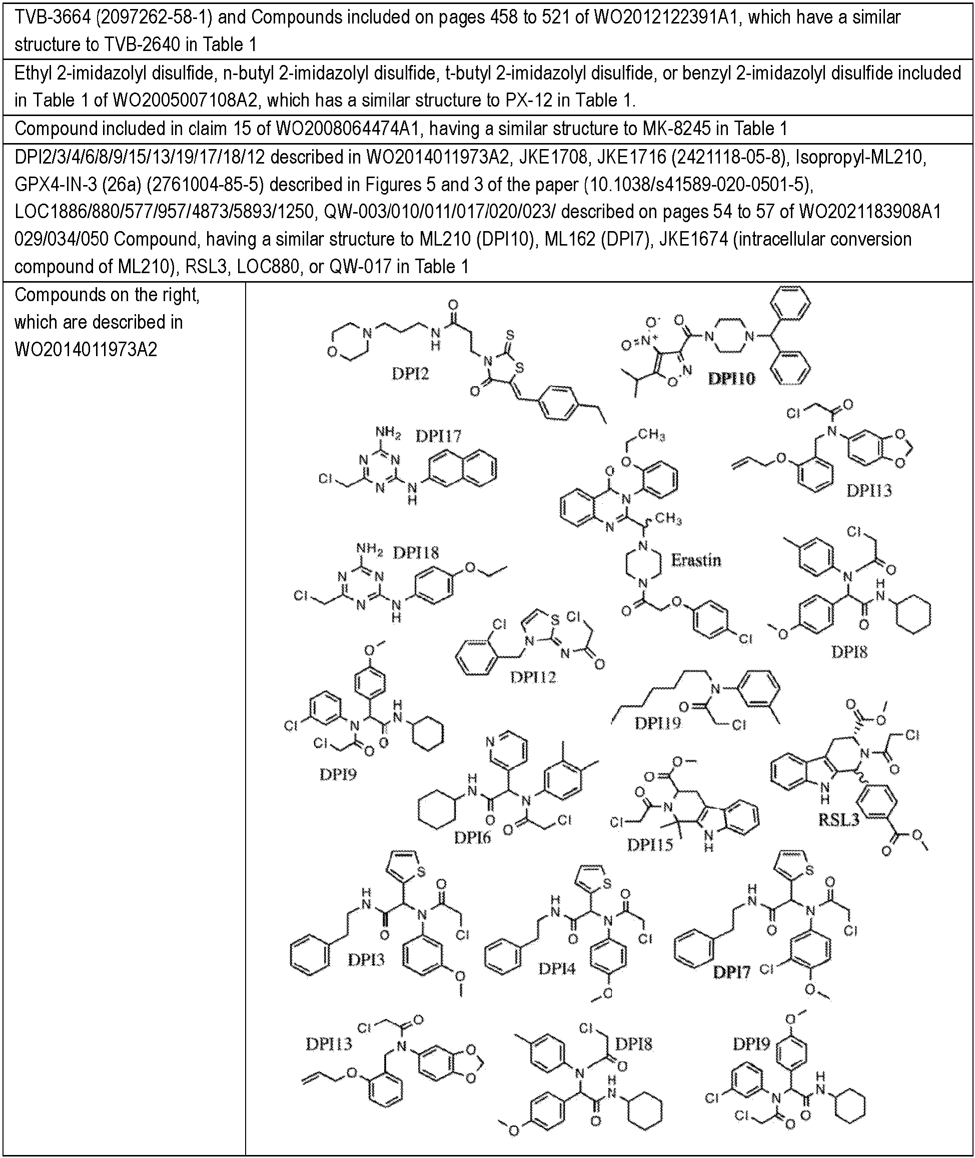

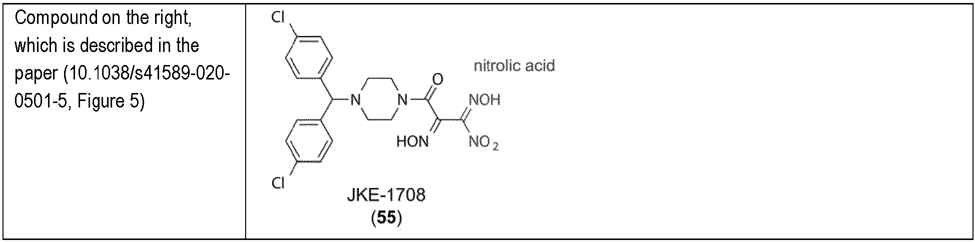

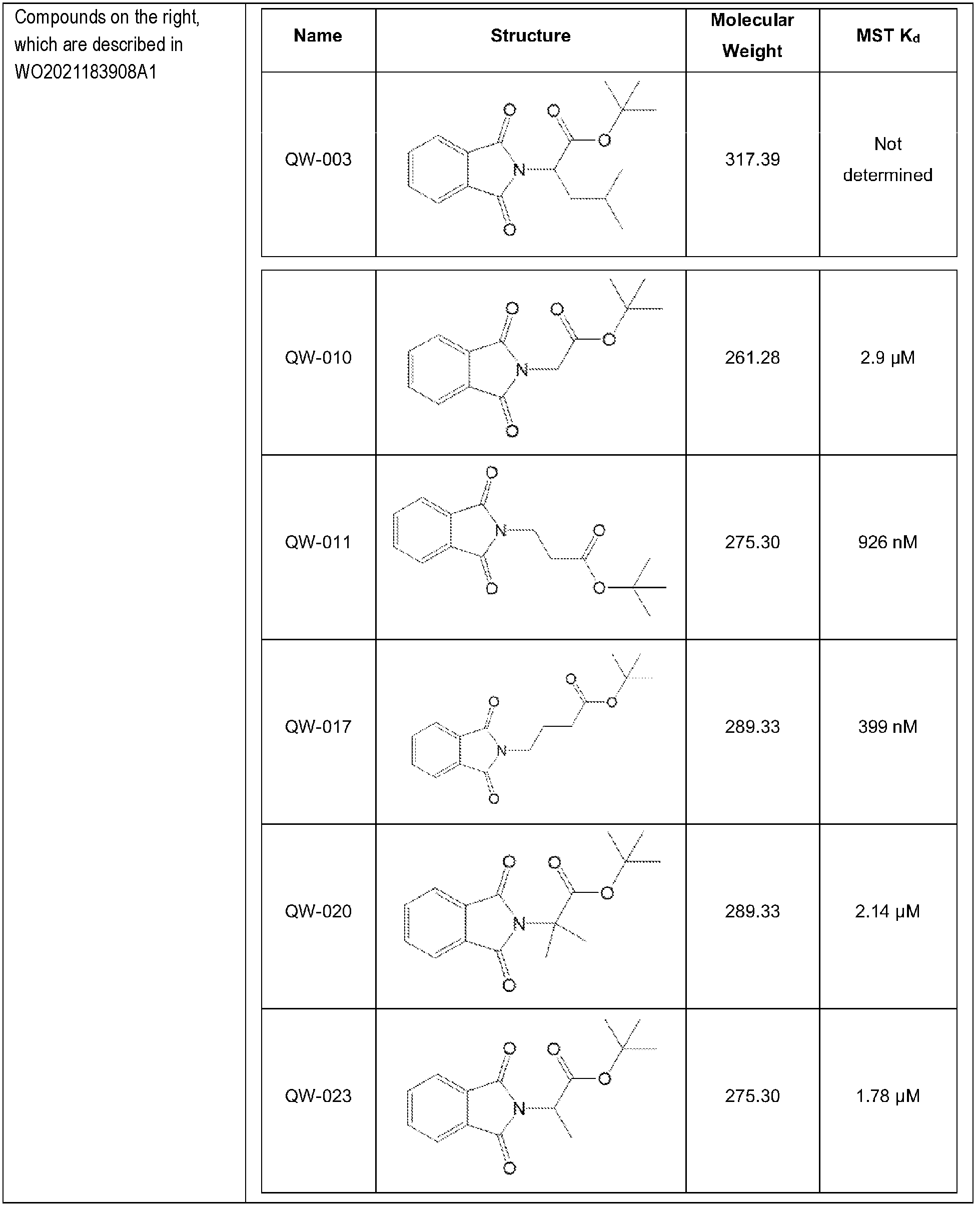

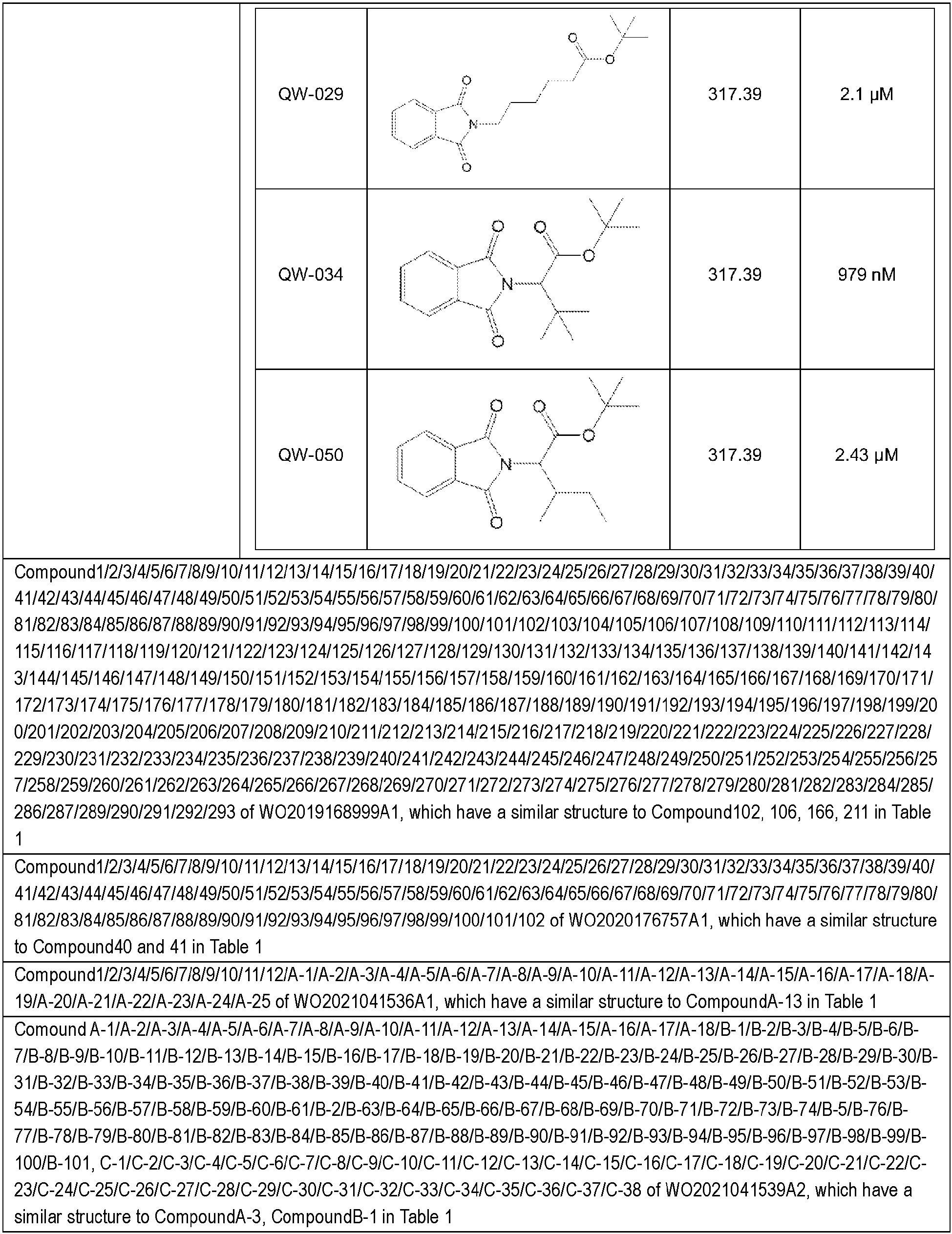

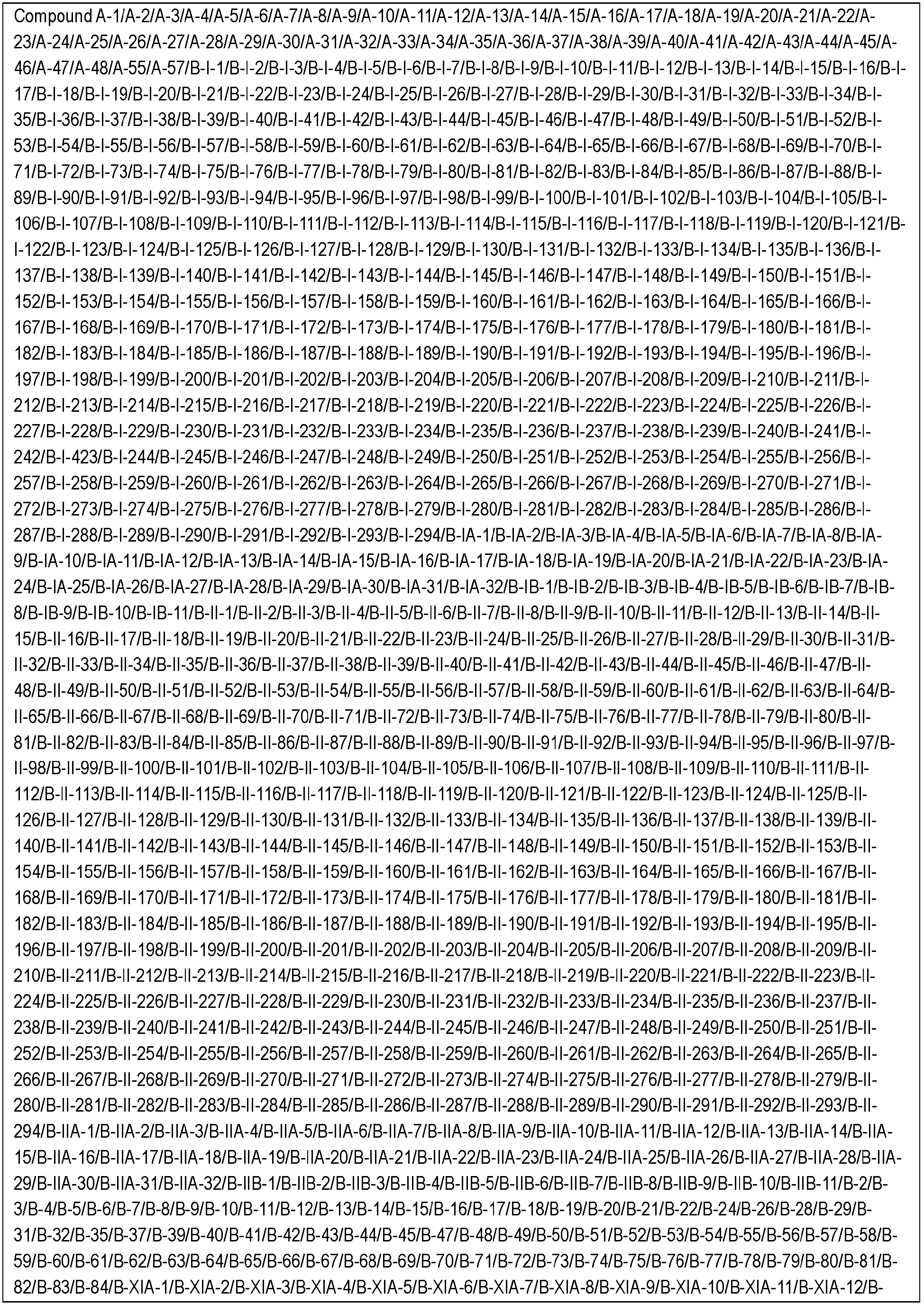

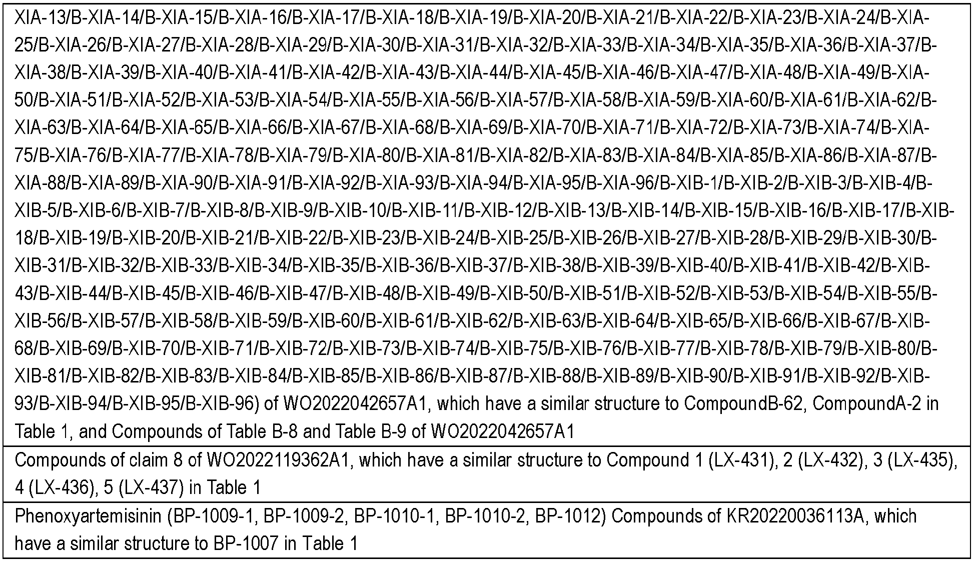

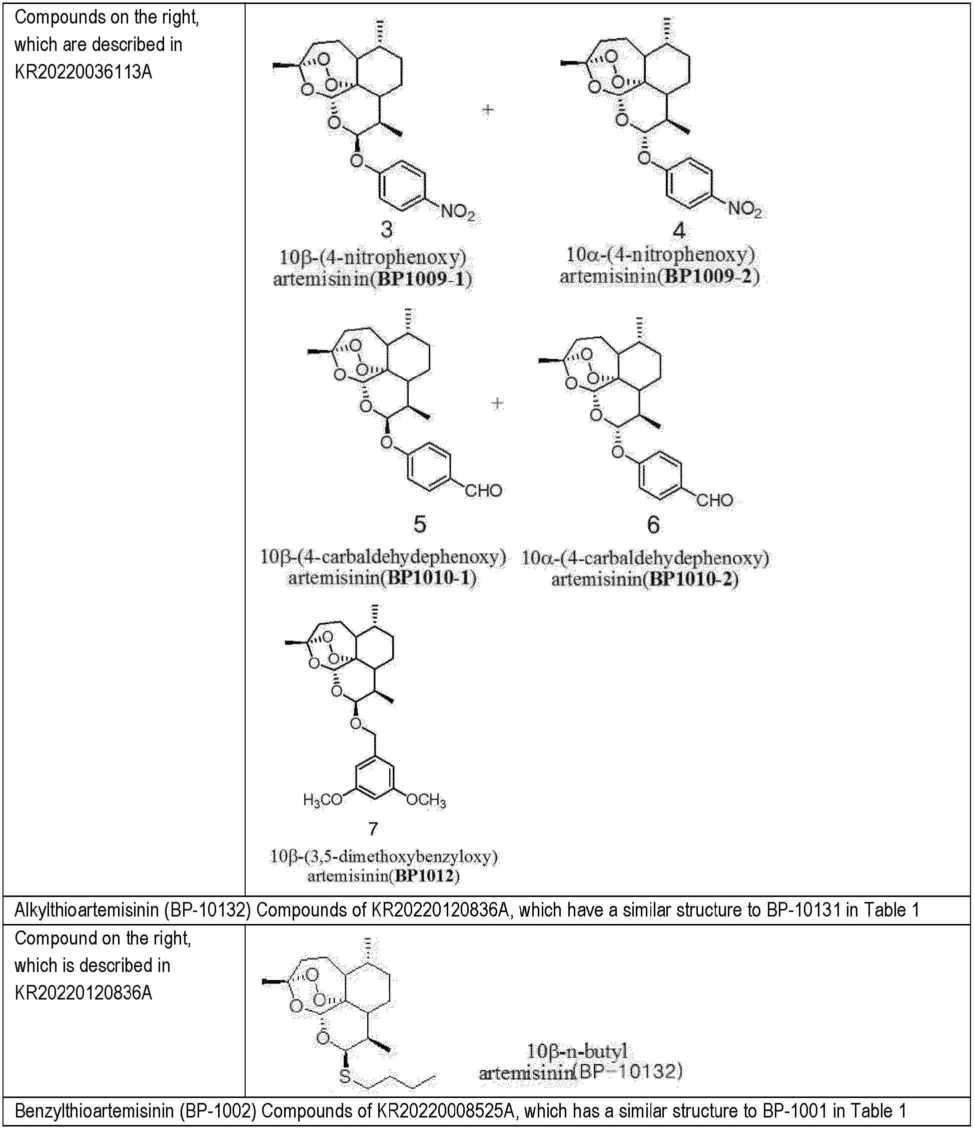

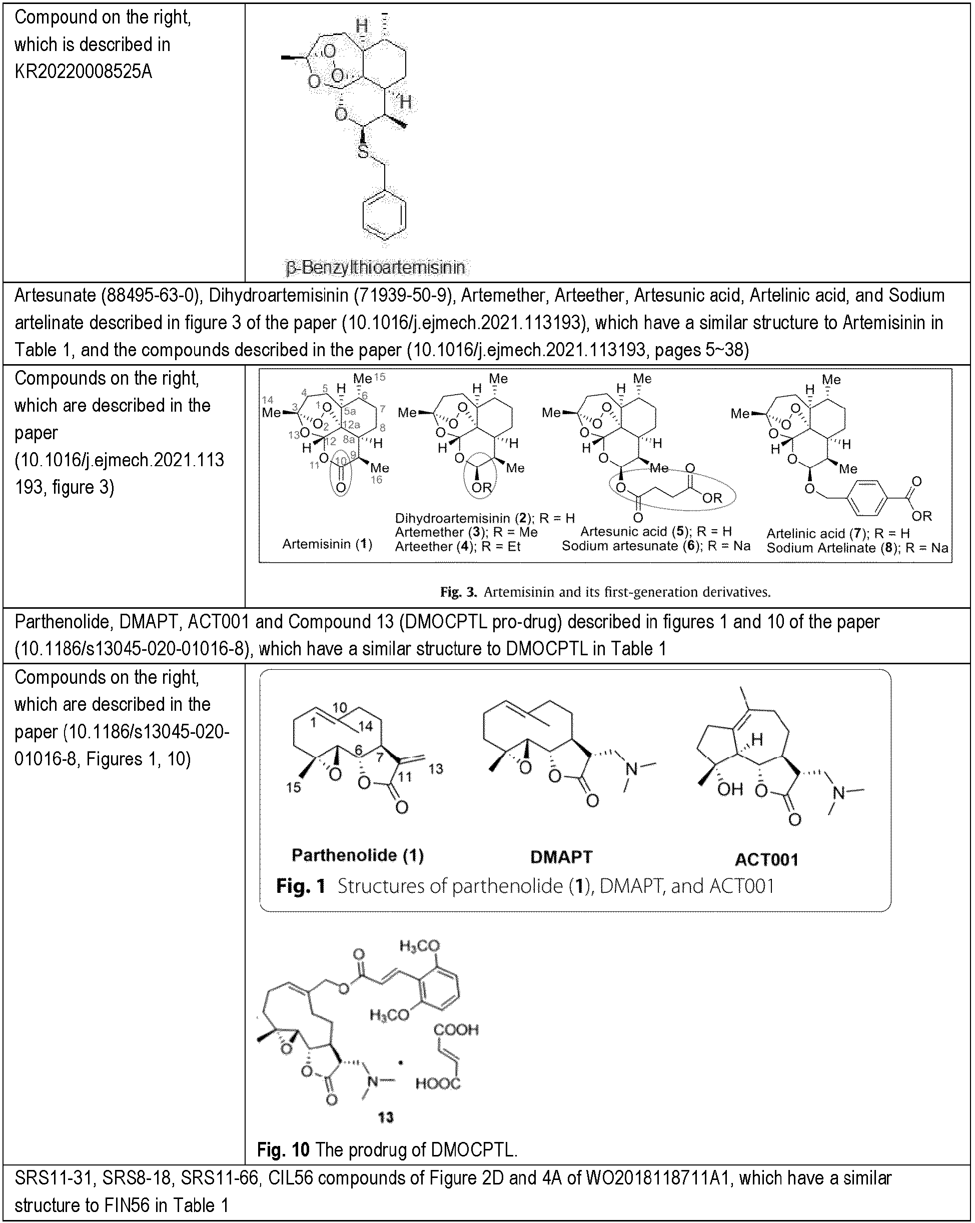

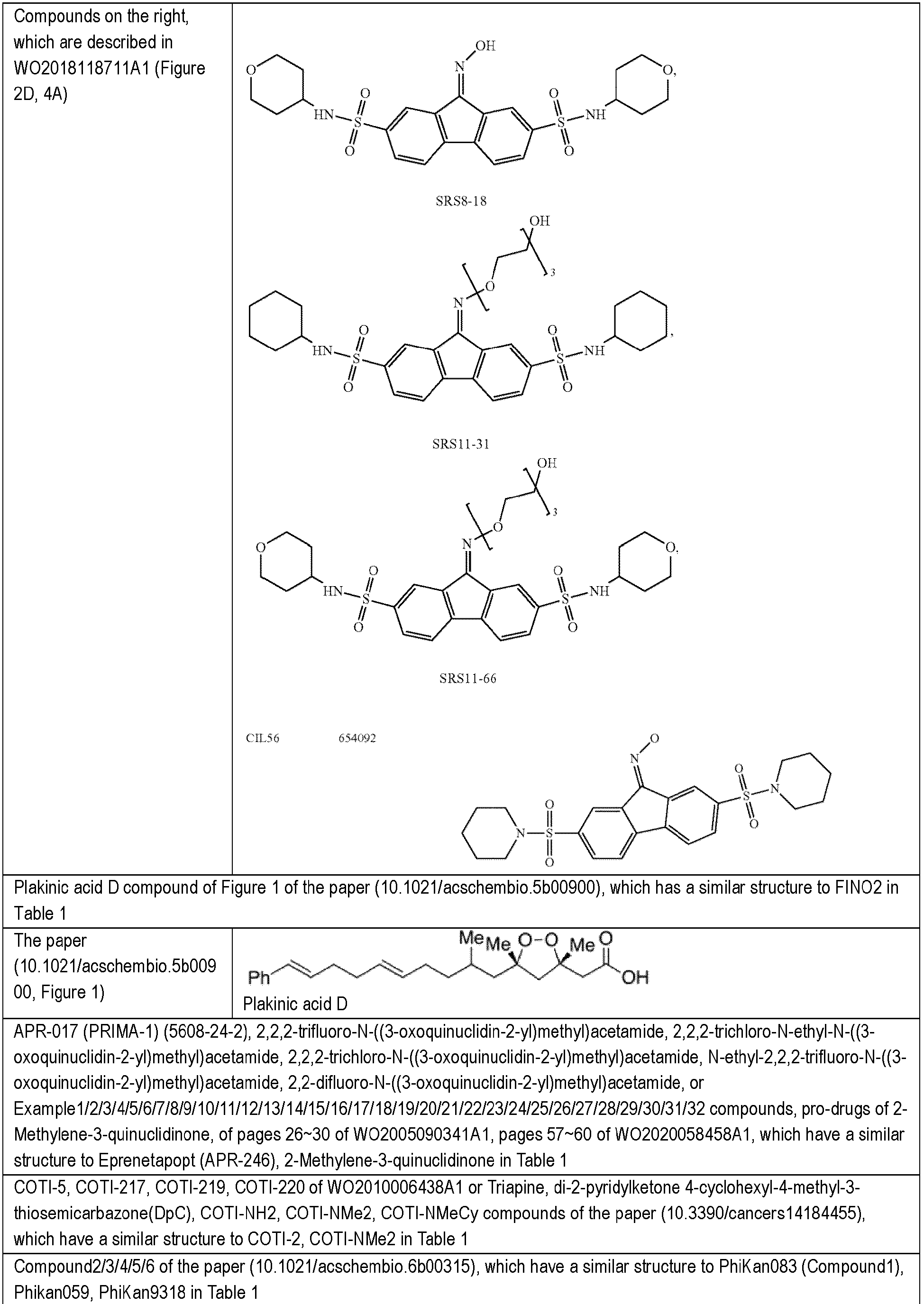

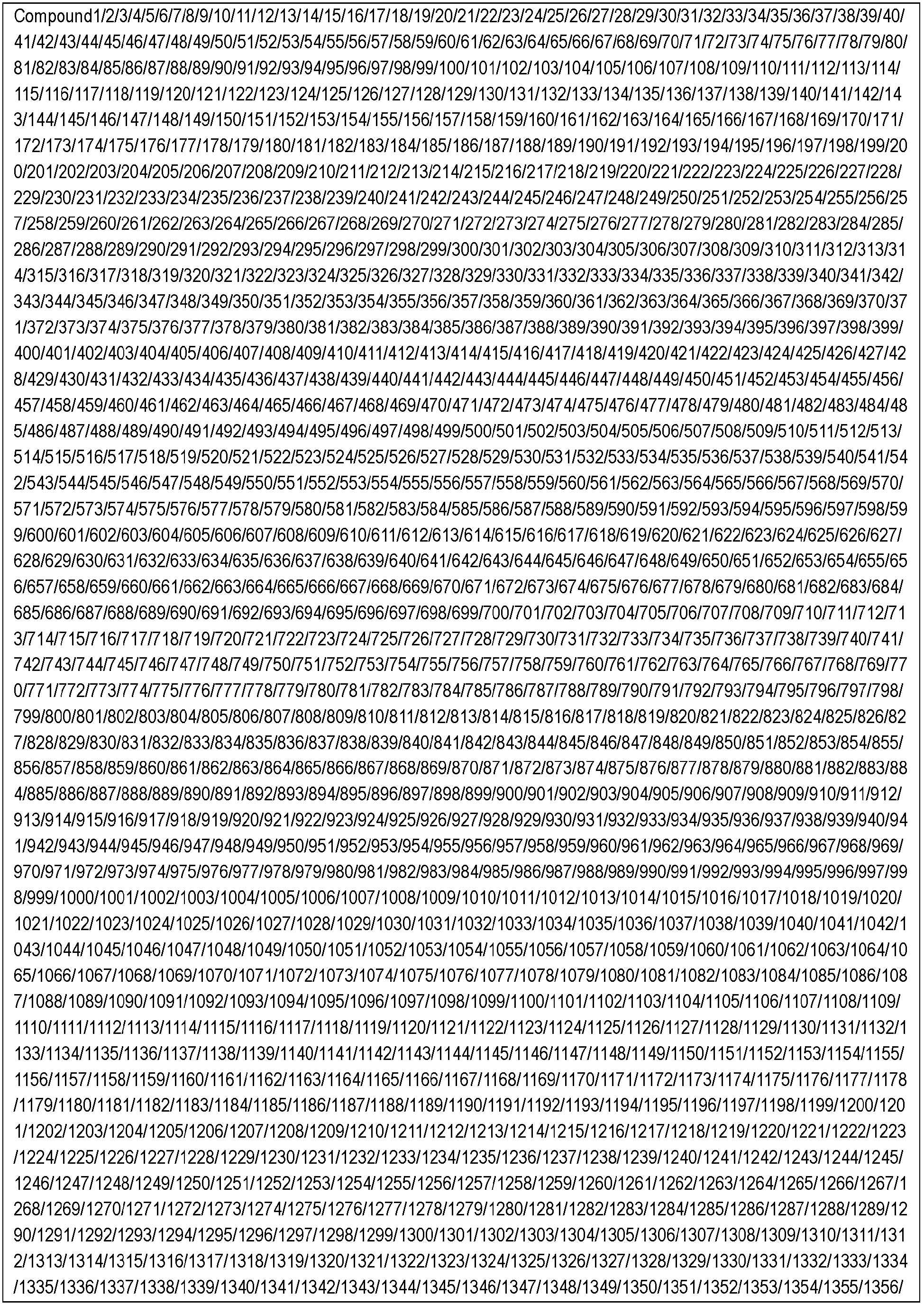

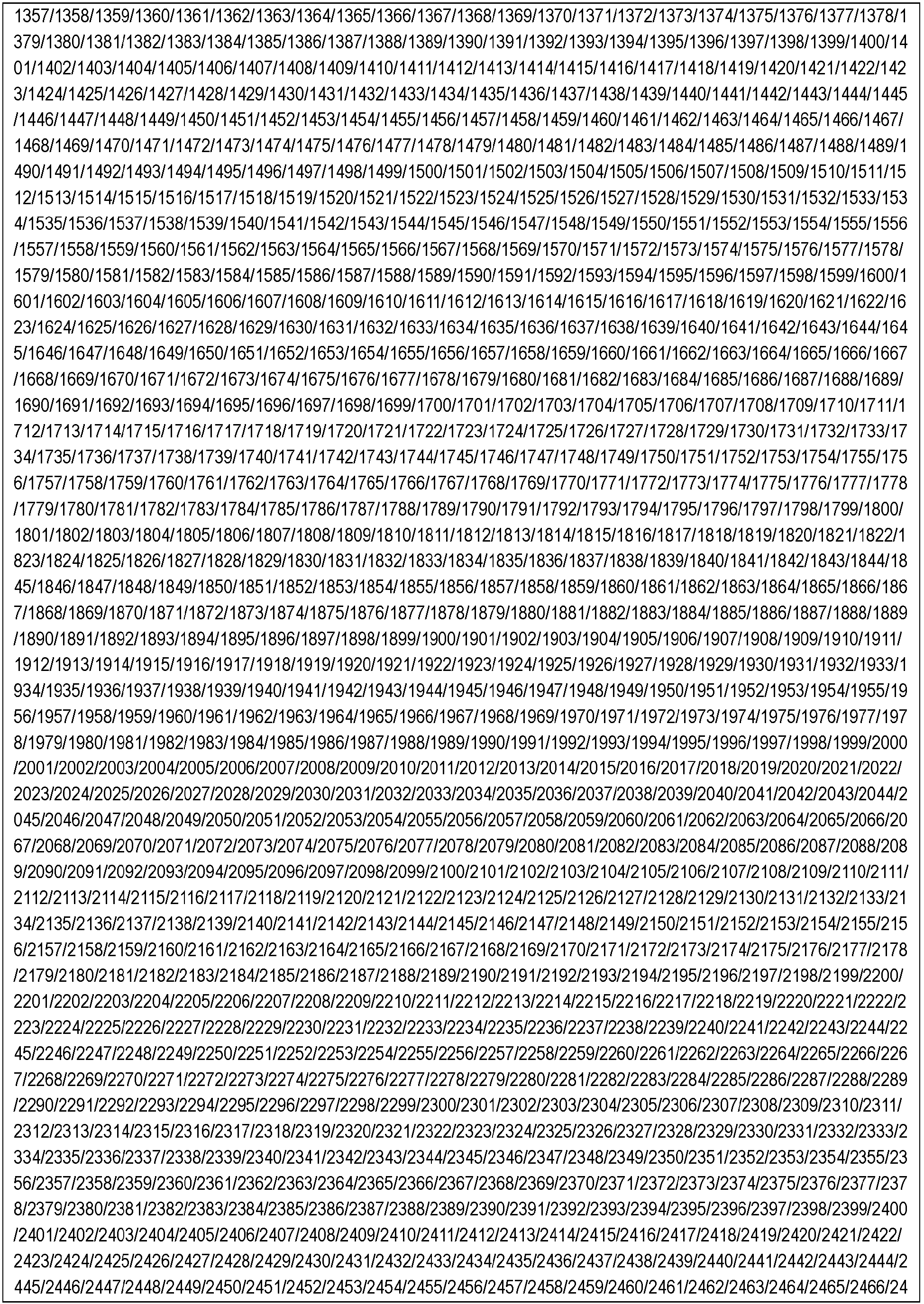

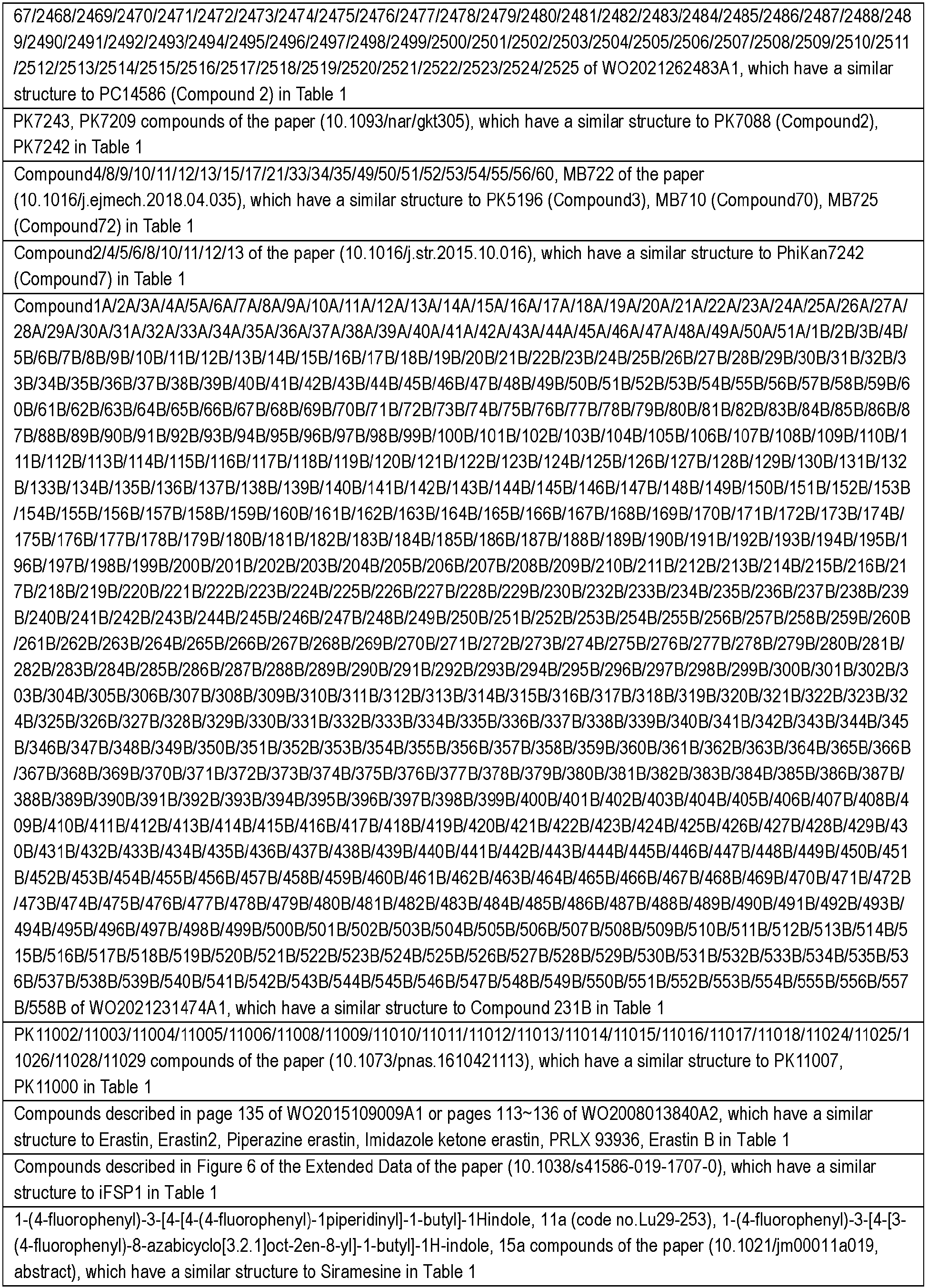

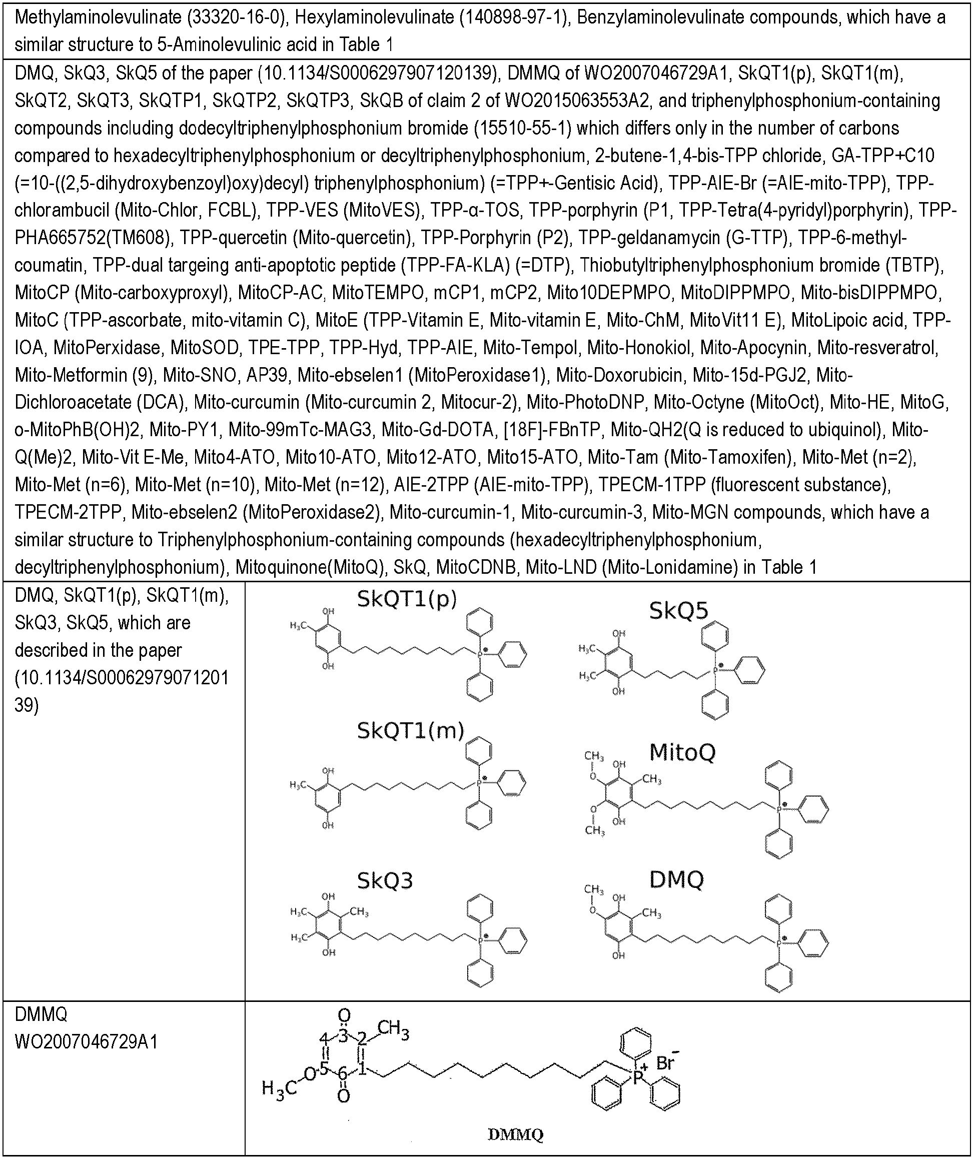

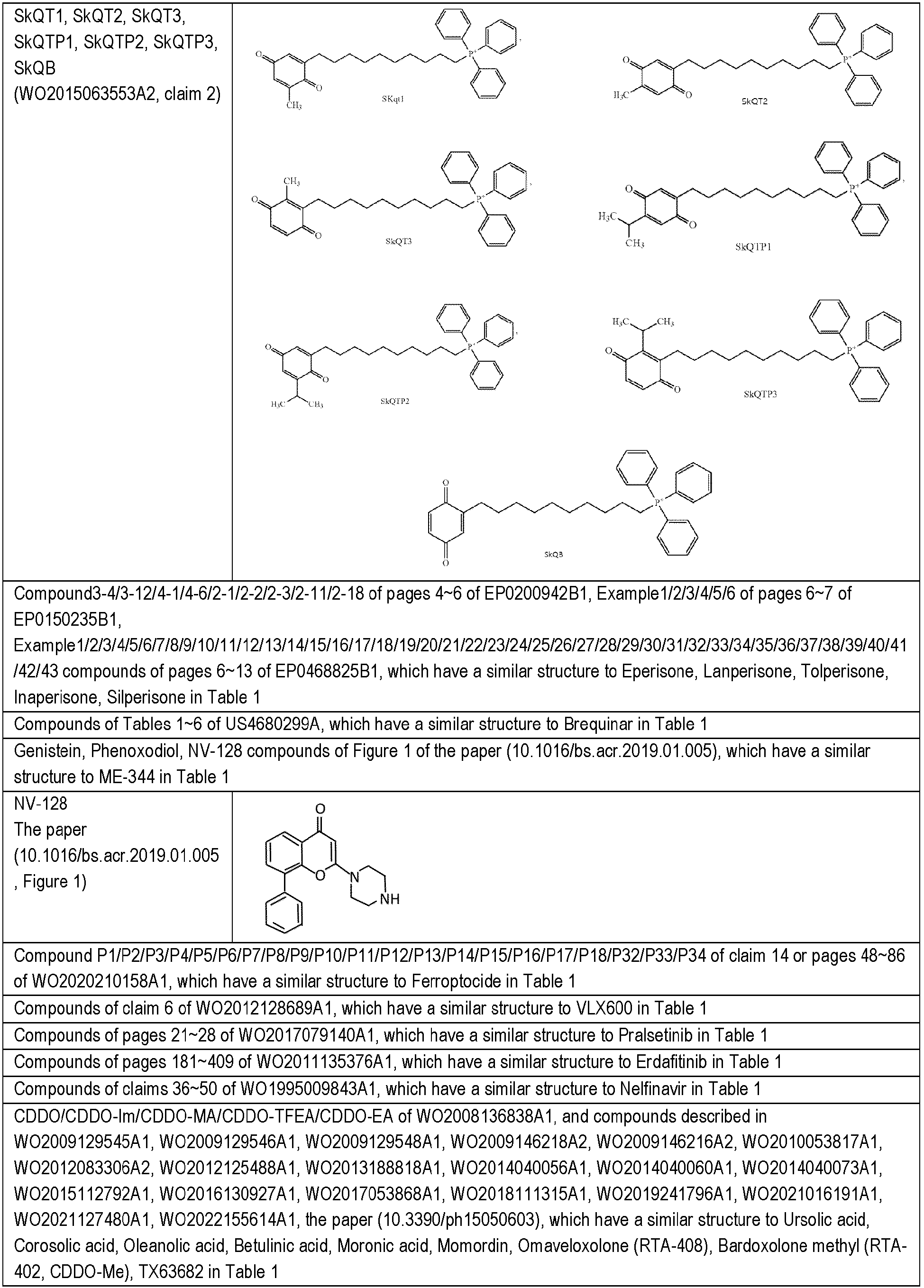

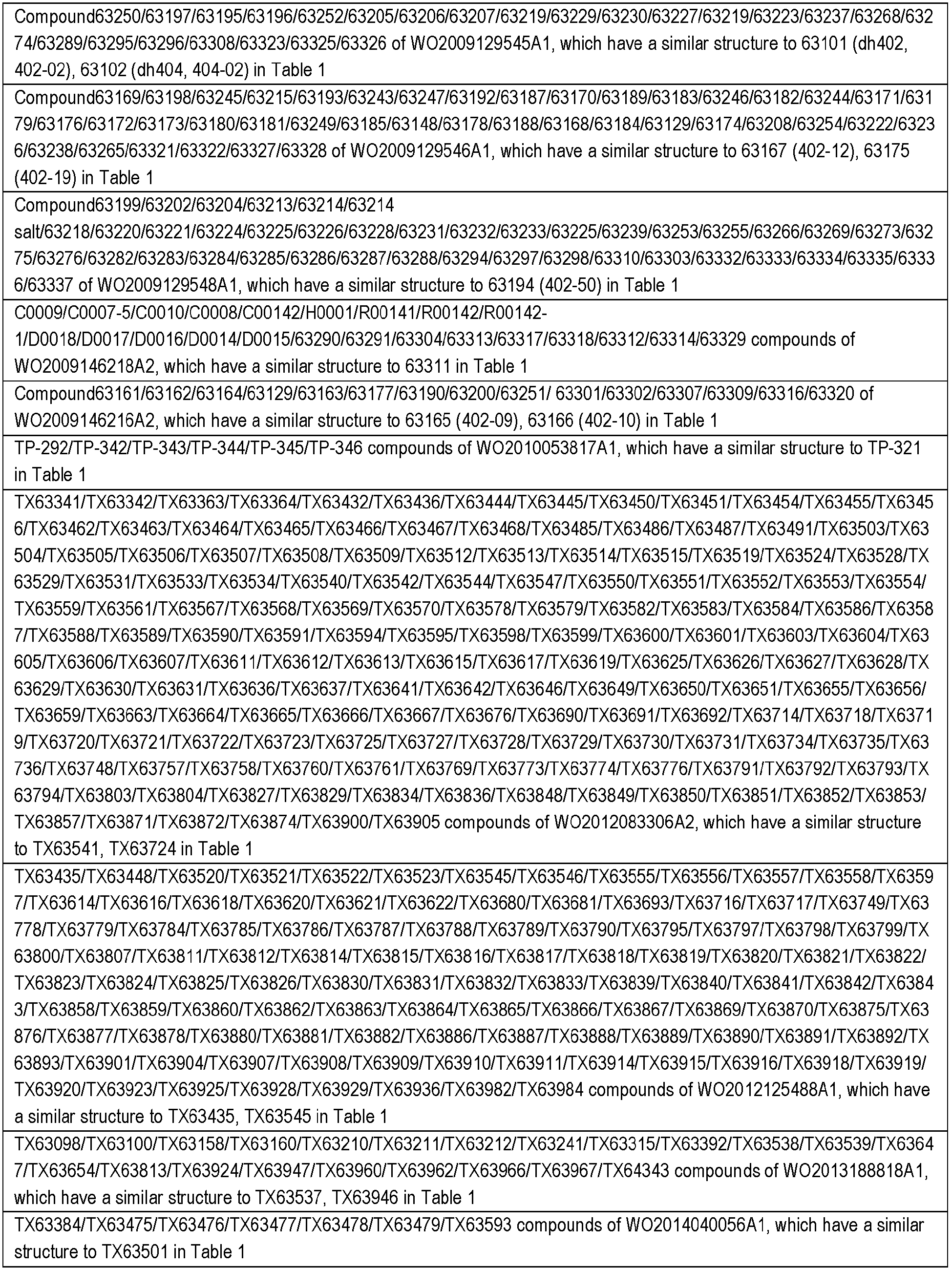

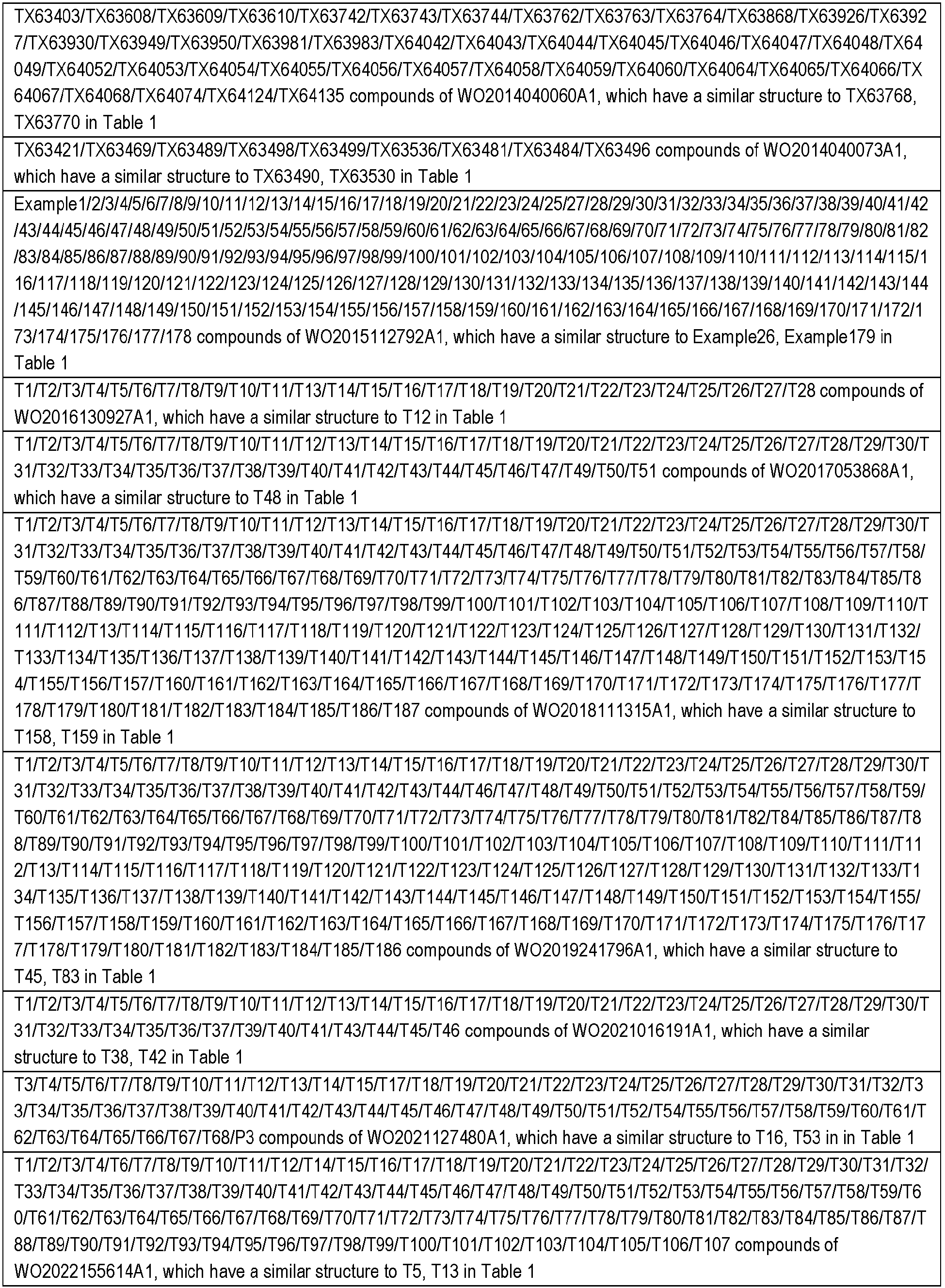

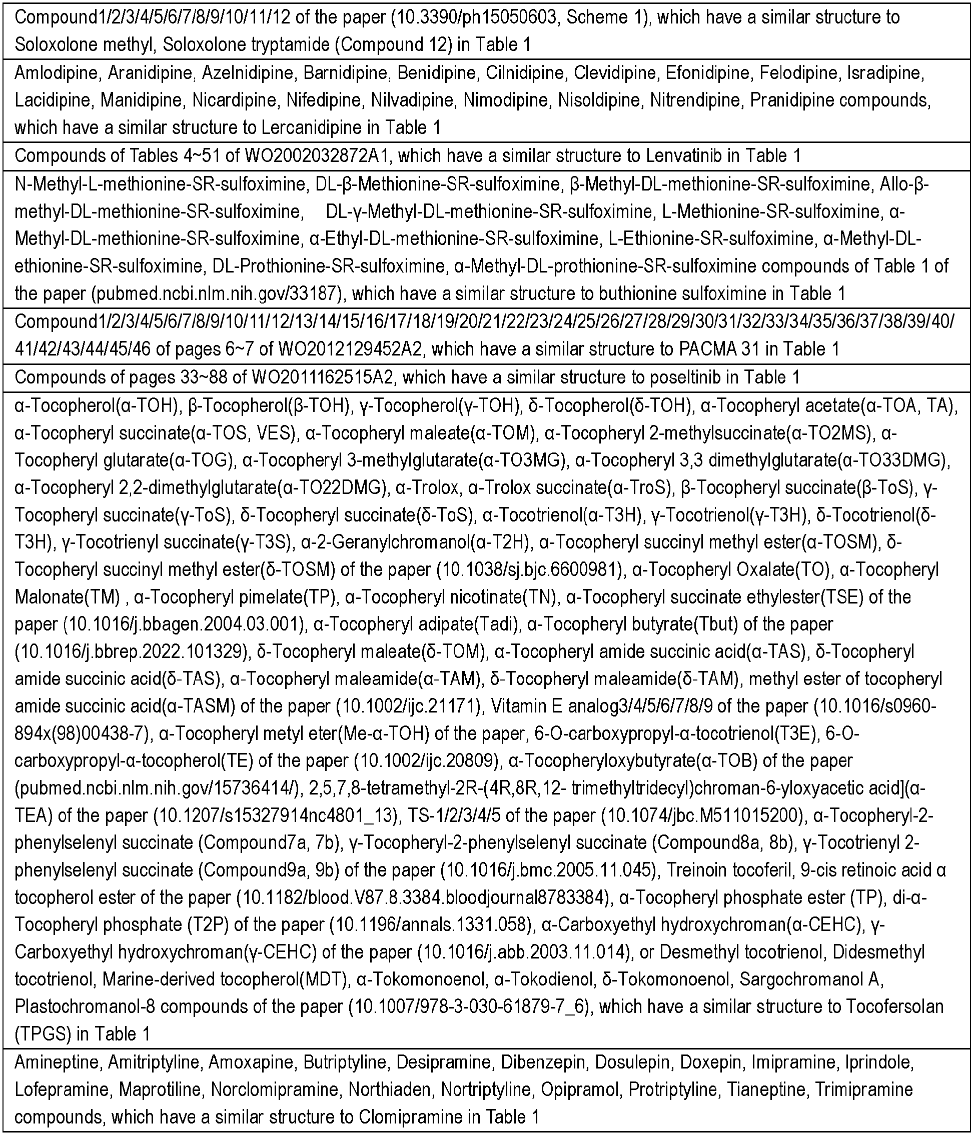

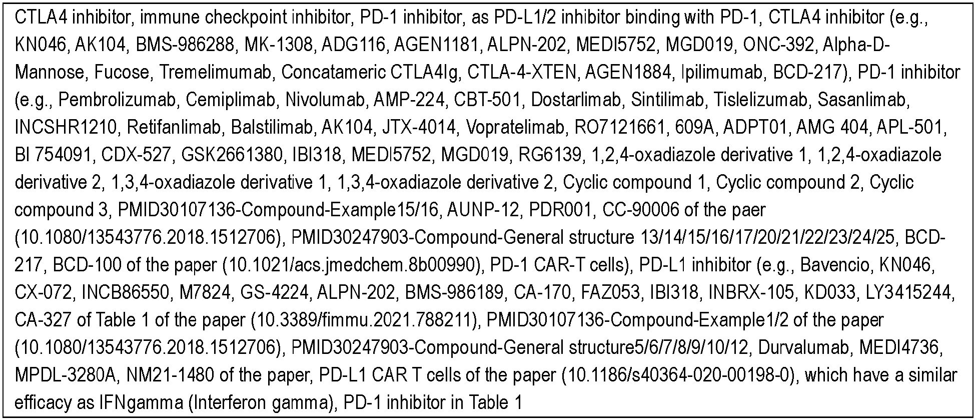

A preferred embodiment of the present invention provides a composition for removing senescent cells, comprising as an active agent (a) the compound of Table 1 or its pharmaceutically acceptable salt; (b) secreted fluid from cells killed by treatment with the compound of Table 1 or its pharmaceutically acceptable salt; or (c) cells killed by treatment with the compound of Table 1 or its pharmaceutically acceptable salt.

In a preferred embodiment of the present invention, the compound having the effect of removing senescent cells is at least one selected from the group consisting of the compounds specified in Table 1 above. For many purposes of the present invention, the compounds disclosed in Table 1 were more preferred.

In one embodiment of the invention, the CTLA4 inhibitor includes, for example, KN046, AK104, BMS-986288, MK-1308, ADG116, AGEN1181, ALPN-202, MEDI5752, MGD019, ONC-392, Alpha-D-Mannose, Fucose, Tremelimumab, Concatameric CTLA4Ig, CTLA-4-XTEN, AGEN1884, Ipilimumab, BCD-217, etc.

In one embodiment of the present invention, the PD-1 inhibitor includes, for example, Pembrolizumab, Cemiplimab, Nivolumab, AMP-224, CBT-501, Dostarlimab, Sintilimab, Tislelizumab, Sasanlimab, INCSHR1210, Retifanlimab, Balstilimab, AK104, JTX-4014, Vopratelimab, RO7121661, 609A, ADPT01, AMG 404, APL-501, BI 754091, CDX-527, GSK2661380, IBB 18, MEDI5752, MGD019, RG6139, 1,2,4-oxadiazole derivative 1, 1,2,4-oxadiazole derivative 2, 1,3,4-oxadiazole derivative 1, 1,3,4-oxadiazole derivative 2, Cyclic compound 1, Cyclic compound 2, Cyclic compound 3, PMID30107136-Compound-Example15/16, AUNP-12, PDR001, CC-90006, PMID30247903-Compound-General structure 13/14/15/16/17/20/21/22/23/24/25, BCD-217, BCD-100, PD-1 CAR T cells, etc. The structures of some of these compounds are as follows:

| The paper (10.1080/13543776.2018.1512706) =PMID30107136 | | |
|---|---|---|
| 1,2,4-oxadiazole derivative 1 = PMID30107136-Compound-Example39 | 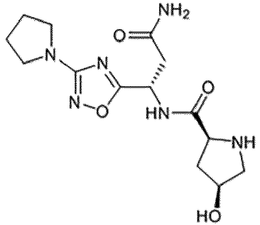 **Example 39** | 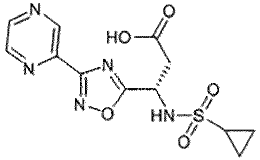 **Example 40** |
| 1,2,4-oxadiazole derivative 2 = PMID30107136-Compound-Example40 | | |
| 1,3,4-oxadiazole derivative 1 = PMID30107136-Compound-Example37 | 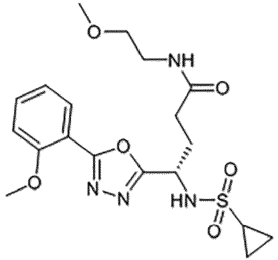 **Example 37** | 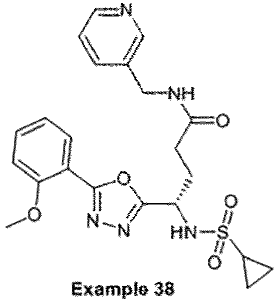 **Exemple 38** |
| 1,3,4-oxadiazole derivative 2 =PMID30107136-Compound-Example38 | | |
| Cyclic compound 1 = PMI030107136-Compound-Example80 | 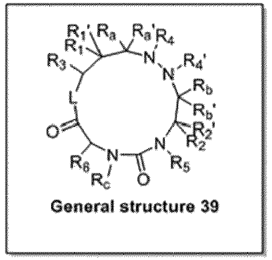 | 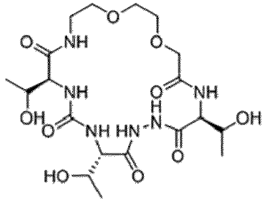 **Example 80** Splenocyte proliferation recovery 91% |
| Cyclic compound 2 = PMI030107136-Compound-Example81 | | |
| Cyclic compound 3 = PMI030107136-Compound-Example82 | | |
| | 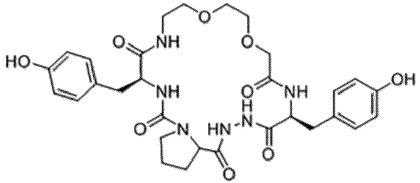 **Example 81** | 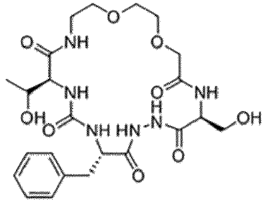 **Example 82** |
| PMID30107136-Compound-Example15 | 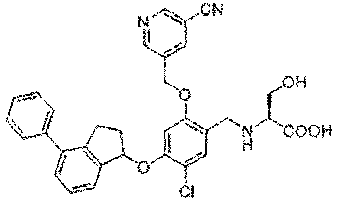 **Example 15** | 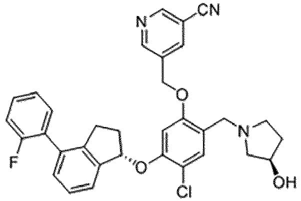 **Example 16** |
| PMID30107136-Compound-Example16 | | |

| The paper (10.1021/acs.jmedchem.8b00990) =PMID30247903 | | |
|---|---|---|
| PMID30247903-Compound-General structure13 | 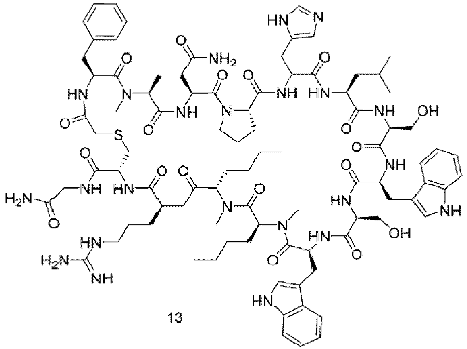 | |
| PMID30247903-Compound-General structure14 | 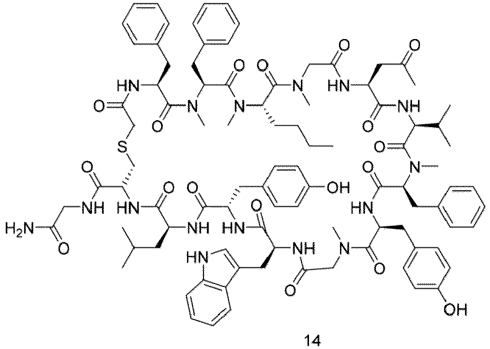 | |
| PMID30247903-Compound-General structure15 | 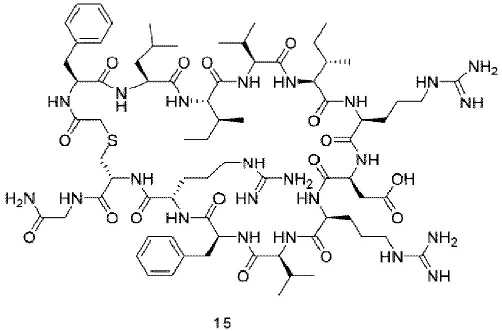 | |
| PMID30247903-Compound-General structure16 | 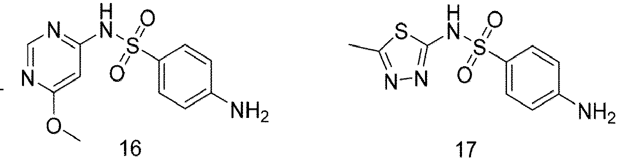 | |
| PMID30247903-Compound-General structure17 | | |
| PMID30247903-Compound-General structure20 | 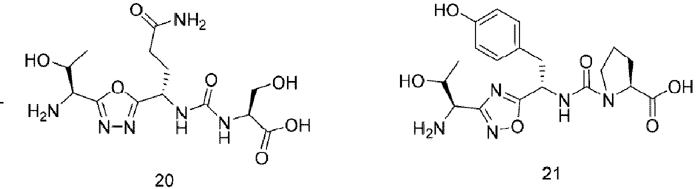 | |
| PMID30247903-Compound-General structure21 | | |
| PMID30247903-Compound-General structure22 | 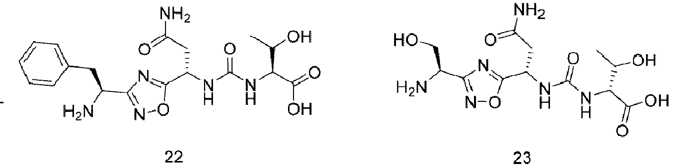 | |
| PMID30247903-Compound-General structure23 | | |
| PMID30247903-Compound-General structure24 | 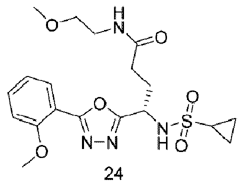 | 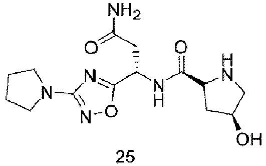 |
| PMID30247903-Compound-General structure25 | | |

In one embodiment of the present invention, the PD-L1 inhibitor includes, for example, Bavencio, KN046, CX-072, INCB86550, M7824, GS-4224, ALPN-202, BMS-986189, CA-170, FAZ053, IBI318, INBRX-105, KD033, LY3415244, CA-327, PMID30107136-Compound-Example1/2, PMID30247903-Compound-General structure5/6/7/8/9/10/12, Durvalumab, MEDI4736, MPDL-3280A, NM21-1480, PD-L1 CAR T cells, etc. The structures of some of these compounds are as follows:

| The paper (10.1080/13543776.2018.1512706) =PMID30107136 | | |
|---|---|---|
| PMID30107136-Compound-Example1 | 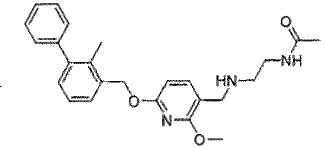 **Example 1** IC₅₀ 18 nM | 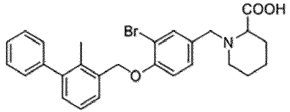 **Example 2** IC₅₀ 146 nM |
| PMID30107136-Compound-Example2 | | |

| The paper (10.1021/acs.jmedchem.8b00990) =PMID30247903 | |
|---|---|
| PMID30247903-Compound-General structures | 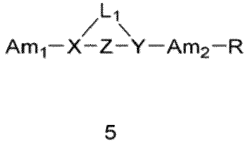 |
| PMID30247903-Compound-General structures | 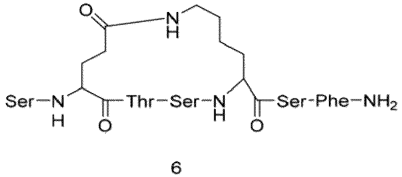 |
| PMID30247903-Compound-General structure7 | 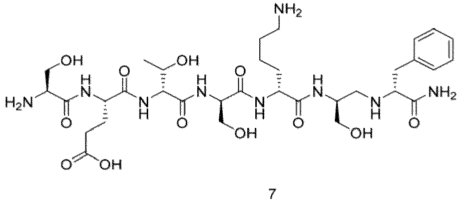 |
| PMID30247903-Compound-General structure8 | 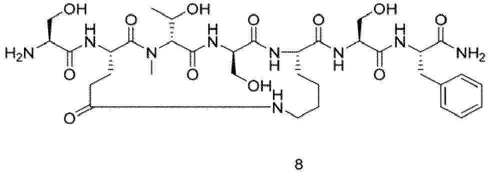 |
| PMID30247903-Compound-General structure9 | 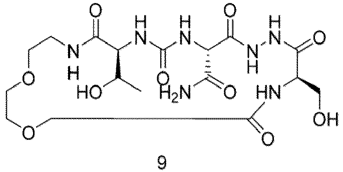 |
| PMID30247903-Compound-General structure10 | 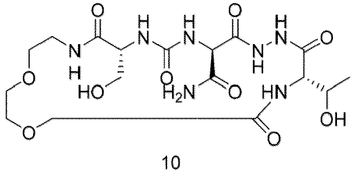 |
| PMID30247903-Compound-General structure12 | 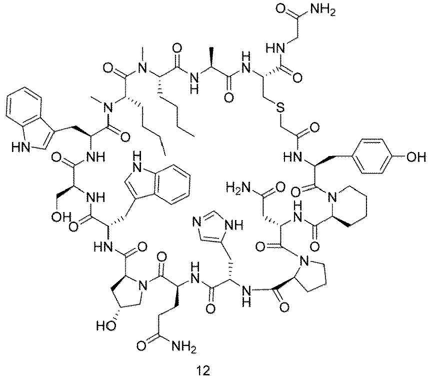 |

In one embodiment of the present invention, anti-PD-L2 antibodies, etc. may be used as the PD-L2 inhibitor.

The composition for removing senescent cells according to the present invention, that is, the composition comprising as an active agent (a) a compound of Table 1 or Table 2 (preferably, Table 1) or its pharmaceutically acceptable salt, (b) secreted fluid of cells killed by treatment with a compound of Table 1 or Table 2 (preferably, Table 1) or its pharmaceutically acceptable salt, or (c) cells killed by treatment with a compound of Table 1 or Table 2 (preferably, Table 1) or its pharmaceutically acceptable salt is useful for improving, treating or preventing diseases and disorders related to senescent cells by removing senescent cells.

That is, one embodiment of the present invention provides the use of the composition according to the present invention as a medicine, health functional food, cosmetics or animal product for removing senescent cells. Another embodiment of the present invention provides the use of the composition according to the present invention to improve, treat or prevent diseases and disorders related to senescent cells through removal of senescent cells.

Accordingly, another embodiment of the present invention provides a method of removing senescent cells, comprising contacting with senescent cells or administering or applying to a subject in need of treatment, improvement, or cosmetic effect for diseases and disorders related to senescent cells therapeutically, health-improvingly, or cosmetically effective amount of (a) a compound of Table 1 or Table 2 (preferably, Table 1) or its pharmaceutically acceptable salt, (b) secreted fluid of cells killed by treatment with a compound of Table 1 or Table 2 (preferably, Table 1) or its pharmaceutically acceptable salt, or (c) cells killed by treatment with a compound of Table 1 or Table 2 (preferably, Table 1) or its pharmaceutically acceptable salt.

Accordingly, another embodiment of the present invention provides a method of improving, treating or preventing diseases and/or disorders related to senescent cells, comprising administering or applying to a subject in need of treatment, improvement, or cosmetic effect for diseases and disorders related to senescent cells therapeutically, health-improvingly, or cosmetically effective amount of (a) a compound of Table 1 or Table 2 (preferably, Table 1) or its pharmaceutically acceptable salt, (b) secreted fluid of cells killed by treatment with a compound of Table 1 or Table 2 (preferably, Table 1) or its pharmaceutically acceptable salt, or (c) cells killed by treatment with a compound of Table 1 or Table 2 (preferably, Table 1) or its pharmaceutically acceptable salt.

In one embodiment of the present invention, the contact with senescent cells may be performed *in vitro* or *ex vivo.*

In one embodiment of the present invention, the composition according to the present invention is applied *in vitro* or *ex vivo* to a tissue, organ or cells before their transplantation or administration in the case of transplantation of tissue, organ or (stem) cells and administration of related therapeutic agents. The composition of the present invention is used to remove senescent cells from tissues, organs or cells that are transplanted or administered through such treatment.

As mentioned above, the composition of the present invention may be a pharmaceutical composition, health functional food composition, cosmetic composition, or animal product composition depending on its purpose. Therefore, another embodiment of the present invention provides a use of the active agent (a), (b), and/or (c) for the manufacture of medicine, health functional food, cosmetic or animal product which is useful for improving, treating or preventing diseases and disorders related to senescent cells.

In the present invention, aging refers to, but is not limited to, natural aging due to passage of time; aging caused by stress from chemotherapy, radiation therapy, etc.; aging due to diets high in fat, high in sugar, or high in salt, or due to nutritional deficiencies; aging due to infections such as viruses and bacteria; or aging caused by physiological disturbances caused by diseases such as obesity, diabetes, cancer, fibrosis, etc. In the present invention, aging refers to a phenomenon in the body that promotes the production and accumulation of senescent cells or activates the function of senescent cells.

The senescent cells can be derived from various cells, such as somatic cells, stem cells, and cells that have changed abnormally due to disease. The senescent cells may also be cells of organ tissue derived from liver, kidney, spleen, brain, lung, muscle, skin, fat, etc. The disease may be selected from the group consisting of inflammation, fibrosis, cancer, etc.

The senescent cells may exhibit any one or more of the following characteristics: (i) Cell division and growth are almost permanently halted. (ii) Compared to non-aging normal cells, the overall size of the cells is increased. In particular, the contents of lysosomes are increased and abnormal mitochondria are increased. (iii) Nuclear structural proteins such as Lamin Bl and chromatin-binding proteins such as HMGB1 are reduced. (iv) β-galactosidase (SA-β-gal) is expressed due to aging. (v) Lipofuscin is produced. (vi) CDK (cyclin-dependent kinase) inhibitory factors such as p16 and p21 are activated and expressed in large quantities. (vii) As a continuous stress response, signal transduction systems such as p53 are activated according to DDR (DNA damage response), and the expression of related target genes is regulated accordingly. In this way, it can be said that cell division and growth stop as a common feature of senescent cells, and that senescence-associated markers such as SA-β-gal, lipofuscin, p16, p21, and p53 are expressed. (See https://doi.org/10. 1016/j.tcb.2018.02.001).

The compound according to the present invention or its pharmaceutically acceptable salt promotes the death of senescent cells or inhibits the expression and secretion of aging-related markers, aging-promoting factors, or senescence-associated secretory factors. In one embodiment of the present invention, since these factors are expressed and secreted in senescent cells, the expression and secretion of these factors can be fundamentally blocked as the senescent cells are removed. Additionally, by removing senescent cells, the subject maintains resilience and resistance to various stresses.

The senescence-associated secretory factor (SASP) includes various aging-related pathological factors, and may be, for example, IL1α, IL1β, IL6, IL10, MCP1, PAIl VCAM1, MMP1, MMP2, MMP3, MMP12, MMP13, TGFβ, ACTA2, COL1A1, COL1A2, FN1, CXCL1, CXCL10, GM-CSF, GROα, GROβ, GROγ, IGFBP7, IL7, IL8, MIP1α, ENA78, GCP2, GITR, HGF, ICAM1, IGFBP2, IGFBP4, IGFBP5, IGFBP6, IL13, MCP4, MIF, MIP3α, MMP14, NAP2, PIGF, RANTES, sgp130, TIMP2, TRAILR3, Acrp30, Axl, bFGF, BLC, BTC, CTACK, EGFR, Fas, FGF7, GCSF, GDNF, HCC4, I309, IFNy, IGFBP1, IGFBP3, IL11, IL15, IL2Rα, IL6R, ITAC, Leptin, LIF, MSPα, PAI2, PDGF-BB, SCF, SDF1, sTNF-RI, sTNF-RII, TIMP-1, tPA, uPA, uPAR, VEGF, MCP3, IGF1, TGFβ3, MIP1, IL4, FGF7, PDGF-BB, IL16, BMP4, MDC, MCP4, IL10, TIMP1, ICAM1, Axl, CNTF, INFy, EGF, BMP6, etc. (See http://www.saspatlas.com).

One embodiment of the present invention is also based on the discovery that secreted fluid or substance from cells killed by treatment with a compound of Table 1 or Table 2 according to the present disclosure, preferably a compound of Table 1 or its pharmaceutically acceptable salt is useful in improving, treating or preventing various aging-related diseases by directly or indirectly inducing and/or promoting death of senescent cells, or directly or indirectly blocking and/or inhibiting the expression and secretion of aging-related markers, aging-promoting factors, or senescence-associated secretory factors (SASP).

In the present invention, "secreted fluid" includes components secreted and released from cells killed by treatment with the compound according to the present disclosure or its pharmaceutically acceptable salt. In one embodiment of the present invention, the "secreted fluid" may be a culture medium (e.g., medium) in which cells are cultured. In another preferred embodiment of the present invention, the secreted fluid of the present invention may be a culture filtrate obtained by removing the compound of the present disclosure from a culture medium (for example, medium) in which cells were cultured, or a purified separation liquid, a concentrate, or a dried product thereof. In the present invention, "culture filtrate" may refer to a culture solution in which cells are inoculated into a medium, treated with a compound of the present disclosure, cultured, and then the cells and compounds are removed. In one embodiment of the present invention, the culture filtrate can be obtained by (ultra)centrifuging the culture solution from which cells, compounds, etc. have been removed, and separating and purifying the obtained supernatant with (ultra)filtration, chromatography, etc. When administered into the body, such as the human body, dying cells producing culture filtrate or secreted fluid can be administered. In one embodiment of the present invention, the secreted fluid is contained in the culture filtrate or is produced and secreted from dying cells.

That is, one embodiment of the present invention is based on the discovery that (a) a compound that has the effect of killing or suppressing senescent cells or its salt, (b) secreted fluid from dying senescent cells, and/or (c) the dying senescent cells themselves can be used for suppressing or removing (other) senescent cells. The secreted fluid (substance) and dying senescent cells in the body may induce the death of senescent cells through activation of an immune response that activates and/or recruits immune cells, but the present invention is not limited to this theoretical mechanism.

In the present invention, "pharmaceutically acceptable salt" may be an acid addition salt formed with a pharmaceutically acceptable free acid. The free acid may be an inorganic acid or an organic acid. In this case, the inorganic acid may be hydrochloric acid, sulfuric acid, nitric acid, phosphoric acid, perchloric acid, hydrobromic acid, etc., and the organic acid may be acetic acid, methanesulfonic acid, ethanesulfonic acid, p-toluenesulfonic acid, fumaric acid, maleic acid, malonic acid, phthalic acid, succinic acid, lactic acid, citric acid, gluconic acid, tartaric acid, salicylic acid, malic acid, oxalic acid, benzoic acid, embonic acid, aspartic acid, glutamic acid, etc. The acid addition salt may be prepared by a conventional method, for example, by dissolving the compound of the present disclosure in an excessive amount of aqueous acid and precipitating the salt using a water-miscible organic solvent, such as methanol, ethanol, acetone, or acetonitrile. Additionally, when the compound of the present disclosure is acidic, the pharmaceutically acceptable salt can form base addition salts with various cations. Examples of such bases may be alkali metal or alkaline earth metal salts, for example calcium, magnesium, sodium, lithium, zinc, potassium and iron salts, among others. The alkali metal salt or alkaline earth metal salt can be obtained by dissolving the compound of the present disclosure in an excessive amount of alkali metal hydroxide or alkaline earth metal hydroxide solution, filtering the undissolved compound salt, and then evaporating and drying the filtrate. In addition, base addition salts include trimethylamine, triethylamine, dicyclohexylamine, ethanolamine, diethanolamine, triethanolamine, procaine, meglumine, ethylenediamine, choline, N,N-dibenzylethylenediamine, benzathine, benetamine, pyridine, picoline, quinoline, isoquinoline, tetramethylammonium, tetraethylammonium, benzyltrimethylammonium, benzyltriethylammonium, benzyltributylammonium, methyltrioctylammonium, tetrabutylammonium, and an organic salt such as, arginine, lysine, or histidine.

As used herein, the phrase "compound(s) of this/the present invention" includes any compound(s) of Table 1 or Table 2, as well as isotopic variants, clathrates, hydrates, solvates, or polymorphs thereof. And, even if the term "compound(s) of the invention" does not mention its pharmaceutically acceptable sat, the term includes salts thereof. In one embodiment, the compounds of this disclosure include stereo-chemically pure compounds, e.g., those substantially free (e.g., greater than 85% ee, greater than 90% ee, greater than 95% ee, greater than 97% ee, or greater than 99% ee) of other stereoisomers. That is, if the compounds of Chemical Formula 1 according to the present disclosure or salts thereof are tautomeric isomers and/or stereoisomers (e.g., geometrical isomers and conformational isomers), such isolated isomers and their mixtures also are included in the scope of this disclosure. If the compounds of the present disclosure or salts thereof have an asymmetric carbon in their structures, their active optical isomers and their racemic mixtures also are included in the scope of this disclosure.

As used herein, the term "isotopic variant" refers to a compound that contains unusual ratios of isotopes at one or more atoms that make up the compound. For example, isotopic variants of a compound may be radiolabeled. For example, the hydrogen atom may be selected from hydrogen, deuterium and tritium, and isotopic variants may contain carbon-13 (¹³C), nitrogen-15 (¹⁵N), etc.

If a compound (prodrug) is isolated in the body to produce a compound of the present disclosure or its salt, such compound is also included within the scope of the present invention. As used herein and unless otherwise indicated, the term "prodrug" refers to a compound that can be hydrolyzed, oxidized and subjected to other reactions under biological conditions *(in vitro* or *in vivo)* to provide an active compound, particularly a compound of the invention. Examples of prodrugs include compounds that contain biohydrolyzable moieties such as biohydrolyzable amides, biohydrolyzable esters, biohydrolyzable carbamates, biohydrolyzable carbonates, biohydrolyzable ureides, and biohydrolyzable phosphate analogs, and that biohydrolyze to produce the compounds of the invention, but are not limited to this specific examples. Preferably, the prodrug of the compound having a carboxyl group functional group is a lower alkyl ester of a carboxylic acid. Carboxylic esters are usually formed by esterifying a portion of the carboxylic acid present in the molecule. Prodrugs can be easily manufactured using methods well known in the field to which the present invention pertains.

As used herein, the term "polymorph" refers to solid crystalline forms of a compound of this disclosure or complex thereof. Different polymorphs of the same compound can exhibit different physical, chemical and/or spectroscopic properties. Different physical properties include, but are not limited to stability (e.g., to heat or light), compressibility and density (important in formulation and product manufacturing), and dissolution rates (which can affect bioavailability). Differences in stability can result from changes in chemical reactivity (e.g., differential oxidation, such that a dosage form discolors more rapidly when comprised of one polymorph than when comprised of another polymorph) or mechanical characteristics (e.g., tablets crumble on storage as a kinetically favored polymorph converts to thermodynamically more stable polymorph) or both (e.g., tablets of one polymorph are more susceptible to breakdown at high humidity). Different physical properties of polymorphs can affect their processing. For example, one polymorph might be more likely to form solvates or might be more difficult to filter or wash free of impurities than another due to, for example, the shape or size distribution of particles of it.

As used herein, the term "solvate" means a compound or its salt according to this disclosure that further includes a stoichiometric or non-stoichiometric amount of a solvent bound by non-covalent intermolecular forces. Preferred solvents are volatile, non-toxic, and acceptable for administration to humans in trace amounts.

As used herein, the term "hydrate" means a compound or its salt according to this disclosure that further includes a stoichiometric or non-stoichiometric amount of water bound by non-covalent intermolecular forces.

As used herein, the term "clathrate" means a compound or its salt in the form of a crystal lattice that contains spaces (e.g., channels) that have a guest molecule (e.g., a solvent or water) trapped within.

(a) A compound of Table 1 or Table 2, preferably a compound of Table 1, or its pharmaceutically acceptable salt, (b) secreted fluid from cells killed by treatment with a compound of Table 1 or Table 2, preferably a compound of Table 1, its pharmaceutically acceptable salt; or (c) cells killed by treatment with a compound of Table 1 or Table 2, preferably a compound of Table 1, or its pharmaceutically acceptable salt according to the present invention has (have) the effect of killing senescent cells or blocking the action of senescent cells, thereby being useful for improving, treating or preventing various diseases and disorders related to senescent cells.

In one embodiment of the present invention, the diseases or disorders that can be improved, treated, or prevented by removing senescent cells or inhibiting the action of senescent cells include, but are not limited to,
proliferative diseases and disorders such as cancer, pre-cancerous/pre-malignant condition, resistance, malignancy, relapse, and metastasis;
inflammatory or autoimmune diseases and disorders such as arthritis, osteoarthritis, rheumatoid arthritis, juvenile idiopathic arthritis, degenerative joint disorder, degenerative articular cartilage, inflammatory bowel disease, ulcerative colitis, Crohn's disease, mucositis, oral mucositis, chronic pancreatitis, tendinopathy, tendinosis, tendonitis or tendinitis, inflamm-aging, amplified hyperinflammation, cytokine release/storm syndrome, COVID19 infection, COVID19 complication, femoroacetabular impingement syndrome, carpal tunnel syndrome, meniscus tear, meniscus derangement, meniscus lesion, meniscus disorder, sepsis, HIV infection, AIDS, inflammatory response syndrome after surgery, and spondylarthritis;
neurological diseases and disorders such as neurodegenerative disease, dementia, alzheimer's disease, memory disorder, cognitive decline dysfunction, parkinson's disease, huntington's disease, amyotrophic lateral sclerosis, motor neuron disease, primary lateral sclerosis, progressive muscular atrophy, lower motor neuron disease, spinal muscular atrophy, progressive bulbar paralysis, pseudobulbar palsy, post-polio syndrome, spastic paraplegia, progressive supranuclear palsy, spinal cord injury, chronic pain, allodynia, anxiety, depression, neuropsychiatric dysfunction, post-traumatic stress disorder, gulf war illness, and sleep disorder;
metabolic diseases and disorders such as obesity, diabetes, diabetic ulcer, osteoporosis, intestinal bowel disease, intestinal bowel microbiome disorder, lipodystrophy, adipose atrophy, hypogonadism, and metabolic syndrome;
cardiovascular diseases and disorders such as atherosclerosis, heart failure, congestive heart failure, cardiomyopathy, heart functional disorder, cardiac dysfunction, angina pectoris, endocarditis, mitral valve prolapse, myocardial infarction, cardiomyocyte hypertrophy, loss of cardiac stress tolerance, idiopathic dilated cardiomyopathy, aortic aneurysm, coronary artery aneurysm, brain aneurysm, vascular hyporeactivity/calcification, aging-related vascular dysfunction, arteriovenous fistula, renal artery stenosis, carotid artery stenosis, neointimal hyperplasia, hypertension, stroke, thrombosis, coagulation disorder, dyslipidemia, atrial fibrillation, arrhythmia, and diabetic cardiomyopathy;
lung diseases and disorders such as chronic obstructive pulmonary disease, emphysema, chronic pneumonia, bronchiectasis, aging-related pulmonary dysfunction, respiratory failure, pulmonary function insufficient, hyperoxic lung injury, bronchopulmonary dysplasia, respiratory distress syndrome, pulmonary hypertension, asthma, viral pneumonia, interstitial pneumonia, cryptogenic organizing pneumonia, idiopathic pleuroparenchymal fibroelastosis, hypersensitivity pneumonitis, granulomatous-lymphocytic interstitial lung disease, and chronic bronchitis;
kidney diseases and disorders such as chronic kidney disease, nephropathy, renal failure/dysfunction, glomerulosclerosis, diabetic nephropathy, diabetic chronic kidney disease, immunoglobulin A nephropathy, kidney stone disease, nephrolithiasis, nephronophthisis, lupus nephritis, tubulointerstitial disease, and renal tubulointerstitial injury;
liver diseases and disorders such as cirrhosis, liver fibrosis, hepatitis, hepatopathy, fatty liver, nonalcoholic fatty liver disease, hepatic steatosis, nonalcoholic steatohepatitis, primary biliary cirrhosis, primary sclerosing cholangitis, biliary atresia, hepatic encephalopathy, and biliary injury;
eye diseases and disorders such as cataract, glaucoma, aging-related macular degeneration, macular degeneration, macular edema, retinal degenerative disease, aging-related vision loss, retinopathy, diabetic retinopathy, diabetic macular edema, dry eye disease, hyperemia, retinal microaneurysm, retinal microangiopathy, fuchs endothelial dystrophy, corneal dystrophies, and birdshot uveitis;
skin diseases and disorders such as skin aging, epidermal atrophy, loss of elasticity, wrinkle, skin photoaging, hair loss (alopecia), skin naevi, skin spot, pigmentation, hyperpigmentation, melasma, blemish, delayed wound healing, keloid, psoriasis, lupus, systemic lupus erythematosus, dermatopolymyositis, immunobullous disease, pemphigus, bullous pemphigoid, blister, wart, human papilloma virus infection, dermatitis, eosinophilic dermatoses, atopic dermatitis, skin lymphoma, erythroderma, lichen planus, lichenoid dermatosis, febrile neutrophilic dermatosis, rosacea, dermatofibroma, keratosis pilaris, herpes, pruritus, sensory defect, eczema, eruption, urticaria, vitiligo, senescence in gingival tissues, dyskeratosis congenita, ichthyosis vulgaris, actinic keratosis, photosensitivity, and photodermatoses;
regenerative maintenance-related diseases and disorders such as various stem cell/bone marrow regeneration maintenance diseases and disorders including bone marrow stem cell functional disorder, various organs/tissues regeneration maintenance diseases and disorders including liver regeneration, kidney organ regeneration, heart organ regeneration, etc., immune regulation diseases and disorders including myelodysplastic syndromes, myelopathy, inflammation, etc., and damage and decline in function of various organs/tissues including sarcopenia, etc.;
transplantation-related diseases and disorders such as transplantation diseases and disorders of various stem cells/bone marrow/organs/tissues/cells including graft versus host disease, stem cell transplantation complication, bone marrow transplantation complication, cardiac organ transplantation complication, renal organ transplantation complication, corneal allograft rejection, chronic allograft nephropathy, etc. and transplantation of various foreign substances/materials diseases and disorders including foreign body response;
fibrosis diseases and disorders such as idiopathic pulmonary fibrosis, pulmonary fibrosis, cystic fibrosis, renal fibrosis, tubulointerstitial fibrosis, liver fibrosis, cardiac fibrosis, pancreatic fibrosis, retroperitoneal fibrosis, mediastinal fibrosis, uterine fibrosis, biliary fibrosis, oral submucous fibrosis, muscle fibrosis, neurofibroma, myelofibrosis, and fibrodysplasia ossificans progressiva;
sclerosis diseases and disorders such as systemic sclerosis, scleroderma, and multiple sclerosis;
β-galactosidase-related diseases and disorders such as progerias, hutchinson-gilford progeria syndrome, werner syndrome, bloom syndrome, marfan syndrome, wiedemann-rautenstrauch syndrome, rothmund-thomson syndrome, cockayne syndrome, down-syndrome, and dyskeratosis congenita;
geriatric diseases and disorders such as reduced lifespan/healthspan, frailty, inflammaging, chronic inflammation, sarcopenia, muscular dystrophy, muscle fibrosis, myotonic dystrophy type 1, amyotonia congenita, fibromyalgia, osteoporosis, skeletal disorder, ataxia, delayed wound healing, delayed fracture healing, lipodystrophy, adipose atrophy, intervertebral disc degeneration, lumbar herniated intervertebral disc, kyphosis, low back pain, fibrodysplasia ossificans progressiva, menopause, tremor, urinary incontinence, urination disorder, prostatic hypertrophy, splenomeglay, hearing loss, olfactory dysfunction, and periodontal disease;
various aging-related diseases and disorders include damages, side effects and sequelae, fatigue, weight loss, physical inactivity, ataxia, cachexia, boredom, depression, anxiety, neuropsychiatric dysfunction, lethargy, neurosis, anorexia nervosa, syncope, panic disorder, chronic inflammation, mucositis, oral mucositis, thrombocytopenia, radiation ulcers, bone marrow disorder, anemia, immune disorder, hormone disorder, menopause, nausea, vomiting, diarrhea, hepato/nephrocomplication, hepato-/nephro-/cardio-/gastrointestinal/ovarian/vascular/skin toxicity, heart functional disorder, cardiomyopathy, congestive heart failure, peripheral neuropathy, hair loss, allopecia, dermatitis, eruption, pain, fluid retention, urticaria, hyperpigmentation, lipodystrophy, adipose atrophy, bone-loss, osteoporosis, infertility, abnormal embryo implantation and placentation, preeclampsia, pregnancy loss, miscarriage, cognitive decline dysfunction, cancer relapse, metastasis, vision loss, hearing loss, ovarian injury, hyposalivation, pulmonary fibrosis, blue light toxicity, etc., wherein the various aging-related diseases and disorders are caused by any one of anticancer drugs such as radiation/chemotherapy/targeted/immunotherapy, drugs/substances with strong toxicity and side effects, physiological disturbances such as obesity/diabetes, diets such as high fat/high sugar/high salt, hemorrhagic shock, myocardial infarction, hypoxia, ischemia and reperfusion damage, nutritional deficiencies, and various acute and chronic stressors such as war, accidents, smoking, various viral/bacterial infections such as HIV, foreign transplants, inflammation, tissue wounds, hyperthermia, etc.; and
rare aging-related diseases and disorders found in small numbers of the population such as acquired lipodystrophy, acromegaly, acute respiratory distress syndrome, alexander disease, alpha-1 antitrypsin deficiency, amyloidosis, autoimmune hemolytic anemia, aplastic anemia, anencephaly, antiphospholipid syndrome, takayasu arteritis, infectious arthritis, ataxia with vitamin E deficiency, behηet's syndrome, benign paroxysmal positional vertigo, beta thalassemia, biliary atresia, bloom syndrome, CDKL5 deficiency disorder, chronic granulomatous disease, common variable immune deficiency, corneal dystrophies, costello syndrome, creatine transporter deficiency, cystic fibrosis, cytochrome C oxidase deficiency, cytomegalovirus infection, danon disease, dengue fever, duchenne muscular dystrophy, dyskeratosis congenita, herpe simplex encephalitis, erdheim chester disease, fanconi anemia, fibrodysplasia ossificans progressiva, focal segmental glomerulosclerosis, friedreich's ataxia, frontotemporal degeneration, glucose-6-phosphate dehydrogenase deficiency, graft versus host disease, hepatic encephalopathy, hermansky pudlak syndrome, hodgkin's disease, huntington's disease, hutchinson-gilford progeria syndrome, progeria, hypoplastic left heart syndrome, idiopathic pulmonary fibrosis, immunoglobulin A nephropathy, immune thrombocytopenia, kawasaki disease, langerhans cell histiocytosis, leprosy, li-fraumeni syndrome, limb-girdle muscular dystrophies, lymphangioleiomyomatosis, malaria, marfan syndrome, measles, multiple myeloma, multiple sclerosis, becker muscular dystrophy, myelodysplastic syndromes, myhre syndrome, myotonic dystrophy, nontuberculous mycobacterial lung disease, pneumocystis pneumonia, primary biliary cholangitis, primary sclerosing cholangitis, pulmonary arterial hypertension, rett syndrome, rothmund-thomson syndrome, scleroderma, sialadenitis, ulcerative colitis, werner syndrome, wiedemann rautenstrauch syndrome, abdominal aortic aneurysm, ankylosing spondylitis, Ankyrin-B syndrome, anterior uveitis, PASLI disease, down syndrome, coronary artery aneurysm, dilated cardiomyopathy, doxorubicin induced cardiomyopathy, eclampsia, high molecular weight kininogen deficiency, interstitial cystitis, interstitial lung disease, kindler syndrome, lewy body dementia, lupus, lupus nephritis, neural tube defects, neuroferritinopathy, nijmegen breakage syndrome, nonalcoholic steatohepatitis, oral leukoplakia, oral submucous fibrosis, paraplegia, parkinson's disease, peripartum cardiomyopathy, psoriasis, silicosis, sjogren syndrome, tuberous sclerosis complex, xeroderma pigmentosum, acute myeloid leukemia, craniopharyngioma, glioblastoma, glioma, hepatocellular carcinoma, malignant melanoma, small cell lung cancer, thyroid cancer, acute monoblastic leukemia, diffuse large B-cell lymphoma, and oral squamous cell carcinoma.

In a preferred embodiment of the present invention, diseases and disorders that can be improved, treated or prevented by removing senescent cells or inhibiting the action of senescent cells include cancer (it is suppressed and treated by removing aged cancer cells through prosenescence anti-cancer therapy, which includes not only cancer but also early stage cancer, recurrence, metastasis, resistance, malignancy, or aftereffects), inflammation (it is inflamm-aging, immuno-senescence, or senescence-associated inflammation caused by senescent cells, and due to this, the progressive chronic and degenerative inflammation observed in the elderly, or it includes hyperamplified inflammation accompanied by cytokine storm observed in the elderly due to infections such as viruses), fibrosis and cirrhosis (including fibrosis and cirrhosis of the liver, lungs or kidneys, etc.), geriatric diseases (e.g., frailty (including decline in activity, endurance, muscle strength, and/or grip strength), sarcopenia, osteoporosis or lipoatrophy), kidney damage, liver damage, skin aging-related diseases and disorders (e.g., wrinkles, loss of elasticity, hair loss, deterioration of hair skin, freckles, blemishes, skin rash, skin spots, skin discoloration, pigmentation, warts, urticaria, worsening pores, delayed poor wound healing, etc.), and side effects and aftereffects (including fatigue, weakness, weight loss, anxiety, depression, lethargy, fatty liver, etc.) due to stress caused by anticancer drugs or obesity.

In relation to the above frailty, frailty is, for example, a specific disease to which the R54 code was recently assigned, which shows the same symptoms as aging-related fatigue, weakness, asthenia, inactivity, muscle weakness, and gait disorder that occur with or without cognitive and memory impairment due to aging. Frailty can be analyzed by symptom indicators (https://en.wikipedia.org/wiki/Frailty_syndrome).

As used herein, the term "treatment" refers to any action (alleviate, reverse or treat) in which the symptoms of aging-related diseases and disorders are improved, ameliorated, or beneficially changed by administration of the active agent of the present invention, and the term "prevention" refers to any action (prevent, inhibit or delay) that blocks, inhibits, or delays the progression of aging-related diseases and disorders by administering a composition according to the present invention.

In an embodiment of the present invention, (a) a compound of Table 1 or Table 2 (preferably, Table 1) or its pharmaceutically acceptable salt, (b) secreted fluid from cells killed by treatment with a compound of Table 1 or Table 2 (preferably, Table 1) or its pharmaceutically acceptable salt, or (c) cells killed by treatment with a compound of Table 1 or Table 2 (preferably, Table 1) or its pharmaceutically acceptable salt can be administered or applied to a patient in a therapeutically, cosmetically, or pharmaceutically effective amount.

One embodiment of the present invention provides a method of improving, treating or preventing diseases and/or disorders related to senescent cells, comprising contacting with senescent cells or administering or applying to a subject in need of treatment, improvement, or prevention for diseases and disorders related to senescent cells therapeutically, cosmetically or pharmaceutically effective amount of a substance that increases the secretion of HMGN1 (High Mobility Group Nucleosome Binding Domain 1) protein (HMG14) in senescent cells. In this method, the 'substance that increases secretion of HMG14' and 'disease and disorders related to senescent cells' are the same as previously described.

Here, "therapeutically effective amount" or "pharmaceutically effective amount" refers to the amount of a compound or composition effective in preventing or treating the target disease, which is sufficient to treat the disease with a reasonable benefit/risk ratio applicable to medical treatment and does not cause side effects. Additionally, the "effective amount" refers to an amount sufficient to inhibit or reduce the activity of senescent cells in aging-related diseases, either *in vitro* or *in vivo.* The level of the effective amount can be determined by factors including the patient's health status, type and severity of the disease, activity of the drug, sensitivity to the drug, administration method, administration time, administration route and excretion rate, treatment period, combination or concurrent use of drugs, and other factors well known in the medical field.

One embodiment of the present invention also provides a method of removing senescent cells by contacting them *in vivo* or *ex vivo* with (a) a compound of Table 1 or Table 2 (preferably, Table 1) or its pharmaceutically acceptable salt, (b) secreted fluid from cells killed by treatment with a compound of Table 1 or Table 2 (preferably, Table 1) or its pharmaceutically acceptable salt, or (c) cells killed by treatment with a compound of Table 1 or Table 2 (preferably, Table 1) or its salt.

In one embodiment of the present invention, the composition of the present invention can be used in combination with an immune checkpoint inhibitor for use as described above (i.e., for removing senescent cells and/or improving, treating or preventing diseases and disorders related to senescent cells). That is, the composition of the present invention is more preferable for the purposes of the present invention when used in combination with an immune checkpoint inhibitor.

In one embodiment of the present invention, the immune checkpoint inhibitor may be comprised in the composition of the present invention, or may be administered simultaneously or sequentially as a separate composition (formulation). Accordingly, one embodiment of the present invention provides a kit or preparation for removing senescent cells, containing the composition according to the present invention and an immune checkpoint inhibitor.

Accordingly, another embodiment of the present invention also provides a method for removing senescent cells, comprising contacting senescent cells with or administering or applying to a subject in need of improvement, treatment, or prevention diseases and disorders related to senescent cells the composition according to the present invention simultaneously or sequentially with one or more additional immune checkpoint inhibitor. Other embodiment of the present invention also provides a method for improving, treating or preventing diseases and disorders related to senescent cells, comprising contacting senescent cells with or administering or applying to a subject in need of improvement, treatment, or prevention diseases and disorders related to senescent cells the composition according to the present invention simultaneously or sequentially with one or more additional immune checkpoint inhibitor.

In this combination or combination method, the immune checkpoint inhibitor may be any one or more of the aforementioned CTLA4 inhibitor, PD-1 inhibitor, PD-L1 inhibitor, and PD-L2 inhibitor. In this case, the compound comprised in the composition according to the present invention, which are used together or combined with an immune checkpoint inhibitor, and the compound used for treating cells to obtain the secreted fluid of cells or dying cells according to the present invention are a compound other than CTLA4 inhibitors, PD-1 inhibitors, PD-L1 inhibitors, and PD-L2 inhibitors.

In this combination or combination method, the composition of the present invention and the immune checkpoint inhibitor are administered in any order or simultaneously. If simultaneous, the compositions of the invention and the immune checkpoint inhibitor may optionally be provided in a single, unified form, or in multiple forms (e.g., as one pill or two separate pills).

Additionally, in this combination or combination method, the combination methods, compositions and preparations are not limited to the use of only two agents, and multiple therapeutic combinations are also contemplated. Dosage regimens for treating, preventing, or ameliorating pathological condition(s) may vary depending on various factors. These factors include the individual's age, weight, sex, diet, and medical condition, as well as any disorders the individual suffers from.

The pharmaceutical preparations that make up the combination treatment disclosed herein are optionally in combined formulations or in separate formulations primarily for simultaneous administration. The pharmaceutical preparations that make up the combination treatment can also be administered sequentially as either of the agents administered by a regimen requiring two-step administration. A two-step dosing regimen may require sequential administration of the active agents or spaced administration of separate active agents. The time period between multiple administration steps can range from several minutes to several hours depending on the properties of each pharmaceutical preparation such as potency, solubility, bioavailability, plasma half-life and kinetic profile of the pharmaceutical agent. It can last up to several hours. Circadian variation in target molecule concentration is used to determine the optimal dosing interval.

For the treatment of the diseases or conditions referred to above, the active agents and/or compositions described herein can be administered as follows:

### Oral administration

The compounds of the present invention may be administered orally, including by swallowing, so that the compound enters the gastrointestinal tract, or absorbed into the blood stream directly from the mouth (e.g., buccal or sublingual administration).

Suitable compositions for oral administration include solid, liquid, gel or powder formulations, and have a dosage form such as tablet, lozenge, capsule, granule or powder.

Liquid formulations can include solutions, syrups and suspensions, which can be used in soft or hard capsules. Such formulations may include a pharmaceutically acceptable carrier, for example, water, ethanol, polyethylene glycol, cellulose, or an oil. The formulation may also include one or more emulsifying agents and/or suspending agents.

In a tablet dosage form the amount of drug, active agent, present may be from about 0.05% to about 95% by weight, more typically from about 2% to about 50% by weight of the dosage form. In addition, tablets may contain a disintegrant, comprising from about 0.5% to about 35% by weight, more typically from about 2% to about 25% of the dosage form. Examples of disintegrants include, but are not limited to, lactose, starch, sodium starch glycolate, crospovidone, croscarmellose sodium, maltodextrin, or mixtures thereof.

Suitable lubricants, for use in a tablet, may be present in amounts from about 0.1% to about 5% by weight, and include, but are not limited to, talc, silicon dioxide, stearic acid, calcium, zinc or magnesium stearate, sodium stearyl fumarate and the like.

Suitable binders, for use in a tablet, include, but are not limited to, gelatin, polyethylene glycol, sugars, gums, starch, polyvinyl pyrrolidone, hydroxypropyl cellulose, hydroxypropylmethyl cellulose and the like. Suitable diluents, for use in a tablet, include, but are not limited to, mannitol, xylitol, lactose, dextrose, sucrose, sorbitol, microcrystalline cellulose and starch.

Suitable solubilizers, for use in a tablet, may be present in amounts from about 0.1% to about 3% by weight, and include, but are not limited to, polysorbates, sodium lauryl sulfate, sodium dodecyl sulfate, propylene carbonate, diethyleneglycol monoethyl ether, dimethyl isosorbide, polyethylene glycol (natural or hydrogenated) castor oil, HCOR^{™} (Nikkol), oleyl ester, Gelucire^{™}, caprylic/caprylic acid mono/diglyceride, sorbitan fatty acid esters, and Solutol HS^{™}.

### Parenteral Administration

The active agent of the present disclosure may be administered directly into the blood stream, muscle, or internal organs. Suitable means for parenteral administration include intravenous, intra-muscular, subcutaneous intraarterial, intraperitoneal, intrathecal, intracranial, and the like. Suitable devices for parenteral administration include injectors (including needle and needle-free injectors) and infusion methods.

Most parenteral formulations are liquid compositions, and the liquid composition is an aqueous solution containing the active ingredient according to the present invention, a salt, a buffering agent, an isotonic agent, and the like.

Parenteral formulations may also be prepared in a dehydrated form (e.g., by lyophilization) or as sterile non-aqueous solutions. These formulations can be used with a suitable vehicle, such as sterile water. Solubility-enhancing agents may also be used in preparation of parenteral solutions.

### Topical Administration

The active agent of the present invention may be administered topically to the skin or transdermally. Formulations for this topical administration can include lotions, solutions, creams, gels, hydrogels, ointments, foams, implants, patches and the like. Pharmaceutically acceptable carriers for topical administration formulations can include water, alcohol, mineral oil, glycerin, polyethylene glycol and the like.

When the composition of the present invention is a cosmetic composition, it may be in the liquid formulation mentioned above.

### Various other administration methods

In addition to the above methods, the present invention includes various administration methods, and further, the composition of the present invention can be used to remove senescent cells *in vitro,* such as in the case of foreign transplantation of tissues/organs or (stem) cells and administration of therapeutic agents.

### [Advantageous Effects]

The active agent and composition according to the present invention can fundamentally block and suppress the expression and secretion of various aging-related pathological factors secreted from senescent cells by removing senescent cells. In addition, the secreted fluid from cells whose death was induced by treatment with the composition for removing senescent cells according to the present invention significantly reduces the survival rate of senescent cells, and thereby can fundamentally block and suppress the expression and secretion of various aging-related pathological factors secreted from these senescent cells. Therefore, the composition for removing senescent cells according to the present invention can be usefully used to improve, treat, or prevent aging-related diseases and disorders. Furthermore, the composition for removing senescent cells according to the present invention can be usefully used in cosmetics, health functional foods, animal products, etc. in relation to aging-related diseases and disorders related to senescent cells.

### [Brief Description of the Drawing]

Figure 1 shows the effect of removing senescent cells using the compound of the present invention.
Figure 2 is a photograph of staining adipose tissues in mice in which ageing has naturally induced under conditions in which they were not treated and under conditions in which they were treated with a representative compound for removing senescent cells.
Figure 3 is a photograph of staining adipose tissues in mice in which aging was induced by doxorubicin (DOX) under conditions in which they were not treated and under conditions in which they were treated with a representative compound for removing senescent cells.

### [Mode for Invention]

Hereinafter, to aid understanding of the present invention, it will be described in detail through examples. However, the examples according to the present invention may be modified into various other forms, and the scope of the present invention should not be construed as being limited to the following examples. Examples of the present invention are provided to more completely explain the present invention to those with average knowledge in the field to which the present invention pertains.

In Examples 1 to 9 below, LX-112 (ethaselen, 1,2-[bis(1,2-benzisoselenazolone-3(2H)-ketone)]ethane) was used as an exemplary compound for removing senescent cells. Several evaluations were conducted to evaluate the effectiveness of improving, treating, or preventing diseases and disorders related to senescent cells by removing senescent cells.

### Example 1. Removal of senescent cells from senescent human cells

### Example 1-1. Induction of cellular senescence in human cells

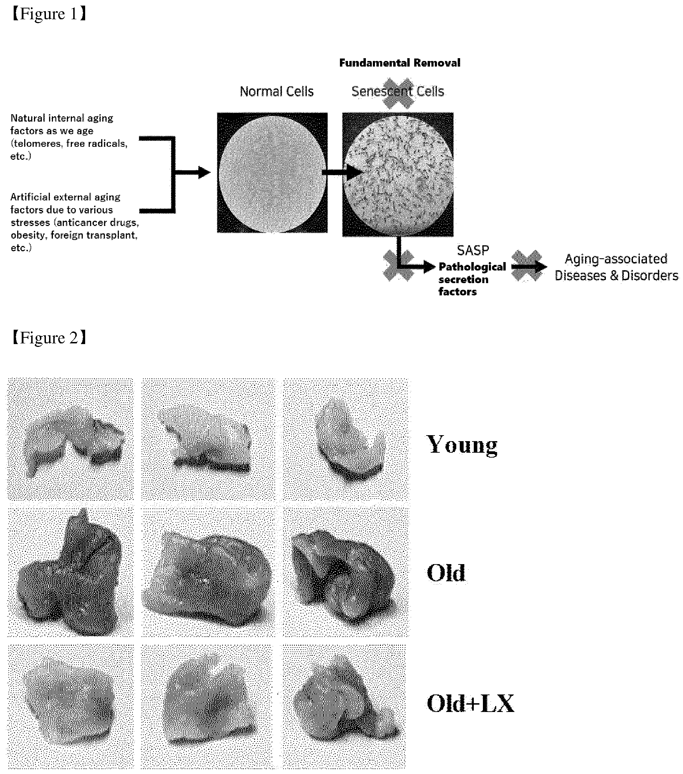

As we age, aging-related diseases and disorders occur due to pathological factors (SASP) secreted by senescent cells that accumulate in the human body due to internal and external factors. To confirm that selenium compounds remove senescent cells, which play a fundamental role in this process, senescent cells were stained with SA-β-gal, an aging marker. The action of such a selenium compound is shown in Figure 1.

Specifically, foreskin cells (source: Chungnam National University College of Medicine) derived from humans were cultured in a designated medium (Iscove's Modified Dulbecco's Medium, source: ATCC; 10% FBS, source: GenDEPOT; 100 U/ml Penicillin & Streptomycin, source: Invitrogen), then treated with doxorubicin (DOX) dissolved in dimethyl sulfoxide (DMSO) at a concentration of 250 nM for 24 hours, and further cultured for 7 days to induce aging. The control group (-), which did not induce aging, was treated with DMSO only. To measure the proportion of aged cells, cells treated with doxorubicin and the control group were inoculated into a 24 well plate and stabilized for 24 hours. Then SA-β-gal, a marker related to aging, was stained using a kit (purchased from Cell Signaling Technology), and the proportion of senescent cells having the stained SA-β-gal was measured using an optical microscope. Results are shown in Table 3.

**[Table 3]**

| | | |
|---|---|---|
| SA-β-gal (%) | - | 9 (±5) |
| | DOX | 90 (±7) |

As a result, the ratio of aging-related marker (SA-β-gal) in cells treated with doxorubicin (DOX) increased significantly compared to the control group (-), and the cells no longer grew. From this, it was confirmed that aging was induced.

### Example 1-2. Removal of senescent cells by LX-112

Cells treated with doxorubicin and the control in the same manner as in Example 1-1 were treated with LX-112 (purchased from BOC) at concentrations of 0 µM, 1.1 µM, 3.3 µM, and 10 µM, respectively, and 3 days later, cell viability and growth rate were measured using CellTiter-Glo Luminescent Cell Viability Assay (purchased from Promega) and are shown in Table 4 and Table 5. At this time, N0 in Table 5 is calculated as 100% of the cell number before LX-112 treatment.

**[Table 4]**

| LX | 0 µM | 1.1 µM | 3.3 µM | 10 µM |
|---|---|---|---|---|
| Survival (%) | 100 (±4) | 67.2 (±7) | 27 (±4) | 1 (±0.4) |

**[Table 5]**

| LX | N0 | 0 µM | 1.1 µM | 3.3 µM | 10 µM |
|---|---|---|---|---|---|
| Growth (%) | 100 (±11) | 253 (±4) | 201 (±8) | 161 (±7) | 123 (±8) |

As a result, as shown in Table 4, in cells where senescence was induced by treatment with doxorubicin, the higher the concentration of LX-112, the faster the cell survival rate decreased. In addition, as shown in Table 5, there was no significant change in cell survival rate even when LX-112 was treated at various concentrations in cells in which senescence was not induced by treatment with the control, and the cells grew normally. In addition to somatic cells, the death of senescent cells by LX-112 was confirmed to be about 50% in stem cells (umbilical cord-derived mesenchymal stem cells, purchased from ATCC) at a concentration of 10 µM. From this, it was confirmed that LX-112 can effectively remove senescent cells by inducing their death.

### Example 2. Removal of senescent cells by LX-112 from senescent human cancer cells and tumors

### Example 2-1. Induction of cellular senescence in human cancer cells

SAOS-2 (purchased from ATCC), a human-derived osteosarcoma cancer cell line, was cultured in the designated medium (DMEM, purchased from GenDEPOT; 10% FBS, purchased from GenDEPOT; 100 U/ml Penicillin & Streptomycin, purchased from Invitrogen). Then, after inducing aging in the same manner as Example 1-1, the ratio of aging-related marker (SA-β-gal) in cells was measured, and the results are shown in Table 6.

**[Table 6]**

| | | |
|---|---|---|
| SA-β-gal (%) | - | 5 (±1) |
| | DOX | 66 (±5) |

As a result, as shown in Table 6, the ratio of aging-related marker (SA-β-gal) in cells treated with doxorubicin (DOX) increased significantly compared to the control group (-), and the cells no longer grew. From this, it was confirmed that aging was induced.

### Example 2-2. Removal of senescent cancer cells by LX-112

The standard for treating various cancers, including osteosarcoma, is repeated chemotherapy. Accordingly, aging was induced in SAOS-2 in the same manner as in Example 2-1, and then (i) treated again with doxorubicin (DOX → DOX) or (ii) treated with the LX-112 (5 µM) composition (DOX → LX-112). Then, after 3 days (3d) and 7 days (7d), cell viability was measured using CellTiter-Glo Luminescent Cell Viability Assay (purchased from Promega), and the results are shown in Table 7 and Table 8.

**[Table 7]**

| | | | |
|---|---|---|---|
| | - | DOX → DOX | |
| | | 3d | 7d |
| Survival (%) | 100 (±6) | 72 (±7) | 55 (±5) |

**[Table 8]**

| | | | |
|---|---|---|---|
| | - | DOX → LX | |
| | | 3d | 7d |
| Survival (%) | 100 (±3) | 35 (±4) | 14 (±5) |

As a result, as shown in Table 7 and Table 8, the combined and sequential treatment of doxorubicin and LX-112 (DOX → LX-112) was more effective in decreasing the survival rate of cancer cells than when SAOS-2, which lacks the intracellular anticancer genes Rb and p53, was repeatedly treated with doxorubicin (DOX → DOX). From this, it was confirmed that the combined and sequential treatment with an anticancer drug that induces aging and LX-112, a composition for removing senescent cells, exhibited a more effective anticancer effect than in the case of repeated treatment with anticancer drugs through chemotherapy.

### Example 2-3. Suppression of senescent tumors by LX-112 in mice

After 7-week-old male BALB/c nude mice (purchased from KRIBB Laboratory Animal Resource Center) were adapted to the experimental environment for one week, the mice were randomized into an experimental group and a control group. SAOS-2 (10⁶ cells), a human osteosarcoma cancer cell line cultured in the same manner as Example 2-1, was mixed with PBS (purchased from Welgene) and then anesthetized using 2% isoflurane (purchased from Sigma). It was administered subcutaneously to mice. When the size of the tumor reached about 50 mm², doxorubicin was mixed at a concentration of 10 mg/kg in a 1:1 solution of distilled water and PBS (pH 7.2) and administered intraperitoneally to induce tumor aging. After 10 days, LX-112 mixed with 0.5% CMC-Na solution (purchased from Sigma) at a concentration of 100 mg/kg was orally administered daily for 21 days, and the size of the tumor was measured. The control group was repeatedly administered a solution mixed with doxorubicin, and then the size of the tumor was measured. The comparison results are shown in Table 9.

**[Table 9]**

| | | |
|---|---|---|
| Tumor Diameter (mm) | DOX → DOX | 15.4 (±1.2) |
| | DOX → LX | 6.2 (±4.8) |

As a result, as shown in Table 9, when aging-induced tumors in mice were treated with LX-112 (DOX → LX), the size of the tumor was further effectively suppressed and reduced compared to when the tumors were repeatedly treated with doxorubicin (DOX → DOX). From this, it was confirmed that treatment with LX-112, a composition for removing senescent cells in the formed tumor, exhibited a more effective anticancer effect than when repeatedly treated with anticancer chemotherapy drugs in mice.

### Example 3. Removal of senescent cells by LX-112 from senescent human cells

### Example 3-1. Induction of replicative senescence in human cells

Foreskin cells derived from humans were subcultured (REP; replicative senescence) in a designated medium (Iscove's Modified Dulbecco's Medium, ATCC; 10% FBS, GenDEPOT; 100 U/ml Penicillin & Streptomycin, Invitrogen) until no longer grown in order to induce natural aging. As a control group (-), foreskin cells before natural aging were used. Afterwards, the ratio of cellular aging-related marker (SA-β-gal) was measured using the same method as Example 1-1, and the results are shown in Table 10.

**[Table 10]**

| | | |
|---|---|---|
| SA-β-gal (%) | - | 7.2 (±2) |
| | REP | 93.8 (±5) |

As a result, as shown in Table 10, the rate of aging-related marker (SA-β-gal) significantly increased in cells that were continuously subcultured (REP) until they no longer grew. From this, it was confirmed that aging was induced.

### Example 3-2. Removal of senescent cells by LX-112

Natural aging of human-derived foreskin cells was induced in the same manner as Example 3-1, then treated with LX-112 at concentrations of 0 µM, 1.1 µM, 3.3 µM, and 10 µM, and the cell survival rate was measured with CellTiter-Glo Luminescent Cell Viability Assay (purchased from Promega) 3 days later. The cell survival rate is shown in Table 11, and the cell growth rate for cells in which senescence was not induced is shown in Table 12. At this time, in Table 12, N0 is calculated as 100% of the cell number before LX-112 treatment.

**[Table 11]**

| LX | 0 µM | 1.1 µM | 3.3 µM | 10 µM |
|---|---|---|---|---|
| Survival (%) | 100 (±4) | 59 (±6) | 29 (±5) | 1 (±0.9) |

**[Table 12]**

| LX | N0 | 0 µM | 1.1 µM | 3.3 µM | 10 µM |
|---|---|---|---|---|---|
| Growth (%) | 100 (±6) | 262 (±13) | 206 (±15) | 151 (±12) | 122.7 (+16) |

As a result, as shown in Table 11 and Table 12, the higher the concentration of LX-112 in cells in which senescence was induced, the cell survival rate decreased rapidly. In cells in which senescence was not induced, treatment with LX-112 at various concentrations caused no change in cell survival rate, and cells grew normally. From this, it was confirmed that LX-112 effectively removes senescent cells by inducing their death.

### Example 4. Reduction in the amount of aging-related marker expressed in senescent cells by LX-112

In the same manner as Example 3, natural aging of human foreskin cells was induced, treated with LX-112 at a concentration of 3 µM, and the amounts of the mRNA of p16 and p21, aging-related markers, were analyzed by qRT-PCR (quantitative real-time PCR). As a control group, foreskin cells before senescence induction were used. Specifically, cells were collected and treated with TRIzol Reagent (purchased from Thermo Fisher Scientific) to extract total RNA. The concentration of extracted total RNA was quantified using Nanodrop (purchased from Micro Digital). Genomic DNA was removed from the extracted total RNA using SuperScript IV VILO Master Mix with ezDNase Enzyme (purchased from Thermo Fisher Scientific), and cDNA was prepared. Using the synthesized cDNA, using the housekeeping gene GAPDH as a standard, qRT-PCR for p16 and p21 was performed using SFCgreen I qPCR Master Mix (purchased from SFC Probe), and was analyzed with CFX Connect Real-Time PCR Detection System (purchased from Bio Rad). Table 13 shows the results showing the mRNA amounts of p16 and p21, which are aging-related markers, before and after inducing aging, and Table 14 shows the mRNA amounts of p16 and p21, which are aging-related markers, under conditions in which aging was induced and LX-112 was not treated or treated. Table 14 calculates the mRNA amounts of p16 and p21 after senescence was induced to 100%, respectively, and compared them with the mRNA amounts of p16 and p21 after treatment with LX-112. The primers used were synthesized through bionics, and the sequences are shown in Table 15 below.

**[Table 13]**

| mRNA (Fold) | p16 | p21 |
|---|---|---|
| - | 1 (±0.1) | 1 (±0.3) |
| REP | 5 (±0.5) | 4 (±0.2) |

**[Table 14]**

| mRNA (%) | p16 | p21 |
|---|---|---|
| - | 100 (±4) | 100 (±6) |
| LX | 43 (±4) | 39 (±7) |

**[Table 151**

| Gene | Primer 1 | Seq. No. | Primer 2 | Seq. No. |
|---|---|---|---|---|
| GAPDH | GGAAGGGCTCATGACCACAG | 1 | ACAGTCTTCTGGGTGGCAGTG | 2 |
| p16 | TGAGCTTTGGTTCTGCCATT | 3 | AGCTGTCGACTTCATGACAAG | 4 |
| p21 | GAGACTAAGGCAGAAGATGTAGAG | 5 | GCAGACCAGCATGACAGAT | 6 |

As a result, as shown in Table 13, when senescence was induced in cells, the mRNA amounts of p16 and p21 rapidly increased. Additionally, as shown in Table 14, when senescence-induced foreskin cells were treated with LX-112, the mRNA amounts of p16 and p21 decreased. From this, it was confirmed that LX-112 effectively reduces the amount of p16 and p21, aging-related markers that increase with cellular aging.

### Example 5. Effect of LX-112 on reducing the amount of aging-related inflammation and fibrosis secretory factors (SASP) expressed and secreted by senescent cells

In the same manner as Example 3, natural aging of human foreskin cells was induced and treated with LX-112 at a concentration of 3 µM. Then the expression levels of aging-related inflammation and fibrosis secretory factors (SASP), IL1α, IL1β, IL6, IL10, MCP1, PAIl VCAM1, MMP1, MMP2, MMP3, MMP12, and TGFβ, were measured using an immunoassay (ProcartaPlex Immunoassay, purchased from Thermo Fisher Scientific) with the obtained culture medium. Table 16 shows the amounts of the aging-related inflammation and fibrosis secretory factor (SASP) before (-) and after (REP) inducing aging, and Table 17 shows the amounts of the aging-related inflammation and fibrosis secretory factor (SASP) in the conditions without and with LX-112 treatment after inducing aging. Table 17 calculates the amount of each factor after inducing aging to 100% and compares it with the amount of each factor after treatment with LX-112.

**[Table 16]**

| Protein (Fold) | IL1α | IL1β | IL6 | IL10 | MCP1 | PAI1 |
|---|---|---|---|---|---|---|
| - | 1 (±0.3) | 1 (±0.2) | 1 (±0.1) | 1 (±0.1) | 1 (±0.2) | 1 (±0.1) |
| REP | 14 (±6) | 11 (±3) | 64 (±6) | 16 (±4) | 25 (±5) | 69 (±9) |
| | | | | | | |

| Protein (Fold) | VCAM1 | MMP1 | MMP2 | MMP3 | MMP12 | TGFβ |
|---|---|---|---|---|---|---|
| - | 1 (±0.2) | 1 (±0.1) | 1 (±0.1) | 1 (±0.2) | 1 (±0.2) | 1 (±0.1) |
| REP | 32 (±4) | 3.6 (±1) | 18 (±8) | 48.2 (±8) | 13 (±4) | 151 (±9) |

**[Table 17]**

| Protein (%) | IL1α | IL1β | IL6 | IL10 | MCP1 | PAI1 |
|---|---|---|---|---|---|---|
| - | 100 (±4) | 100 (±3) | 100 (±6) | 100 (±4) | 100 (±3) | 100 (±7) |
| LX | 57 (±5) | 46 (±6) | 41 (±4) | 57 (±7) | 38 (±8) | 41 (±4) |
| | | | | | | |

| Protein (%) | VCAM1 | MMP1 | MMP2 | MMP3 | MMP12 | TGFβ |
|---|---|---|---|---|---|---|
| - | 100 (±5) | 100 (±2) | 100 (±8) | 100 (±4) | 100 (±3) | 100 (±4) |
| LX | 45 (±5) | 43 (±3) | 39 (±9) | 38 (±8) | 41 (±6) | 49 (±9) |

As a result, as shown in Table 16, when senescence was induced in cells, the expression levels of IL1α, IL1β, IL6, IL10, MCP1, PAIl, VCAM1, MMP1, MMP2, MMP3, MMP12, and TGFβ rapidly increased. Additionally, as shown in Table 17, when senescence-induced foreskin cells were treated with LX-112, the amount of each factor decreased. From this, it was confirmed that LX-112 effectively reduces the amount of various aging-related inflammation and fibrosis secretory factors (SASP) that increase with cell aging.

### Example 6. Induction of fibrosis by senescence-associated secretory phenotype (SASP) and inhibition of fibrosis by LX-112 in mice.

### Example 6-1. Induction of fibrosis in normal cells by senescence-associated secretory phenotype (SASP) expressed and secreted from senescent cells.

Culture medium (REP) of human foreskin cells in which natural aging was induced was obtained in the same manner as in Example 5. The foreskin cells that were not induced to age and were growing normally were treated with the medium for 1 day. Next, the mRNA amounts of aging-related fibrosis factors ACTA2, COL1A1, COLIA2, FN1, and SDF1, which are aging-related fibrosis factors, were measured by qRT-PCR using the same method as in Example 4, and are shown in Table 18. The control group (-) was foreskin cells that were not treated with the culture medium of senescent cells. The primers used were synthesized through bionics, and the sequences are shown in Table 19 below.

**[Table 18]**

| mRNA (Fold) | ACTA2 | COL1A1 | COL1A2 | FN1 | SDF1 |
|---|---|---|---|---|---|
| - | 1 (±0.3) | 1 (±0.2) | 1 (±0.1) | 1 (±0.2) | 1 (±0.2) |
| REP | 3.3 (±0.5) | 4.5 (±0.6) | 3.9 (±1) | 4.1 (±0.4) | 0.2 (±0.0) |

**[Table 191**

| Gene | Primer 1 | Seq. No. | Primer 2 | Seq. No. |
|---|---|---|---|---|
| ACTA2 | GTGAAGAAGAGGACAGCACTG | 7 | CCCATTCCCACCATCACC | 8 |
| COL1A1 | AAGGGACACAGAGGTTTCAGTGG | 9 | CAGCACCAGTAGCACCATCATTTC | 10 |
| COL1A2 | CTTGCAGTAACCTTATGCCTAGCA | 11 | CCCATCTAACCTCTCTACCCAGTCT | 12 |
| FN1 | TGTCAGTCAAAGCAAGCCCG | 13 | TTAGGACGCTCATAAGTGTCACCC | 14 |
| SDF1 | TGCCAGAGCCAACGTCAAG | 39 | CAGCCGGGCTACAATCTGAA | 40 |

As a result, as shown in Table 18, it was confirmed that the factors regulated by senescence-associated secretory factor (SASP) include ACTA2, COL1A1, COL1A2, FN1, and SDF1, which are regulators of fibrosis and pigmentation. From this, it was confirmed that the amounts of factors that induce fibrosis increases with aging. It was confirmed that the amount of SDF1, which suppresses pigmentation, decreases with aging.

### Example 6-2. Inhibition of fibrosis by LX-112 in mice

After 8-week-old male C57BL6J mice (purchased from KRIBB Laboratory Animal Resource Center) were adapted to the experimental environment for 1 week, the mice were randomized into an experimental group and a control group. To induce peritoneal fibrosis, 0.1% chlorhexidine gluconate (CHG, purchased from Sigma) was dissolved in 15% ethanol phosphate buffer solution and administered intraperitoneally at a concentration of 10 ml/kg every 2 days for 20 days. From day 9, LX-112 was mixed with 0.5% CMC-Na solution (purchased from Sigma) and administered intraperitoneally at a concentration of 70 mg/kg every day. The control group (-) was administered only the solution in the same volume. On the 20th day, paraffin sections of peritoneal tissue prepared by fixation in 4% paraformaldehyde solution were stained with H/E (Hematoxylin/Eosin) and trichrome, and the thickness of the peritoneal mesothelial cell layer was measured under a light microscope. Table 20 shows the results quantitatively showing the thickness of the peritoneal mesothelial cell layer in mice with peritoneal fibrosis induced by chlorhexidine gluconate (CHG) under conditions without and with LX-112 treatment.

**[Table 20]**

| | | | |
|---|---|---|---|
| Submesothelial Thickness (µm) | - | - | 10 (±1.1) |
| | CHG | - | 69.1 (±6.4) |
| | | LX | 43.1 (±10.4) |

As a result, as shown in Table 20, the thickness of the peritoneum increased due to chlorhexidine gluconate, and LX-112 suppressed and reduced the increased thickness. From this, it was confirmed that LX-112 effectively inhibits and treats fibrosis.

### Example 7. Elimination of senescent cells and related anti-aging activity by LX-112 in naturally aged mice

### Example 7-1. Establishment of a naturally aged mouse model

After 22-month-old female C57BL6J mice (purchased from KRIBB Laboratory Animal Resource Center) were adapted to the experimental environment for one week, the aged mice were randomized into an experimental group and a control group. 8-week-old mice that were not aged were used as controls. LX-112 was mixed with 0.5% CMC-Na solution (purchased from Sigma) and administered orally at a concentration of 70 mg/kg daily for 21 days. The control group was administered only the solution in the same volume. After drug administration was completed, the method described in each Example below was performed.

### Example 7-2. Decreased amounts of aging-related markers (p16, p21) by LX-112

The mouse was sacrificed, RNA was extracted from the organ tissue of each region, and qRT-PCR was performed using the synthesized cDNA. Specifically, in order to maintain the quality of RNA during the RNA extraction process, the tissue from each collected area was immediately cut into 30 mg to 50 mg pieces, placed in RNA stabilization solution (purchased from Thermo Fisher Scientific), and absorbed into the tissue for 1 day. Afterwards, it was stored at - 80 °C until use. The collected tissue was placed together with the lysate in a tube containing beads for tissue homogenization (purchased from MP Bio) and homogenized using tissue homogenization equipment (purchased from MP Bio). Total RNA was extracted from the homogenized tissues. The extraction method used an RNA extraction kit (PureLink RNA Mini Kit, purchased from Thermo Fisher Scientific) for soft tissues including liver, kidney, lung, and brain, or TRIzol Reagent (purchased from Thermo Fisher Scientific) for hard tissues including muscle, skin, fat, etc. The concentration of extracted total RNA was quantified using Nanodrop (purchased from Micro Digital) or SpectraMax iD3 Multi-Mode Microplate Reader (purchased from Molecular Device). Genomic DNA was removed from the extracted total RNA using a cDNA synthesis kit (purchased from Thermo Fisher Scientific), and cDNA was prepared. Using the synthesized cDNA, qRT-PCR was performed for each gene using the housekeeping gene β-actin as a standard, using Taqman Fast Abvanced Master Mix (purchased from Applied Biosystems), SFCgreen I qPCR Master Mix (purchased from SFC Probe), or AmfiSure qGreen qPCR Master Mix (purchased from GenDEPOT), and analyzed using the CFX Connect Real-Time PCR Detection System (purchased from Bio Rad). Table 21 shows the mRNA amount of p16, an aging marker, before (Young) and after (Old) inducing aging, and Table 22 shows the mRNA amount of p16, an aging marker, under conditions in which aging was induced and with and without LX-112 treatment. Table 22 calculates the amount of p16 mRNA after senescence was induced as 100% and compared it with the amount of p16 mRNA after treatment with LX-112. The primers used were synthesized through bionics, except that TaqMan primers (purchased from Thermo Fisher Scientific) were used for p16 and IL6. The sequences of the primers used are shown in Table 23 below.

**[Table 21]**

| p16 mRNA (Fold) | Liver | Kidney | Lung | Brain | eWAT | Muscle | Skin |
|---|---|---|---|---|---|---|---|
| Young | 1 (±0.7) | 1 (±0.6) | 1 (±0.1) | 1 (±0.3) | 1 (±0.4) | 1 (±0.5) | 1 (±0.0) |
| Old | 53.9 (±10.8) | 18.6 (±2.4) | 3.3 (±1) | 9 (±3.2) | 3.5 (±1.1) | 11 (±9.8) | 7.3 (±2.5) |

**[Table 22]**

| p16 mRNA (%) | Liver | Kidney | Lung | Brain | eWAT | Muscle | Skin |
|---|---|---|---|---|---|---|---|
| - | 100 (±4.8) | 100 (±3.8) | 100 (±5.7) | 100 (±3.8) | 100 (±9.7) | 100 (±8.4) | 100 (±1.5) |
| LX | 62 (±3.5) | 52 (±7.9) | 69 (±1) | 89 (±5) | 79 (±2) | 76 (±3.1) | 74 (±6.7) |

**[Table 23]**

| Gene | Primer 1 | Seq. No. | Primer 2 | Seq. No. |
|---|---|---|---|---|
| β-actin | CATCCGTAAAGACCTCTATGCCAAC | 15 | ATGGAGCCACCGATCCACA | 16 |
| p21 | GCAGATCCACAGCGATATCCA | 17 | AACAGGTCGGACATCACCAG | 18 |
| IL1α | TCCATAACCCATGATCTGGAA | 19 | TTGGTTGAGGGAATCATTCAT | 20 |
| IL1β | GTATGGGCTGGACTGTTTC | 21 | GCTGTCTGCTCATTCACG | 22 |
| CXCL1 | ACCGAAGTCATAGCCACACTC | 23 | CTCCGTTACTTGGGGACACC | 24 |
| CXCL10 | GCTGCCGTCATTTTCTGC | 25 | TCTCACTGGCCCGTCATC | 26 |
| MMP3 | GTTGGAGAACATGGAGACTTTGT | 27 | CAAGTTCATGAGCAGCAACCA | 28 |
| MMP12 | TGCACTCTGCTGAAAGGAGTCT | 29 | GTCATTGGAATTCTGTCCTTTCCA | 30 |
| MMP13 | AAGGGGATAACAGCCACTACAA | 31 | ACCAACATAAAAATTAAGCCAAATG | 32 |
| MCP1 | GCATCTGCCCTAAGGTCTTCA | 33 | GTGGAAAAGGTAGTGGATGCATT | 34 |
| COL1A1 | CAGTCGATTCACCTACAGCAC | 35 | TGGAGGGAGTTTACACG | 36 |
| Agtr1a | AAGGGCCATTTTGCTTTTCT | 37 | AACTCACAGCAACCCTCCAA | 38 |

As a result, as shown in Table 21, when mice aged, the mRNA amount of p16 rapidly increased in various organ tissues of in liver, kidney, lung, brain, epididymal fat (eWAT), muscle, skin, etc. Additionally, as shown in Table 22, when mice with induced aging were treated with LX-112, the amount of p16 mRNA decreased in various organ tissues. From this, it was confirmed that LX-112 effectively reduces the amount of p16, an aging marker, that increases in various organ tissues of mice by removing senescent cells that increase as mice age.

### Example 7-3. Decreased amount of aging-related marker (SA-β-gal) by LX-112

The mouse was sacrificed, tissue sections were prepared from the organ tissues of each region, and stained for an aging marker (SA-β-gal) in the same manner as Example 1-1. Specifically, fat was removed from the tissues of each area collected from mice and fixed in 4% formalin at room temperature for more than 24 hours. For cryopreservation, the cells were placed in 30% sucrose for more than 8 hours and then rapidly frozen in cryoprotectant OCT medium for cryosectioning. The rapidly frozen tissue was commissioned to Histoa, a company specializing in histopathology services, and frozen sections were produced. The prepared frozen tissue sections were cut to a thickness of 8 µm, sectioned on a slide cover, dried at room temperature for 30 minutes, and then stored at -80 °C until use. After staining the aging marker (SA-β-gal), the images were taken through an optical microscope, and the area of the stained area was calculated using the Image J program and quantified by normalizing it based on the total area. Table 24 shows the results of staining of SA-β-gal, an aging marker, before (Young) and after (Old) inducing aging, and Table 25 shows the results of staining of SA-β-gal, an aging marker, under conditions in which aging was induced and LX-112 was not treated and treated. Figure 2 is a photo result of staining of adipose tissue.

**[Table 24]**

| SA-β-gal (Fold) | Liver | Kidney | Lung | Skin | eWAT |
|---|---|---|---|---|---|
| Young | 1 (±0.5) | 1 (±0.6) | 1 (±0.2) | 1 (±0.3) | 1 (±0.1) |
| Old | 4.5 (±0.2) | 3 (±0.4) | 1.1 (±0.5) | 2.8 (±0.3) | 45.9 (±9.3) |

**[Table 25]**

| SA-β-gal (%) | Liver | Kidney | Lung | Skin | eWAT |
|---|---|---|---|---|---|
| - | 100 (±9) | 100 (±8) | 100 (±6) | 100 (±9) | 100 (±7) |
| LX | 58 (±6) | 55 (±17) | 67 (±6) | 68 (±9) | 38 (±7) |

As a result, as shown in Table 24, when mice aged, The staining area of SA-β-gal increased in various organ tissues such as liver, kidney, lung, skin, and epididymal fat (eWAT). Additionally, as shown in Table 25 and Figure 2, when senescence-induced mice were treated with LX-112, the staining area of SA-β-gal decreased in various organ tissues. From this, it was confirmed that LX-112 effectively reduces the amount of SA-β-gal, an aging marker, that increases in various organ tissues of mice by removing senescent cells that increase as mice age.

### Example 7-4. Reduction of the amount of aging-related degenerative marker (lipofuscin) by LX-112

Mice was sacrificed and tissue sections from muscle tissue were prepared in the same manner as in Example 7-3. Lipofuscin is a degenerative abnormal complex of proteins and lipids produced in cell lysosomes with aging and is a marker of aging-related degeneration. The amount of lipofuscin was measured as follows: the sections were stained using Sudan Black B (purchased from Sigma) and photographed, and then the area of the stained area was calculated using the Image J program and quantified by normalizing it based on the total area. Table 26 shows the staining area of lipofuscin, an aging-related degenerative marker, before and after inducing aging, and Table 27 shows the staining area of lipofuscin, an aging-related degenerative marker, under conditions in which aging was induced and LX-112 was not treated and treated.

**[Table 26]**

| | Muscle | |
|---|---|---|
| Lipofuscin (Fold) | Young | 1 (±0.2) |
| | Old | 1.5 (±0.4) |

**[Table 27]**

| | Muscle | |
|---|---|---|
| Lipofuscin (%) | - | 100 (±8) |
| | LX | 71 (±6) |

As a result, as shown in Table 26, as mice aged, the lipofuscin staining area increased in muscle tissue. Additionally, as shown in Table 27, when mice with induced aging were treated with LX-112, the lipofuscin staining area decreased in various tissues. From this, it was confirmed that LX-112 effectively reduces the amount of lipofuscin, an aging-related degenerative marker, that increases in muscle tissue of mice by removing senescent cells that increase as mice age.

### Example 7-5. Reduction of amounts of senescence-associated secretory phenotype (SASP) by LX-112

Serum collected from mice was stored at -80 °C until use. The amounts of IL6 and MCP1, which are aging-related inflammation secretory factors (SASPs), were measured in serum using an enzyme-linked immunosorbent assay (ELISA Kit, purchased from R&D Systems). Table 28 shows the results showing the amount of IL6 and MCP1, which are senescence-associated secretory factors (SASP), before and after inducing aging, and Table 29 shows the amount of IL6 and MCP1, which are senescence-associated secretory factors (SASP), under conditions in which aging was induced and LX-112 was not treated and treated.

**[Table 28]**

| Fold | IL6 | MCP1 |
|---|---|---|
| Young | 1 (±0.3) | 1 (±0.2) |
| Old | 4.2 (±0.3) | 2.8 (±1.1) |

**[Table 29]**

| % | IL6 | MCP1 |
|---|---|---|
| - | 100 (±29) | 100 (±27.1) |
| LX | 63 (±12.8) | 58 (±9.8) |

As a result, as shown in Table 28, when mice aged, the amounts of IL6 and MCP1 increased in the serum. Additionally, as shown in Table 29, when senescence-induced mice were treated with LX-112, the amounts of IL6 and MCP1 in the serum decreased. From this, it was confirmed that LX-112 effectively reduces the amount of IL6 and MCP1, which are aging-related inflammation secretory factors (SASP), that increase in the serum of mice by removing senescent cells that increase as mice age.

### Example 7-6. Reduction of the amount of aging-related inflammation and fibrosis secretory factors (SASP) by LX-112

Using cDNA synthesized in the same manner as in Example 7-2, qRT-PCR was performed and analyzed for MMP3, IL1β, MMP12, IL6, and MMP13, which are aging-related inflammation and fibrosis secretory factors (SASP). Table 30 shows the mRNA amounts of MMP3, IL1β, MMP12, IL6 and MMP13, which are aging-related inflammation and fibrosis secretion factors (SASP) before and after inducing aging, and Table 31 shows the mRNA amounts under conditions without and with LX-112 treatment after inducing aging. Table 31 calculates the mRNA amount of each factor after senescence was induced as 100% and compared it with the mRNA amount of each factor after treatment with LX-112.

**[Table 30]**

| mRNA (Fold) | Liver | Kidney | Lung | Muscle | Skin | | |
|---|---|---|---|---|---|---|---|
| | MMP3 | IL1β | MMP12 | IL6 | MMP3 | MMP12 | MMP13 |
| Young | 1 (±0.2) | 1 (±0.4) | 1 (±0.5) | 1 (±0.1) | 1 (±0.3) | 1 (±0.4) | 1 (±0.1) |
| Old | 2.2 (±0.2) | 2 (±0.2) | 3.6 (±1.9) | 2.9 (±1.3) | 1.5 (±0.2) | 10 (±6.3) | 1.3 (±0.6) |

**[Table 31]**

| mRNA (%) | Liver | Kidney | Lung | Muscle | Skin | | |
|---|---|---|---|---|---|---|---|
| | MMP3 | IL1β | MMP12 | IL6 | MMP3 | MMP12 | MMP13 |
| - | 100 (±5.1) | 100 (±9.9) | 100 (±15) | 100 (±12) | 100 (±38) | 100 (±8.4) | 100 (±49) |
| LX | 75 (±13) | 67 (±3.9) | 81 (±13) | 65 (±21) | 92 (±6) | 78 (±14) | 95 (±2) |

As a result, as shown in Table 30, when mice aged, the amounts of mRNA such as MMP3, IL1β, MMP12, IL6, and MMP13 increased. In addition, as shown in Table 31, when mice with induced aging were treated with LX-112, the mRNA amount of each aging-related inflammation and fibrosis secretory factor (SASP) decreased in various organ tissues. From this, it was confirmed that LX-112 effectively reduced the amounts of MMP3, IL1β, MMP12, IL6, and MMP13, aging-related inflammation and fibrosis secretory factors (SASP), which increased due to aging in various organ tissues of mice by eliminating senescent cells that increase as mice age.

### Example 7-7. Improvement of aging-related frailty (activity, endurance, muscle strength and grip strength, sarcopenia, lipoatrophy, etc.) by LX-112

To analyze aging-related senescence in mice, activity evaluation (open field test), endurance evaluation (wire hanging test), and muscle strength and grip strength test (grip strength test) were performed. Specifically, in the open field test, the mouse was placed in a white square box and the trajectory it moved for a certain period of time was measured. The measurement results were compared by analyzing the ratio of time spent in the central area to the total time. In the wire hanging test, the time the mouse could endure hanging on a wire was measured. In the muscle strength and grip test (grip strength test), the mouse was made to hold the grip strength measuring device (purchased from Jeongdo B&P) with its front paws by holding its tail, then pulled slightly until it missed, and the force applied to the measuring device just before missing was measured. Table 32 shows the quantitative results of activity, endurance, muscle strength, and grip strength, which are criteria for determining aging, before and after inducing aging, and Table 33 shows the results under conditions in which aging was induced and with or without LX-112 treatment. Table 33 calculates the amount of each evaluation after induced aging as 100% and compares it with the amount of each evaluation after treatment with LX-112.

**[Table 32]**

| | Field Test Time in Center (%) | Wire Hanging Test Time (Sec) | Grip Strength Test Strength (N) |
|---|---|---|---|
| Young | 13.1 (±3.93) | 64 (±10) | 1 (±0.05) |
| Old | 5.2 (±1.51) | 45 (±7) | 0.8 (±0.12) |

**[Table 33]**

| | Open Field Test Time in Center (%) | Wire Hanging Test Time (%) | Grip Sterenght Test Strength (%) | Muscle Mass (g) |
|---|---|---|---|---|
| - | 100 (±18) | 100 (±15) | 100 (±14) | 0.5 (±0.0) |
| LX | 121 (±9) | 119 (±10) | 132 (±11) | 0.7 (±0.1) |

As a result, as shown in Table 32, the mouse's activity, endurance, muscle strength, and grip strength decreased as aging was induced. Additionally, as shown in Table 33, treatment of mice with induced aging with LX-112 increased activity, endurance, muscle strength, and grip strength. In addition, in muscle tissue, p16 (Tables 21 and 22) and SA-β-gal (Tables 24 and 25), which are aging-related markers, lipofuscin (Tables 26 and 27), which is aging-related degenerative markers, and IL6 (Tables 28, 29, and 31), which is aging-related inflammation secretory factors (SASP) were also decreased by treatment with LX-112. From this, it was confirmed that LX-112 improves aging in mice by removing senescent cells that increase as mice age. Consistent with this improvement effect, it was confirmed from the results in Table 33 that LX-112 increased the weight of the muscle tissue of the quadriceps and gastrocnemius muscles and overall fat tissue in aged mice.

### Example 7-8. Improvement of kidney damage due to aging by LX-112

The amounts of urea and creatinine, markers of kidney damage, in serum collected from mice were measured by spectrophotometry using a kit (purchased from BioAssay Systems and Sigma). Additionally, the amount of Agtrla mRNA, a kidney damage marker, was measured by qRT-PCR as described in Example 7-2. Table 34 shows the quantitative results of urea, creatinine, and Agtrla mRNA, which are kidney damage markers, under conditions that induced aging and were not treated with LX-112 and treated with LX-112, respectively. The amount of each factor after inducing aging was calculated as 100% and compared with the amount of each factor after treatment with LX-112.

**[Table 34]**

| | Urea (%) | Creatinine (%) | Agtr1a mRNA (%) |
|---|---|---|---|
| - | 100 (±4) | 100 (±11) | 100 (±9) |
| LX | 69 (±6) | 87 (±7.8) | 64 (±7) |

As a result, as shown in Table 34, when mice with induced aging were treated with LX-112, the amounts of urea, creatinine, and Agtrla mRNA decreased. From this, it was confirmed that LX-112 improves kidney damage and dysfunction in mice by eliminating senescent cells that increase as mice age.

### Example 7-9. Improvement of skin aging due to aging by LX-112

Frozen sections of skin tissue were stained with H/E (Hematoxylin/Eosin), and the thickness of the dermis was observed under an optical microscope. Additionally, the mRNA amount of collagen (COL1A1) was measured by qRT-PCR as described in Example 7-2. Table 35 quantitatively shows the degree of skin aging according to the thickness of the skin (dermal) and the amount of collagen (COL1A1) mRNA in mice in which aging was naturally induced, under conditions without and with LX-112 treatment.

**[Table 35]**

| | Dermal Thickness (µm) | Collagen mRNA (Fold) |
|---|---|---|
| - | 221 (±22.1) | 1 (±0.2) |
| LX | 265 (±17.2) | 2.3 (±0.4) |

As a result, as shown in Table 35, when mice with induced aging were treated with LX-112, skin thickness and collagen amount increased. In addition, in skin tissue, aging-related markers p16 (Tables 21 and 22), SA-β-gal (Tables 24 and 25), and MMP1, MMP12, and MMP13 (Table 31), aging-related inflammation and fibrosis secretion factor (SASP) and collagen decomposing enzymes, were also decreased by treatment with LX-112. From this, it was confirmed that LX-112 improves skin aging in mice by removing senescent cells that increase as mice age.

### Example 8. Removal of senescent cells and related anti-aging activity by LX-112 in chemotherapy induced aged mice

### Example 8-1. Establishment of a chemotherapy induced aged mice

After adapting 8-week-old male C57BL6J mice (purchased from KRIBB Laboratory Animal Resource Center) to the experimental environment for 1 week, doxorubicin, an anticancer drug, was administered intraperitoneally at a concentration of 10 mg/kg, and mice that were induced to age for more than 10 days were assigned to an experimental group and a control group. LX-112 was mixed with 0.5% CMC-Na solution (purchased from Sigma) and administered orally at a concentration of 70 mg/kg daily for 21 days. The control group was administered only the solution in the same volume. After drug administration was completed, the method described in each Example below was performed.

### Example 8-2. Reduction of amounts of aging-related markers (p16, p21) by LX-112

The mouse was sacrificed in the same manner as in Example 7-2, RNA was extracted from the organ tissue of each region, and qRT-PCR was performed and analyzed using the synthesized cDNA. Table 36 shows the amounts of the aging-related markers p16 and p21 mRNA before and after inducing aging by treatment with the anticancer drug, and Table 37 shows the amounts of the aging-related markers p16 and p21 under conditions in which aging was induced by treatment with the anticancer drug and without and with LX-112 treatment. Table 37 calculates the mRNA amounts of p16 and p21 after aging was induced by treatment with the anticancer drug as 100% and compared them with the mRNA amounts of p16 and p21 after treatment with LX-112.

**[Table 36]**

| | | Liver | Kidney | eWAT |
|---|---|---|---|---|
| p16 mRNA (Fold) | - | 1 (±0.3) | 1 (±0.3) | 1 (±0.3) |
| | DOX | 2.6 (±0.8) | 1.3 (±0.3) | 3.2 (±1.2) |
| p21 mRNA (Fold) | - | 1 (±0.6) | 1 (±0.4) | 1 (±0.2) |
| | DOX | 3.8 (±2.3) | 2.6 (±2.1) | 3.9 (±3) |

**[Table 37]**

| | | Liver | Kidney | eWAT |
|---|---|---|---|---|
| p16 mRNA (%) | - | 100 (±24) | 100 (±9.89) | 100 (±9.8) |
| | LX | 70 (±8.3) | 75 (±14.81) | 72 (±18.75) |
| p21 mRNA (%) | - | 100 (±18.58) | 100 (±25.8) | 100 (±18.51) |
| | LX | 78 (±11.5) | 83.8 (±9.5) | 68.4 (±15.1) |

As a result, as shown in Table 36, when mice aged by treatment with the anticancer drug, the amounts of p16 and p21 mRNA increased in various organ tissues such as liver, kidney, and epididymal fat (eWAT). Additionally, as shown in Table 37, when mice whose aging was induced by the anticancer drug were treated with LX-112, the amounts of p16 and p21 mRNA were decreased in various organ tissues. From this, it was confirmed that LX-112 effectively reduces the amounts of aging-related markers p16 and p21 that increase in various organ tissues of mice by removing senescent cells that increase in mice due to treatment with the anticancer drug.

### Example 8-3. Reduction of amount of aging-related marker (SA-β-gal) by LX-112

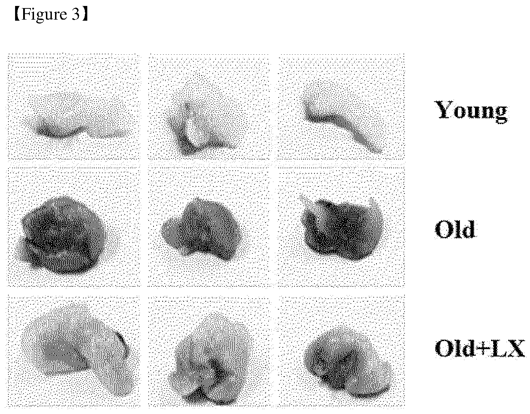

Mice were sacrificed in the same method in described in Example 7-3, and tissue sections were prepared from the organ tissues of each region. SA-β-gal, an aging marker was stained. Table 38 shows the stained area of SA-β-gal, an aging marker, before and after inducing aging by treatment with the anticancer drug, and Table 39 shows the stained area of SA-β-gal, an aging marker, under conditions in which aging was induced by treatment with the anticancer drug and without and with LX-112 treatment. Figure 3 is a photographic result of staining of adipose tissue.

**[Table 38]**

| SA-β-gal (Fold) | Liver | Kidney | Lung | eWAT |
|---|---|---|---|---|
| - | 1 (±0.6) | 1 (±0.9) | 1 (±0.2) | 1 (±0.1) |
| DOX | 3.7 (±0.9) | 4.1 (±1.5) | 1.3 (±0.4) | 4.2 (±0.9) |

**[Table 39]**

| SA-β-gal (%) | Liver | Kidney | Lung | eWAT |
|---|---|---|---|---|
| - | 100 (±7.2) | 100 (±10.2) | 100 (±6.2) | 100 (±8.4) |
| LX | 67.4 (±9.9) | 69.2 (±10.2) | 64.2 (±9.2) | 51.4 (±13.4) |

As a result, as shown in Table 38, when mice are aged by treatment with the anticancer drug, the stained area of SA-β-gal was decreased in various organ tissues such as liver, kidney, lung, and epididymal fat (eWAT). In addition, as shown in Table 39 and Figure 3, when mice whose aging was induced by treatment with the anticancer drug were treated with LX-112, the stained area of SA-β-gal decreased in various organ tissues. From this, it was confirmed that LX-112 effectively reduces the amount of SA-β-gal, an aging marker, that increases in various organ tissues of mice by removing senescent cells that increase with the aging of mice by treatment with the anticancer drug.

### Example 8-4. Reduction of amount of aging-related degenerative marker (lipofuscin) by LX-112

Mice were sacrificed in the same manner as in Example 7-4, and tissue sections were prepared from muscle tissue. Lipofuscin, an aging-related degenerative marker was stained. Table 40 shows the stained area of lipofuscin, an aging-related degenerative marker, before and after inducing aging by treatment with the anticancer drug, and Table 41 shows the stained area of lipofuscin, an aging-related degenerative marker, under conditions in which aging was induced by treatment with the anticancer drug and without and with LX-112 treatment.

**[Table 40]**

| | Muscle | |
|---|---|---|
| Lipofuscin (Fold) | - | 1 (±0.2) |
| | DOX | 2.2 (±0.5) |

**[Table 41]**

| | Muscle | |
|---|---|---|
| Lipofuscin (%) | - | 100 (±19.3) |
| | LX | 77.1 (±8) |

As a result, as shown in Table 40, when mice aged by treatment with the anticancer drug, the stained area of lipofuscin in muscle tissue increased. Additionally, as shown in Table 41, when mice whose aging was induced by treatment with the anticancer drug were treated with LX-112, the stained area of lipofuscin in muscle tissue decreased. From this, it was confirmed that LX-112 effectively reduces the amount of lipofuscin, an aging-related degenerative marker, that increases in the muscle tissue of mice by removing senescent cells that increase as mice age through treatment with the anticancer drug.

### Example 8-5. Reduction of amount of aging-related inflammation secretory phenotype (SASP) by LX-112

The amount of IL6, an aging-related inflammation secretory factor (SASP), was measured in serum collected from mice in the same manner as in Example 7-5 using an enzyme-linked immunosorbent assay (ELISA Kit, purchased from R&D Systems). Table 42 shows the amount of IL6, the aging-related inflammation secretory factor (SASP), before and after inducing aging by treatment with the anticancer drug, and Table 43 shows the amount of IL6, the aging-related inflammation secretory factor (SASP), in conditions without and with LX-112 treatment after inducing aging by treatment with the anticancer drug.

**[Table 42]**

| | | |
|---|---|---|
| IL6 (Fold) | - | 1 (±1.1) |
| | DOX | 11.7 (±0.5) |

**[Table 43]**

| | | |
|---|---|---|
| IL6 (%) | - | 100 (±38.4) |
| | LX | 69.8 (±12.2) |

As a result, as shown in Table 42, when mice aged by treatment with the anticancer drug, the amount of IL6 in the serum increased. Additionally, as shown in Table 43, when mice whose aging was induced by treatment with the anticancer drug were treated with LX-112, the amount of IL6 in the serum decreased. From this, it was confirmed that LX-112 effectively reduces the amount of IL6, an aging-related inflammation secretory factor (SASP), that increases in the serum by removing senescent cells that increase with aging in mice by treatment with the anticancer drug.

### Example 8-6. Reduction of amount of aging-related inflammation and fibrosis secretory factors (SASP) by LX-112

Using cDNA synthesized in the same manner as Example 7-6, qRT-PCR was performed and analyzed for aging-related inflammation and fibrosis secretory factors (SASP) such as IL6, CXCL1, CXCL10, and IL1β. Table 44 shows the mRNA amount of IL6, CXCL1, CXCL10, and IL1β, aging-related inflammation and fibrosis secretory factors (SASP), before and after inducing aging by treatment with the anticancer drug, and Table 45 shows the mRNA amount of IL6, CXCL1, CXCL10, and IL1β, aging-related inflammation and fibrosis secretory factors (SASP), under conditions in which aging was induced by treatment with the anticancer drug and without and with LX-112 treatment. Table 45 calculates the mRNA amount of each factor after aging was induced by treatment with the anticancer drug as 100% and compared it with the mRNA amount of each factor after treatment with LX-112.

**[Table 44]**

| mRNA (Fold) | | IL6 | CXCL1 | CXCL10 | IL1β |
|---|---|---|---|---|---|
| Liver | - | 1 (±0.5) | 1 (±0.6) | 1 (±0.3) | 1 (±0.5) |
| | DOX | 1.8 (±0.3) | 3 (±0.9) | 2.6 (±0.9) | 1.6 (±0.3) |
| eWAT | - | 1 (±0.5) | 1 (±0.2) | 1 (±0.4) | 1 (±0.5) |
| | DOX | 3.2 (±0.3) | 1.3 (±0.4) | 3 (±1.2) | 1.6 (±0.2) |

**[Table 45]**

| mRNA (%) | | IL6 | CXCL1 | CXCL10 | IL1β |
|---|---|---|---|---|---|
| Liver | - | 100 (±35.4) | 100 (±38.5) | 100 (±13.6) | 100 (±28.4) |
| | LX | 42 (±23.5) | 68 (±21.1) | 84 (±11.5) | 42 (±35.2) |
| eWAT | - | 100 (±38.4) | 100 (±8.5) | 100 (±38.7) | 100 (±78.7) |
| | LX | 48.6 (±12.5) | 94.8 (±1.2) | 74 (±11) | 38 (±37) |

As a result, as shown in Table 44, when mice aged by treatment with the anticancer drug, the mRNA amounts of IL6, CXCL1, CXCL10, and IL1β increased in organ tissues such as liver and epididymal fat (eWAT). In addition, as shown in Table 45, when mice whose aging was induced by treatment with the anticancer drug were treated with LX-112, the mRNA amount of each aging-related inflammation and fibrosis secretory factor (SASP) was decreased in various organ tissues. From this, it was confirmed that LX-112 removes senescent cells that increase as mice age due to treatment with the anticancer drug, thereby effectively reducing the increased amounts of the aging-related inflammation and fibrosis secretory factors (SASP), such as IL6, CXCL1, CXCL1, and IL1β in various organ tissues of mice.

### Example 8-7. Improvement of fatigue, weakness, and weight loss due to anticancer drug by LX-112

In the same way as Example 7-7, in order to analyze fatigue and weakness that occur as a side effect due to treatment with anticancer drugs in mice, activity evaluation (open field test), endurance evaluation (wire hanging test), and muscle strength and grip strength test (grip strength test) were performed. Table 46 shows the quantitative results of activity, endurance, muscle strength and grip strength, criteria for determining frailty, before and after inducing aging by treatment with the anticancer drug, and Table 47 shows them under conditions in which aging was induced by treatment with the anticancer drug and without and with LX-112 treatment. Table 48 shows body weight results before and after inducing aging by treatment with the anticancer drug, and under conditions in which aging was induced by treatment with the anticancer drug and treated with LX-112. In Table 47, the amount of each evaluation after aging was induced by treatment with the anticancer drug was calculated as 100% and compared it with the amount of each evaluation after treatment with LX-112.

**[Table 46]**

| | Open Field Test Time in Center (%) | Wire Hanging Test Time (Sec) | Grip Strength Test Strength (N) |
|---|---|---|---|
| - | 11.9 (±4.2) | 69 (± 7) | 1.3 (±0.1) |
| DOX | 4.9 (±2.2) | 32 (±11) | 0.7 (±0.1) |

**[Table 47]**

| | Open Field Test Time in Center (%) | Wire Hanging Test Time (%) | Grip Strength Test Strength (%) |
|---|---|---|---|
| - | 100 (±22) | 100 (±25) | 100 (±29) |
| LX | 129 (±15) | 127 (±15) | 122 (±18) |

**[Table 48]**

| | | | |
|---|---|---|---|
| Mean Body Weight (g) | - | DOX | DOX+LX |
| | 26.5 (±5.7) | 21.3 (±2.6) | 22.8 (±3.1) |

As a result, as shown in Tables 46 to 48, when mice whose aging was induced by treatment with the anticancer drug were treated with LX-112, activity, endurance, muscle strength, and grip strength increased, and weight loss was improved. In addition, lipofuscin (Tables 40 and 41), an aging-related degenerative marker in muscle tissue, was also decreased by treatment with LX-112. From this, it was confirmed that LX-112 improves fatigue and weakness in mice that occur as a side effect of anticancer drug treatment by removing senescent cells that increase as mice age. Consistent with this improvement effect, it was confirmed from the results in Table 48 that LX-112 increased the body weight of aged mice by treatment with anticancer drugs.

### Example 8-8. Improvement of liver damage due to anticancer drug by LX-112

In the same manner as in Example 7-5, the amounts of AST (aspartate aminotransferase) and ALT (alanine transaminase) released into the blood during liver damage were measured by spectrophotometry in the serum collected from mice using a kit (purchased from Sigma) according to the method described in the manual. Table 49 shows the amounts of AST and ALT, liver damage markers, before and after inducing aging by treatment with the anticancer drug, and Table 50 shows them under conditions in which aging was induced by treatment with the anticancer drug and without and with LX-112 treatment. Table 50 calculates the amount of each factor after aging was induced by treatment with the anticancer drug as 100% and compared it with the amount of each factor after treatment with LX-112.

**[Table 49]**

| Fold | AST | ALT |
|---|---|---|
| - | 1 (±0.1) | 1 (±0.1) |
| DOX | 1.3 (±0.0) | 1.4 (±0.5) |

**[Table 50]**

| % | AST | ALT |
|---|---|---|
| - | 100 (±14.4) | 100 (±9.2) |
| LX | 78 (±17.3) | 72.2 (±14.1) |

As a result, as shown in Table 49, when mice aged by treatment with the anticancer drug, the amounts of AST and ALT in the serum increased. Additionally, as shown in Table 50, when mice whose aging was induced by treatment with the anticancer drug were treated with LX-112, the amounts of AST and ALT in the serum decreased. From this, it was confirmed that LX-112 improves liver damage and dysfunction in mice by eliminating senescent cells that increase as mice age by treatment with the anticancer drug.

### Example 8-9. Improvement of kidney damage due to anticancer drug by LX-112

The amounts of urea and creatinine, markers of kidney damage, in serum collected from mice were measured using spectrophotometry in the same manner as in Example 7-8. Table 51 shows the quantitative results of urea, creatinine, and Agtrla mRNA, which are kidney damage markers, under conditions in which aging was induced by treatment with the anticancer drug and with or without LX-112 treatment. The amount of each factor after aging was induced by treatment with the anticancer drug was calculated as 100% and compared it with the amount of each factor after treatment with LX-112.

**[Table 51]**

| % | Urea | Creatinine | Agtr1a mRNA |
|---|---|---|---|
| - | 100 (±0.2) | 100 (±5) | 100 (±6) |
| LX | 91.2 (±1.2) | 89 (±4) | 75 (±9) |

As a result, as shown in Table 51, when mice whose aging was induced by treatment with the anticancer drug were treated with LX-112, the amounts of urea, creatinine, and Agtrla mRNA decreased. From this, it was confirmed that LX-112 improves kidney damage and dysfunction in mice by eliminating senescent cells that increase as mice age through treatment with the anticancer drug.

### Example 9. Removal of senescent cells and related anti-aging activity by LX-112 in aging mice induced by obesity

### Example 9-1. Establishment of a mouse model of obesity-induced aging

15-week-old male C57BL6J mice (purchased from Jackson Laboratories), whose obesity was induced by a high-fat diet, were adapted to the experimental environment for one week, and then the mice were randomized into an experimental group and a control group. LX-112 was mixed with 0.5% CMC-Na solution (purchased from Sigma) and administered orally at a concentration of 70 mg/kg daily for 21 days. The control group was administered only the solution in the same volume. After drug administration was completed, the method described in each Example below was performed. Mice were sacrificed and the weight of adipose tissue in each area was measured. Table 52 quantitatively shows the weights of body weight, each adipose tissue, which is epididymal fat (eWAT), inguinal fat (iWAT), subcutaneous fat (asWAT), mesenteric fat (mWAT), or brown fat between the shoulder blades (iBAT), before and after inducing obesity in mice with high-fat diet (DIO).

**[Table 52]**

| | Body Weight (g) | eWAT (mg) | iWAT (mg) | asWAT (mg) | mWAT (mg) | iBAT (mg) |
|---|---|---|---|---|---|---|
| - | 20.7 (±0.1) | 169.1 (±70.1) | 188.4 (±2.1) | 205.6 (±88.4) | 96.6 (±19.1) | 100.5 (±35.9) |
| DIO | 41.5 (±3.8) | 2437.4 (±161.4) | 1471.9 (±162.8) | 1269.6 (±24.7) | 1018.3 (±481.5) | 344.7 (±140.7) |

As a result, as shown in Table 52, the weight of fatty tissue in various areas increased along with body weight. From this, it was confirmed that obesity was induced.

### Example 9-2. Reduction of amounts of aging-related markers (p16, p21) by LX-112

Mice were sacrificed in the same manner as in Example 7-2, RNA was extracted from the organ tissue of each region, and qRT-PCR (quantitative real-time PCR) was performed and analyzed using the synthesized cDNA. Table 53 shows the mRNA amounts of p 16 and p21, aging-related markers, before and after inducing aging by obesity, and Table 54 shows them under conditions in which aging was induced by obesity and without and with LX-112 treatment. Table 54 calculates the mRNA amounts of p16 and p21 after aging induced by obesity as 100% and compares them with the mRNA amounts of p16 and p21 after treatment with LX-112.

**[Table 53]**

| Fold | | Liver | Muscle | Lung | Skin | eWAT | iWAT | asWAT | iBAT |
|---|---|---|---|---|---|---|---|---|---|
| p16 mRNA | - | 1 (±0.1) | 1 (±0.6) | 1 (±0.3) | 1 (±0.3) | 1 (±0.4) | 1 (±0.0) | 1 (±0.2) | 1 (+1) |
| | DIO | 1.2 (±0.5) | 2.2 (±0.2) | 1.1 (±0.2) | 1.3 (±0.5) | 3.3 (±2.2) | 1.2 (±0.1) | 1.2 (±0.2) | 2.1 (±1) |
| p21 mRNA | - | 1 (±0.7) | 1 (±0.1) | 1 (±0.2) | 1 (±0.1) | 1 (±0.4) | 1 (±0.5) | 1 (±0.8) | 1 (±0.1) |
| | DIO | 20.4 (±1.9) | 1.2 (±0.1) | 1.1 (±0.2) | 1.2 (±0.0) | 1.7 (±0.3) | 2.9 (±0.2) | 1.2 (±0.3) | 1.4 (±1.1) |

**[Table 54]**

| % | | Liver | Muscle | Lung | Skin | eWAT | iWAT | asWEIT | iBAT |
|---|---|---|---|---|---|---|---|---|---|
| p16 | - | 100 | 100 | 100 | 100 | 100 | 100 | 100 | 100 |
| mRNA | | (±3.4) | (±4.7) | (±5.5) | (±1.6) | (±3) | (±3.4) | (±1.5) | (±1.3) |
| | LX | 71.3 (±4.5) | 77.8 (±7.9) | 81 (±3) | 79.1 (±3) | 61.2 (±4.3) | 79.1 (±7) | 84.1 (±3.3) | 89.2 (±2.6) |
| p21 mRNA | - | 100 (±7.9) | 100 (±9.4) | 100 (±9.6) | 100 (±9.4) | 100 (±8.2) | 100 (±4.5) | 100 (±6.2) | 100 (±4.3) |
| | LX | 62.9 (±9.7) | 73.2 (±7.2) | 79.3 (±8.4) | 78.1 (±9.2) | 67.2 (±6.8) | 71.2 (±3.3) | 82.1 (±7.7) | 70 (±6.1) |

As a result, as shown in Table 53, when mice age due to aging, the mRNA amounts of p16 and p21 increased in various organ tissues, such as liver, muscle, lung, skin, epididymal fat (eWAT), inguinal fat (iWAT), and subcutaneous fat (asWAT) and interscapular brown fat (iBAT). Additionally, as shown in Table 54, when aging-induced mice were treated with LX-112, the mRNA levels of p16 and p21 decreased in various organ tissues. From this, it was confirmed that LX-112 effectively reduces the amounts of aging-related markers p16 and p21 that increase in various organ tissues of mice by removing senescent cells that increase in mice due to aging due to obesity.

### Example 9-3. Reduction of amounts of aging-related inflammation secretory factor (SASP) by LX-112

Using the same method as in Example 7-5, the amount of IL6 and activin A, which are aging-related inflammation secretory factors (SASP), were measured in serum collected from mice using an enzyme-linked immunosorbent assay (ELISA Kit, purchased from R&D Systems). Table 55 shows the amounts of IL6 and activin A, which are aging-related inflammation secretory factors (SASP), before and after inducing aging by obesity, and Table 56 shows them under conditions in which aging was induced by obesity and without and with LX-112 treatment.

**[Table 55]**

| Fold | IL6 | Activin A |
|---|---|---|
| - | 1 (±0.6) | 1 (±0.5) |
| DIO | 2 (±0.5) | 2.9 (±1.2) |

**[Table 56]**

| % | IL6 | Activin A |
|---|---|---|
| - | 100 (±9.2) | 100 (±13.2) |
| LX | 51.8 (±14.2) | 64.5 (±12.3) |

As a result, as shown in Table 55, when mice aged due to obesity, the amounts of IL6 and activin A in the serum increased. Additionally, as shown in Table 56, when mice with aging induced by obesity were treated with LX-112, the amounts of IL6 and activin A in the serum decreased. From this, it was confirmed that LX-112 effectively reduces the amount of IL6 and activin A, which are aging-related inflammation secretory factors (SASP), that increase in the serum of mice by removing senescent cells that increase as mice age due to obesity.

### Example 9-4. Improvement of anxiety, depression, and lethargy due to obesity by LX-112

It has been revealed that anxiety and depression caused by obesity have no causal relationship with increased body weight and are caused by the accumulation of senescent cells. Accordingly, the open field test was performed to analyze lethargy due to anxiety and depression caused by obesity in mice using the same method as Example 7-7. Table 57 shows the quantitative results of activity, which is a criterion for determining lethargy due to anxiety and depression, before and after inducing aging by obesity, and Table 58 shows them under conditions in which aging was induced by obesity and without and with LX-112 treatment. Table 58 calculates the amounts of activity after aging induced by obesity as 100% and compares it with the amount of activity evaluation after treatment with LX-112.

**[Table 57]**

| | | |
|---|---|---|
| Open Field Test Time in Center (%) | - | 7.3 (±0.4) |
| | DIO | 2.8 (±0.0) |

**[Table 58]**

| | | |
|---|---|---|
| Open Field Test Time in Center (%) | - | 100 (±21) |
| | LX | 137.5 (±15) |

As a result, as shown in Table 57, activity decreased in mice whose aging was induced by obesity. Additionally, as shown in Table 58, when mice with aging induced by obesity were treated with LX-112, activity increased. From this, it was confirmed that LX-112 improves anxiety, depression, and lethargy in mice caused by obesity by eliminating senescent cells that increase as mice age.

### Example 9-5. Improvement of fatty liver due to obesity by LX-112

Mice were sacrificed in the same manner as in Example 7-4, sections were prepared from the liver tissue, and fat accumulation was stained with Oil Red O. Table 59 shows the stained area of Oil Red O, which is a criterion for determining fatty liver, before and after inducing aging by obesity, and Table 60 shows it under conditions in which aging was induced by obesity and without or with LX-112 treatment. Table 60 calculates the stained area of Oil Red O after aging induced by obesity as 100% and compares it with the stained area of Oil Red O after treatment with LX-112.

**[Table 59]**

| | | |
|---|---|---|
| Oil Red O (Fold) | - | 5.8 (±1.4) |
| | DIO | 47.7 (±4.2) |

**[Table 60]**

| | | |
|---|---|---|
| Oil Red O (%) | - | 100 (±11.3) |
| | LX | 74 (±7.3) |

As a result, as shown in Table 59, when mice aged due to obesity, the stained area of Oil Red O increased. Additionally, as shown in Table 60, when mice with aging induced by obesity were treated with LX-112, the stained area of Oil Red O decreased. From this, it was confirmed that LX-112 reduces the amount of fat accumulated in the liver tissue of mice by removing senescent cells that increase as mice age due to obesity.

### Example 10. Study on the mechanism of removal of senescent human cells by LX-112

Existing senescent cell removal agents such as ABT-263/737 are widely known to induce the death of senescent cells through apoptosis. Accordingly, the death mechanisms of senescent cells were compared and analyzed to determine whether LX-112 of the present invention also exhibits the effect of killing senescent cells through the same mechanism. In particular, because of the serious side effects of ABT-263/737, it is important to develop new compounds that eliminate senescent cells through different death mechanisms.

In the same manner as in Example 1, QVD-Oph (purchased from APExBIO), a caspase inhibitor used as an apoptosis inhibitor, was applied at a concentration of 20 µM to human foreskin cells (source: Chungnam National University College of Medicine) in which aging was induced by treatment with doxorubicin for 20 days. After 4 hours, the cells were treated with ABT-263 (purchased from APExBIO) or LX-112 (purchased from BOC) at a concentration of 1 µM or 10 µM, respectively, and 3 days later cell viability was measured with CellTiter-Glo Luminescent Cell Viability Assay (purchased from Promega). The results are shown in Table 61 below.

**[Table 61]**

| | | | |
|---|---|---|---|
| Survial (%) | | - | 100 (±2.3) |
| | | QVD | 105.1 (±4.7) |
| | ABT-263 | - | 23.8 (±5.5) |
| | | QVD | 98.4 (±2.6) |
| | LX-112 | - | 39.5 (±3.7) |
| | | QVD | 53.9 (±2.2) |

As a result, the caspase inhibitor completely inhibited the death of senescent cells by ABT-263, while it did not effectively inhibit the death of senescent cells by LX-112. From this, it was confirmed that LX-112 induces the death of senescent cells through a completely different mechanism from ABT-263 since caspase inhibitors inhibit apoptosis, a representative cell death mechanism induced by many agents such as ABT-263.

To further verify this fact, human foreskin cells in which aging was induced by treatment with doxorubicin in the same manner as in Example 1 were treated with ABT-263 or LX-112 at a concentration of 1 µM or 10 µM, respectively, to induce cell death. After 6 hours, the amounts of HMGB1 and HMGN1 secreted into the cell culture medium were measured using an enzyme-linked immunosorbent assay (ELISA Kit, purchased from AssayGenie). The results are shown in Table 62 below.

**[Table 62]**

| | | |
|---|---|---|
| HMGB1 (pg/ml) | - | 494.6 (±29.9) |
| | ABT-263 | 462.7 (±21.6) |
| | LX-112 | 454.9 (±22.4) |
| HMGN1 (pg/ml) | - | 90.1 (±6) |
| | ABT-263 | 105.3 (±13.5) |
| | LX-112 | 262.45 (±23.4) |

As a result, when comparing the substances secreted in the culture medium from dying senescent cells, HMGN1 was greatly increased in the case of LX-112, while there was no significant change in the case of ABT-263. HMGB1 showed little change in both ABT-263 and LX-112. From this, it was confirmed that the substances secreted from senescent cells when they die by ABT-263 and LX-112 are also different. From this, it was confirmed again that LX-112 induces the death of senescent cells through a completely different mechanism from apoptosis, which is the cell death mechanism of ABT-263.

Furthermore, the pattern in which senescent cells are killed by LX-112 and the pattern in which the amount of HMGN1 secreted from dying senescent cells increases are compared and shown in Table 63 below.

**[Table 63]**

| LX-112 | 0 µM | 1.1 µM | 3.3 µM | 10 µM |
|---|---|---|---|---|
| Survival (%) | 100 (±4) | 67.2 (±7) | 27 (±4) | 1 (±0.4) |
| HMGN1 (pg/ml) | 90.1 (±6) | 129.7(±9.3) | 189.9(±11.4) | 262.45 (±23.4) |

As a result, the increase in the amount of HMGN1 and the degree of cell death were proportional to each other. From this, it was confirmed that the degree to which senescent cells are killed by LX-112 can be effectively analyzed by measuring the amount of HMGN1. In particular, unlike the method of crushing cells after a set time and ultimately measuring cell viability using the CellTiter-Glo Luminescent Cell Viability Assay (purchased from Promega), there is a great advantage in that the cells are cultured in the medium without crushing the cells, and cell viability can be analyzed multiple times in real time by frequently measuring the amount of HMGN1.

Based on the above-mentioned various differences, it was confirmed that LX-112 kills senescent cells through a unique mechanism different from ABT-263, which is known as a senescent cell remover. Furthermore, with regard to the characteristics of HMGN1 that causes cell death and inflammation, it was confirmed in Example 13 below that substances secreted by senescent cells killed by LX-112 induce the death of senescent cells.

### Example 11. Screening of various compounds that remove senescent human cells through a different mechanism from existing senescent cell removers

Based on the results of Example 10, compounds that induce the death of senescent cells through a mechanism similar to LX-112, unlike existing senescent cell removers such as ABT-263, were screened based on HMGN1. In the same manner as in Example 1, cells were treated with doxorubicin or control (DMSO) and cultured with 30 µM test compounds (except that, LX-305, LX-501, LX-506, LX-1302, LX-1603, LX-1703, LX-2005, LX-2603, LX-2701, LX-2702, LX-2703, LX-2705, LX-2901, LX-3106, and LX-3301 were 90 µM; LX-1103, LX-1701, LX-2007, LX-2805, and LX-3109 were 270 µM; and LX-3401 was 20 ng/ml). The following test compounds were synthesized by and purchased from Medchemexpress, Selleckchem, Cayman, Medkoo, Sigma, Molport, Enzo, TCI, Santa Cruz, REONSIMI, Abeam, Alfa Aesar, APExBIO, BOCSCI, Merck, Thermo Fisher Scientific, R&D Systems, ChemFaces, Aobious, etc. In the same manner as Example 10, the amount of HMGN1 secreted in the cell culture was measured using an enzyme-linked immunosorbent assay (ELISA Kit, purchased from AssayGenie). In cells in which senescence was not induced by treatment with the control group (DMSO), the amount of HMGN1 above the effective value (sensitivity: 35 pg/ml), analysis limit, was not detected in both untreated and treated conditions with test compounds. On the other hand, in cells where senescence was induced by treatment with doxorubicin, the amount of HMGN1 was measured at 80 to 90 pg/ml. This amount was calculated as 100%, and the amount of HMGN1 was measured under conditions in which aged cells were treated with test compounds, and the results are shown in Table 64 below.

**[Table 64]**

| | HMG N1 | | HMG N1 | | HMGN1 | | HM GN1 | | HMG N1 |
|---|---|---|---|---|---|---|---|---|---|
| DMSO | 100 | LX-201 | 291 | LX-202 | 287 | LX-305 | 199 | LX-307 | 258 |
| LX-309 | 283 | LX-401 | 263 | LX-402 | 298 | LX-403 | 275 | LX-405 | 285 |
| LX-406 | 302 | LX-407 | 299 | LX-411 | 300 | LX-412 | 293 | LX-413 | 287 |
| LX-415 | 295 | LX-416 | 293 | LX-417 | 294 | LX-418 | 284 | LX-419 | 274 |
| LX-420 | 286 | LX-421 | 293 | LX-422 | 294 | LX-431 | 312 | LX-432 | 304 |
| LX-435 | 284 | LX-436 | 299 | LX-437 | 309 | LX-501 | 168 | LX-502 | 274 |
| LX-503 | 284 | LX-505 | 299 | LX-506 | 169 | LX-601 | 293 | LX-602 | 248 |
| LX-603 | 263 | LX-701 | 277 | LX-702 | 278 | LX-801 | 265 | LX-802 | 301 |
| LX-901 | 257 | LX-902 | 268 | LX-903 | 324 | LX-905 | 318 | LX-907 | 297 |
| LX-909 | 267 | LX-910 | 278 | LX-911 | 294 | LX-912 | 317 | LX-913 | 283 |
| LX-915 | 297 | LX-916 | 317 | LX-917 | 269 | LX-918 | 247 | LX-919 | 321 |
| LX-920 | 278 | LX-921 | 311 | LX-923 | 271 | LX-1001 | 231 | LX-1002 | 286 |
| LX-1101 | 268 | LX-1102 | 271 | LX-1103 | 172 | LX-1201 | 311 | LX-1202 | 289 |
| LX-1203 | 311 | LX-1205 | 279 | LX-1203 | 298 | LX-1207 | 285 | LX-1301 | 281 |
| LX-1302 | 171 | LX-1401 | 296 | LX-1402 | 304 | LX-1403 | 282 | LX-1405 | 219 |
| LX-1407 | 232 | LX-1601 | 154 | LX-1602 | 228 | LX-1603 | 168 | LX-1605 | 284 |
| LX-1607 | 238 | LX-1608 | 296 | LX-1701 | 195 | LX-1703 | 206 | LX-1801 | 291 |
| LX-1901 | 301 | LX-1902 | 303 | LX-1903 | 281 | LX-1905 | 315 | LX-2001 | 328 |
| LX-2002 | 305 | LX-2003 | 298 | LX-2005 | 212 | LX-2007 | 255 | LX-2101 | 274 |
| LX-2102 | 288 | LX-2201 | 318 | LX-2301 | 283 | LX-2302 | 279 | LX-2305 | 292 |
| LX-2306 | 301 | LX-2307 | 271 | LX-2308 | 329 | LX-2309 | 307 | LX-2310 | 315 |
| LX-2311 | 271 | LX-2312 | 293 | LX-2315 | 235 | LX-2316 | 274 | LX-2317 | 317 |
| LX-2318 | 264 | LX-2319 | 241 | LX-2401 | 303 | LX-2402 | 287 | LX-2403 | 319 |
| LX-2405 | 266 | LX-2406 | 274 | LX-2407 | 308 | LX-2408 | 297 | LX-2409 | 239 |
| LX-2501 | 309 | LX-2502 | 258 | LX-2503 | 297 | LX-2505 | 248 | LX-2506 | 279 |
| LX-2507 | 267 | LX-2508 | 313 | LX-2509 | 301 | LX-2510 | 288 | LX-2511 | 306 |
| LX-2512 | 276 | LX-2513 | 325 | LX-2517 | 298 | LX-2601 | 263 | LX-2602 | 208 |
| LX-2603 | 218 | LX-2701 | 194 | LX-2702 | 206 | LX-2703 | 218 | LX-2705 | 202 |
| LX-2707 | 235 | LX-2801 | 241 | LX-2802 | 268 | LX-2803 | 282 | LX-2805 | 182 |
| LX-2807 | 288 | LX-2809 | 286 | LX-2901 | 167 | LX-2902 | 297 | LX-3001 | 301 |
| LX-3002 | 335 | LX-3101 | 284 | LX-3102 | 291 | LX-3103 | 243 | LX-3105 | 195 |
| LX-3106 | 254 | LX-3107 | 196 | LX-3109 | 232 | LX-3200 | 234 | LX-3201 | 286 |
| LX-3203 | 315 | LX-3206 | 222 | LX-3207 | 219 | LX-3209 | 274 | LX-3301 | 228 |
| LX-3302 | 268 | LX-3303 | 275 | LX-3307 | 288 | LX-3309 | 293 | LX-3401 | 198 |

As a result, the amount of HMGN1 secreted from senescent cells that die when treated with test compounds increased like LX-112. In addition, as shown in the results obtained by treating with LX-112 in Example 10, caspase inhibitors did not effectively inhibit the death of senescent cells by the test compounds. Despite the presence of caspase inhibitors, the test compounds still could eliminate senescent cells by inducing their death. From this, it was confirmed that the test compounds induce the death of senescent cells through a different mechanism from ABT-263.

Furthermore, the degree of death of senescent cells were analyzed based on the increase in the amount of HMGN1 in the same manner as in Example 1. Cells in which senescence was induced by treatment with doxorubicin were treated with test compounds at designated concentrations and measured using the CellTiter-Glo Luminescent Cell Viability Assay (purchased from Promega). The cell viability results are shown in Table 65 below.

**[Table 65]**

| Survival (%) | 0 µM | 1.1 µM | 3.3 µM | 10 µM | 30 µM | 90 µM | 270 µM |
|---|---|---|---|---|---|---|---|
| LX-201 | 100 (±3) | 98.2 (±5.5) | 3 (±2.1) | | | | |
| LX-202 | 100 (±2.1) | 97.4 (±4.7) | 7.6 (±4.3) | | | | |
| LX-305 | 100 (±5.7) | 101.1 (±5.4) | 97.2 (±2.1) | 91 (±4) | 78 (±5.1) | 23.2 (±3.5) | |
| LX-307 | 100 (±1) | 101.05 (±6.3) | 85.6 (±6.4) | 14.5 (±4.5) | 3.5 (±4.7) | | |
| LX-309 | 100 (±7.1) | 52.3 (±2.7) | 12.5 (±6.4) | 4.3 (±5.1) | | | |
| LX-401 | 100 (±1) | 20.4 (±18.4) | 12.4 (±7.3) | 10.6 (±5.7) | 7.8 (±3.4) | | |
| LX-402 | 100 (±6.2) | 67.2 (±6.7) | 27 (±7.1) | 4.5 (±0.7) | | | |
| LX-403 | 100 (±4.8) | 36.9 (±4.2) | 15.5 (±3.2) | 6.5 (±1.8) | | | |
| LX-405 | 100 (±3.3) | 4.9 (±0.6) | | | | | |
| LX-406 | 100 (±8.2) | 49.4 (±3.3) | 24.9 (±6.6) | 8.9 (±1.7) | | | |
| LX-407 | 100 (±7.4) | 15.9 (±2.2) | | | | | |
| LX-411 | 100 (±5.9) | 0.2 (±0.0) | | | | | |
| LX-412 | 100 (±9.7) | 0.3 (±0.0) | | | | | |
| LX-413 | 100 (±5.1) | 0.5 (±0.0) | | | | | |
| LX-415 | 100 (±6.3) | 0.4 (±0.0) | | | | | |
| LX-416 | 100 (±2.4) | 70.3 (±4.3) | 29.1 (±2) | | | | |
| LX-417 | 100 (±4.4) | 21.8 (±2) | | | | | |
| LX-418 | 100 (±6.3) | 5.1 (±0.3) | | | | | |
| LX-419 | 100 (±8.1) | 10.3 (±0.8) | | | | | |
| LX-420 | 100 (±8.9) | 0.0 (±0.0) | | | | | |
| LX-421 | 100 (±5.8) | 3.4 (±0.2) | | | | | |
| LX-422 | 100 (±3.9) | 1.8 (±0.0) | | | | | |
| LX-431 | 100 (±4.8) | 89.5 (±5.4) | 12.6 (±0.6) | | | | |
| LX-432 | 100 (±9.5) | 84.2 (±3.7) | 23.8 (±2.1) | | | | |
| LX-435 | 100 (±7.6) | 89.1 (±3.6) | 5.9 (±0.3) | | | | |
| LX-436 | 100 (±8.8) | 84.4 (±4.1) | 3.8 (±0.2) | | | | |
| LX-437 | 100 (±10.2) | 86.2 (±6.1) | 10.9 (±0.5) | | | | |
| LX-501 | 100 (±7.1) | 102.7 (±3.9) | 104.8 (±1.6) | 98.9 (±0.3) | 85.2 (±4.1) | 53.7 (±1.8) | |
| LX-502 | 100 (±8.1) | 59.1 (±3.9) | 10.4 (±1.1) | | | | |
| LX-503 | 100 (±5.9) | 25.1 (±1.3) | | | | | |
| LX-505 | 100 (±12.7) | 35.2 (±0.6) | 0.4 (±0.0) | | | | |
| LX-506 | 100 (±5.7) | 101.1 (±5.4) | 97.2 (±2.1) | 91 (±4) | 78 (±5.1) | 33.2 (±3.5) | |
| LX-601 | 100 (±2) | 6.1 (±7.4) | 3.3 (±2.1) | | | | |
| LX-602 | 100 (±3.4) | 79 (±11.6) | 55.2 (±5.6) | 25.9 (±1.9) | 16.1 (±0.7) | | |
| LX-603 | 100 (±1.3) | 91.7 (±0.3) | 74.1 (±2.1) | 51.6 (±3.2) | 16.8 (±1.1) | | |
| LX-701 | 100 (±2.2) | 99.3 (±3.3) | 100.2 (±3.7) | 45.9 (±3.5) | 27.4 (±4.8) | | |
| LX-702 | 100 (±3) | 82.1 (±3) | 41.3 (±1.3) | 6.5 (±1.4) | | | |
| LX-801 | 100 (±4.4) | 50.5 (±2.7) | 48.9 (±5.1) | 28.2 (±5.4) | | | |
| LX-802 | 100 (±7.6) | 42.7 (±3.6) | 5.7 (±0.1) | | | | |
| LX-901 | 100 (±8.4) | 99.5 (±4.5) | 98.4 (±3.8) | 67.5 (±3.6) | 15.8 (±0.9) | | |
| LX-902 | 100 (±9.1) | 97.9 (±3.9) | 91.6 (±4.5) | 72.8 (±8.3) | 23.9 (±1.8) | | |
| LX-903 | 100 (±6.3) | 103.7 (±4.1) | 89.8 (±5.1) | 73.2 (±5.2) | 5.7 (±0.4) | | |
| LX-905 | 100 (±7.3) | 97.6 (±5.2) | 91.6 (±9.7) | 42.5 (±2.8) | 6.9 (±0.1) | | |
| LX-907 | 100 (±5.1) | 98.8 (±3.8) | 95.1 (±9.1) | 62.8 (±6.8) | 9.7 (±0.4) | | |
| LX-909 | 100 (±12.5) | 100.5 (±4.1) | 85.5 (±5.6) | 75.1 (±4.4) | 2.8 (±0.0) | | |
| LX-910 | 100 (±3.9) | 95.6 (±5.2) | 84.7 (±6.2) | 68.4 (±1.6) | 1.1 (±0.0) | | |
| LX-911 | 100 (±4.8) | 94.3 (±3.9) | 91.6 (±3.5) | 43.5 (±3.7) | 7.5 (±0.3) | | |
| LX-912 | 100 (±7.9) | 103.8 (±8.1) | 85.2 (±8.8) | 32.7 (±3.3) | 3.1 (±0.1) | | |
| LX-913 | 100 (±13.5) | 97.7 (±7.8) | 84.4 (±2.9) | 65 (±1.5) | 14.5 (±1.7) | | |
| LX-915 | 100 (±9.7) | 94.7 (±7.4) | 83.2 (±5.6) | 72.8 (±2.1) | 8.5 (±0.7) | | |
| LX-916 | 100 (±8.6) | 103.5 (±9.3) | 94.2 (±6.3) | 54.5 (±2.9) | 3.5 (±0.7) | | |
| LX-917 | 100 (±7.1) | 101.8 (±4.1) | 93.2 (±2.1) | 45.8 (±5.8) | 4.7 (±0.8) | | |
| LX-918 | 100 (±3.6) | 98.9 (±5.9) | 95.4 (±3.9) | 94.5 (±4.7) | 32.7 (±1.9) | | |
| LX-919 | 100 (±4.2) | 97.2 (±8.1) | 93.3 (±2.9) | 82.1 (±4.6) | 43.7 (±3.1) | | |
| LX-920 | 100 (±8.9) | 100.1 (±5.5) | 95.4 (±2.9) | 86.2 (±4.5) | 23.8 (±1.7) | | |
| LX-921 | 100 (±11.7) | 99.6 (±6.2) | 89.2 (±2.5) | 89.1 (±7.8) | 15.7 (±1.2) | | |
| LX-923 | 100 (±6.9) | 97.7 (±3.8) | 94.5 (±3.1) | 75.7 (±8.4) | 19.5 (±2) | | |
| LX-1001 | 100 (±2.7) | 89.9 (±2.5) | 81.8 (±1.7) | 57.7 (±5) | 34.5 (±1.5) | | |
| LX-1002 | 100 (±6.2) | 4.2 (±0.7) | | | | | |
| LX-1101 | 100 (±2.2) | 108.2 (±2.8) | 111.7 (±3.5) | 47.1 (±7.2) | 22.8 (±3.8) | | |
| LX-1102 | 100 (±1.5) | 94 (±4.4) | 13.9 (±4.4) | 3.1 (±1.2) | | | |
| LX-1103 | 100 (±4.1) | 103.6 (±4.2) | 98.9 (±5.1) | 99.6 (±7) | 95.2 (±10.4) | 88 (±10.8) | 61.3 (±9) |
| LX-1201 | 100 (±3.5) | 23 (±4.5) | | | | | |
| LX-1202 | 100 (±0.2) | 22.7 (±0.4) | 3.1 (±0.9) | | | | |
| LX-1203 | 100 (±5.3) | 4.6 (±1.7) | | | | | |
| LX-1205 | 100 (±6.4) | 0.1 (±0.0) | | | | | |
| LX-1203 | 100 (±9.3) | 54.6 (±7.2) | 38.2 (±0.7 | 1.5 (±0.3) | | | |
| LX-1207 | 100 (±5.5) | 36.8 (±4) | 10 (±2.5) | | | | |
| LX-1301 | 100 (±5) | 104.5 (±1) | 105.7 (±2) | 83 (±6) | 0.4 (±0.0) | | |
| LX-1302 | 100 (±3.7) | 102 (±2) | 105.2 (±7.7) | 92.2 (±2.4) | 79.6 (±4.3) | 52.4 (±4.6) | |
| LX-1401 | 100 (±2) | 96.4 (±6) | 88.1 (±2.2) | 10.2 (±12.4) | 9 (±11.3) | | |
| LX-1402 | 100 (±4.5) | 100.9 (±2.9) | 97.6 (±6.3) | 15.6 (±4.1) | 2.1 (±5.5) | | |
| LX-1403 | 100 (±2) | 101.8 (±3.6) | 97.6 (±7) | 55.4 (±5.2) | 18.3 (±15.6) | | |
| LX-1405 | 100 (±8.2) | 101.8 (±8.2) | 92.7 (±4.7) | 69.6 (±4.4) | 28.5 (±3.5) | | |
| LX-1407 | 100 (±6.9) | 99.4 (±7.1) | 95.9 (±5.2) | 72.8 (±2.4) | 19.9 (±2.2) | | |
| LX-1601 | 100 (±6.4) | 93.7 (±2.9) | 90 (±3.5) | 95.6 (±7.9) | 61.4 (±0.8) | | |
| LX-1602 | 100 (±5) | 109.4 (±6.8) | 93.8 (±10.9) | 57.7 (±11.1) | 36.4 (±5.2) | | |
| LX-1603 | 100 (±8) | 100.8 (±4.8) | 95.6 (±7.3) | 91.1 (±10) | 84.3 (±8.5) | 48.3 (±9.6) | |
| LX-1605 | 100 (±8.4) | 83.4 (±4.3) | 63.8 (±1.8) | 43.7 (±0.9) | 11.3 (±4.1) | | |
| LX-1607 | 100 (±3.6) | 77 (±2.6) | 68 (±4.2) | 54.3 (±2.1) | 32.8 (±3.6) | | |
| LX-1608 | 100 (±5.3) | 45.1 (±5.5) | 21.8 (±1.7) | 3.9 (±4.3) | | | |
| LX-1701 | 100 (±7.3) | 106.7 (±5.4) | 101.5 (±11.1) | 97.6 (±3.9) | 92.7 (±4.6) | 84.2 (±1.7) | 39.4 (±1.4) |
| LX-1703 | 100 (±6.9) | 118.2 (±11.9) | 105.6 (±7.4) | 101.2 (±2.3) | 97.5 (±4.2) | 29.6 (±2.5) | |
| LX-1801 | 100 (±2) | 8.7 (±9.4) | | | | | |
| LX-1901 | 100 (±8) | 66 (±8.3) | 26.6 (±3.4) | 9.9 (±2) | | | |
| LX-1902 | 100 (±1.1) | 59 (±4.2) | 39.6 (±4.1) | 11.9 (±4.7) | 3.9 (±1.2) | | |
| LX-1903 | 100 (±0.8) | 73.4 (±2.2) | 50.3 (±1.2) | 22.4 (±5.1) | 11.8 (±1.7) | | |
| LX-1905 | 100 (±0.6) | 101.9 (±5.2) | 66.4 (±9.9) | 27.9 (±5.3) | 13.2 (±4.3) | | |
| LX-2001 | 100 (±7.5) | 75.2 (±8.3) | 0.8 (±0.0) | | | | |
| LX-2002 | 100 (±10.7) | 100.9 (±8.4) | 12.4 (±1.7) | | | | |
| LX-2003 | 100 (±9.1) | 89.6 (±10.3) | 65.8 (±6.7) | 34.6 (±6.5) | 14 (±2.6) | | |
| LX-2005 | 100 (±10.2) | 97.5 (±6.2) | 93.3 (±4.2) | 87.2 (±4.7) | 73.8 (±3.2) | 31.5 (±1.1) | |
| LX-2007 | 100 (±9.6) | 105.4 (±8) | 98.9 (±7.1) | 91.2 (±6.1) | 83.9 (±7.3) | 47.2 (±1.4) | 1.3 (±0.0) |
| LX-2101 | 100 (±6.3) | 1.8 (±0.0) | | | | | |
| LX-2102 | 100 (±8.4) | 9.4 (±1.4) | | | | | |
| LX-2201 | 100 (±10.6) | 9.8 (±0.9) | | | | | |
| LX-2301 | 100 (±4.5) | 2.6 (±0.0) | | | | | |
| LX-2302 | 100 (±5.4) | 48.9 (±2.5) | 6.3 (±0.5) | | | | |
| LX-2305 | 100 (±4.3) | 19.6 (±1.7) | | | | | |
| LX-2306 | 100 (±8.7) | 29.8 (±1.8) | 7.1 (±0.4) | | | | |
| LX-2307 | 100 (±7.1) | 15.4 (±3.1) | | | | | |
| LX-2308 | 100 (±9.8) | 49.6 (±1.6) | 5.9 (±0.1) | | | | |
| LX-2309 | 100 (±7.6) | 69.7 (±2.4) | 2.7 (±0.0) | | | | |
| LX-2310 | 100 (±4.9) | 53.7 (±2.9) | 6.9 (±0.3) | | | | |
| LX-2311 | 100 (±8.6) | 39.5 (±3.8) | 4.1 (±0.2) | | | | |
| LX-2312 | 100 (±7.4) | 48.6 (±6.1) | 5.2 (±0.1) | | | | |
| LX-2315 | 100 (±9.8) | 75.2 (±5.1) | 2.9 (±0.0) | | | | |
| LX-2316 | 100 (±12.1) | 36.6 (±4.1) | 0.9 (±0.0) | | | | |
| LX-2317 | 100 (±8.2) | 48.2 (±2.9) | 7.8 (±0.1) | | | | |
| LX-2318 | 100 (±6.2) | 78.3 (±6.7) | 12.1 (±0.2) | | | | |
| LX-2319 | 100 (±7.8) | 52.9 (±2) | 21.9 (±0.3) | | | | |
| LX-2401 | 100 (±8.2) | 46.4 (±4.1) | 6.8 (±0.0) | | | | |
| LX-2402 | 100 (±6.3) | 45.2 (±2.8) | 5.1 (±0.1) | | | | |
| LX-2403 | 100 (±4.8) | 38.1 (±2.1) | 15.2 (±0.5) | | | | |
| LX-2405 | 100 (±10.8) | 19.6 (±1.6) | 12.1 (±0.7) | | | | |
| LX-2406 | 100 (±9.9) | 34.8 (±3.2) | 2 (±0.1) | | | | |
| LX-2407 | 100 (±9.8) | 15.4 (±1.9) | 5.9 (±0.8) | | | | |
| LX-2408 | 100 (±8.1) | 85.9 (±8.1) | 37.5 (±0.4) | | | | |
| LX-2409 | 100 (±7.9) | 37.1 (±0.9) | 12.8 (±1.8) | | | | |
| LX-2501 | 100 (±6.8) | 11.1 (±2.7) | | | | | |
| LX-2502 | 100 (±5.8) | 49.6 (±4.6) | 13.4 (±0.9) | | | | |
| LX-2503 | 100 (±7.7) | 82.4 (±5.9) | 25.8 (±2.5) | | | | |
| LX-2505 | 100 (±8.5) | 78.6 (±5.4) | 36.9 (±1.7) | | | | |
| LX-2506 | 100 (±5.4) | 91 (±5.5) | 11.8 (±1.1) | | | | |
| LX-2507 | 100 (±3.6) | 46.6 (±6.7) | 38.6 (±2.1) | | | | |
| LX-2508 | 100 (±3.8) | 75.6 (±2.5) | 28.2 (±1.3) | | | | |
| LX-2509 | 100 (±6.9) | 81.1 (±5.2) | 13.5 (±0.8) | | | | |
| LX-2510 | 100 (±8.2) | 62.9 (±1.5) | 11.8 (±1.6) | | | | |
| LX-2511 | 100 (±7.1) | 43.5 (±0.7) | 8.4 (±1.2) | | | | |
| LX-2512 | 100 (±5.8) | 38.2 (±3.3) | 3.2 (±0.7) | | | | |
| LX-2513 | 100 (±7.6) | 49.6 (±0.6) | 2.6 (±0.9) | | | | |
| LX-2517 | 100 (±6.6) | 0.6 (±0.0) | | | | | |
| LX-2601 | 100 (±6.4) | 95.2 (±7.4) | 81.8 (±7.9) | 58.6 (±2.3) | 21.8 (±1.7) | | |
| LX-2602 | 100 (±5) | 97.3 (±2.3) | 92.5 (±1.8) | 76.9 (±1.1) | 47.6 (±3.3) | | |
| LX-2603 | 100 (±7.4) | 100.4 (±4.8) | 102.9 (±5.1) | 95.8 (±4.1) | 52.6 (±3.9) | 37.8 (±2.6) | |
| LX-2701 | 100 (±5.1) | 103.4 (±3.6) | 97.3 (±2.4) | 76.8 (±4.8) | 48.3 (±2.9) | 29.4 (±0.6) | |
| LX-2702 | 100 (±8.1) | 103.9 (±7.3) | 99.1 (±8.6) | 87.4 (±4.4) | 49.6 (±0.4) | 22.9 (±9.1) | |
| LX-2703 | 100 (±2.1) | 100.5 (±5.1) | 100.1 (±5.9) | 89.5 (±5.4) | 55.2 (±4.1) | 35.2 (±4.1) | |
| LX-2705 | 100 (±5.4) | 102.4 (±3.9) | 101.8 (±8.3) | 72.1 (±3.6) | 45.5 (±1.2) | 31.7 (±2.3) | |
| LX-2707 | 100 (±8.2) | 95.1 (±6.3) | 90.4 (±6.8) | 64.8 (±4.5) | 29 (±4.8) | | |
| LX-2801 | 100 (±4.4) | 103.7 (±8.7) | 98.5 (±4.7) | 51.2 (±2.8) | 25.3 (±5) | | |
| LX-2802 | 100 (±3.1) | 102.3 (±2.2) | 81.2 (±5.1) | 58.4 (±6.1) | 17.2 (±1.2) | | |
| LX-2803 | 100 (±5.1) | 62 (±7.2) | 43.9 (±3.1) | 11.5 (±1.1) | 3.6 (±4) | | |
| LX-2805 | 100 (±11.5) | 99.4 (±7.8) | 97.3 (±8.1) | 92 (±4.8) | 84.2 (±5.6) | 82 (±7.4) | 51.1 (±6.8) |
| LX-2807 | 100 (±9.4) | 32.7 (±5.3) | 16.8 (±1.7) | | | | |
| LX-2809 | 100 (±8.7) | 85.1 (±76.3) | 30.4 (±3.1) | | | | |
| LX-2901 | 100 (±9.3) | 103.3 (±8.7) | 97.8 (±5.4) | 104.8 (±6.6) | 93.6 (±8.7) | 49.9 (±8.2) | |
| LX-2902 | 100 (±4.9) | 1.9 (±0.0) | | | | | |
| LX-3001 | 100 (±7.2) | 74.8 (±6.7) | 1.1 (±0.1) | | | | |
| LX-3002 | 100 (±6.8) | 87.5 (±5.7) | 1.2 (±0.1) | | | | |
| LX-3101 | 100 (±7.5) | 93.3 (±3.4) | 75.8 (±8.4) | 11.7 (±2.4) | | | |
| LX-3102 | 100 (±6.1) | 27.8 (±4.3) | | | | | |
| LX-3103 | 100 (±6.2) | 84.4 (±6.3) | 71.2 (±7.4) | 48.5 (±2.5) | 25 (±1.7) | | |
| LX-3105 | 100 (±7.7) | 82.1 (±5.8) | 59.5 (±8.4) | 41.1 (±5.7) | 37.7 (±2.8) | 4.1 (±0.6) | |
| LX-3106 | 100 (±3.9) | 97.7 (±3.8) | 87.4 (±6.2) | 82.5 (±4.8) | 59.7 (±4.2) | 9.8 (±0.5) | |
| LX-3107 | 100 (±5) | 87.7 (±5.8) | 83.4 (±4.8) | 74.2 (±3.3) | 33.4 (±1.7) | | |
| LX-3109 | 100 (±9.3) | 97.2 (±0.9) | 96 (±2.5) | 88.7 (±4) | 74 (±2.5) | 48.7 (±4.2) | 19.7 (±2.1) |
| LX-3200 | 100 (±4.5) | 92.2 (±6.5) | 78.6 (±3.9) | 14.3 (±3.1) | | | |
| LX-3201 | 100 (±7.5) | 99.3 (±6.1) | 91.9 (±2.1) | 20.1 (±2.1) | | | |
| LX-3203 | 100 (±10) | 106.9 (±3) | 15.4 (±0.7) | | | | |
| LX-3206 | 100 (±6.6) | 95.8 (±5.6) | 96 (±5) | 64.7 (±3.5) | 21.3 (±0.9) | | |
| LX-3207 | 100 (±7.7) | 99.3 (±7.6) | 91.9 (±8.7) | 56.4 (±3.2) | 17.9 (±2.4) | | |
| LX-3209 | 100 (±2) | 105.2 (±3.9) | 52.8 (±8) | 2 (±0.1) | | | |
| LX-3301 | 100 (±7.6) | 100.1 (±6.3) | 99.2 (±9.7) | 67.4 (±2.8) | 39.5 (±0.8) | 2.7 (±0.8) | |
| LX-3302 | 100 (±6.3) | 99.3 (±9.8) | 51.9 (±8.4) | 6.7 (±1.1) | | | |
| LX-3303 | 100 (±8.8) | 83.9 (±1) | 69.4 (±7.4) | 13.9 (±0.6) | | | |
| LX-3307 | 100 (±5) | 108.5 (±3.6) | 96.1 (±4.5) | 32.7 (±1.7) | | | |
| LX-3309 | 100 (±4.6) | 99.3 (±7.4) | 87.1 (±2.3) | 36.9 (±2.5) | | | |
| LX-3401 | 100 (±9.8) | 101.8 (±9.9) | 98.1 (±8.3) | 75.8 (±4.2) | 35.4 (±3) | | |

As a result, the higher the concentration of test compounds in senescence-induced cells, the faster the cell survival rate decreased. From this, it was confirmed again that the test compounds of the present invention can effectively eliminate senescent cells by inducing their death.

In this way, the cell survival rate decreases in senescent cells and the total number of cells decreases sharply. However, when cells in which senescence was not induced by treatment with the control group (DMSO) were treated with test compounds, the total number of cells continued to increase with growth. From this, it was reconfirmed that the test compounds can selectively remove senescent cells, as shown in the results of LX-112 in Example 1-2. The property of killing and removing senescent cells is not simply related to the generation of reactive oxygen species (ROS) within cells. This is because even though H₂O₂ (purchased from Deoksan Science), which has a strong oxidizing effect that increases oxygen radicals within cells, or NAC (N-acetyl cysteine, purchased from Sigma), which has a strong antioxidant effect that reduces reactive oxygen species, is used at excessive concentrations (1 or 20 mM), senescent cells were hardly removed even after treatment. Ultimately, it was confirmed that the test compounds of the present invention eliminate senescent cells through a unique target and a different death mechanism from apoptosis.

### Example 12. Removal of senescent cancer cells by compounds of the present invention

A549, a human-derived lung adenocarcinoma cancer cell line (purchased from ATCC), was cultured in the designated medium (RPMI-1640, purchased from GenDEPOT; 10% FBS, purchased from GenDEPOT; 100 U/ml Penicillin & Streptomycin, purchased from Invitrogen). After culturing, aging was induced in the same manner as Example 1. Cells were treated with doxorubicin or control (DMSO) and cultured. Then, cells were treated with test compounds at 30 µM (except that LX-305, LX-501, LX-506, LX-901, LX-902, LX-903, LX-905, LX-907, LX-909, LX-910, LX-911, LX-912, LX-913, LX-915, LX-916, LX-917, LX-918, LX-919, LX-920, LX-921, LX-923, LX-1001, LX-1101, LX-1302, LX-1601, LX-1603, LX-2003, LX-2603, LX-2701, LX-2702, LX-2703, LX-2705, LX-2901, LX-3105, LX-3106, and LX-3301 were 90 µM; LX-1103, LX-1701, LX-1703, LX-2005, LX-2007, LX-2805, and LX-3109 were 270 µM; and LX-3401 was 20 ng/ml). In the same manner as Example 10, the amount of HMGN1 secreted in the cell culture was measured using an enzyme-linked immunosorbent assay (ELISA Kit, purchased from AssayGenie). As in Example 11, in cancer cells in which senescence was not induced by treatment with the control group (DMSO), the amount of HMGN1 above the detection limit could not measured in both untreated and treated conditions with test compounds. On the other hand,
the amount of HMGN1 measured in cancer cells in which senescence was induced by treatment with doxorubicin was not treated with test compounds, and the amount was calculated as 100%, and the amount of HMGN1 was measured in conditions in which test compounds were treated in senescent cancer cells. The results are shown in Table 66 below. The amount of HMGN1 was measured under conditions in which aged cancer cells were treated with test compounds, and the results are shown in Table 66 below. The amount of HMGN1 measured in cancer cells in which senescence was induced by treatment with doxorubicin and was not treated with test compounds was calculated as 100%.

**[Table 66]**

| | HMG N1 | | HMG N1 | | HMG N1 | | HMG N1 | | HMGN 1 |
|---|---|---|---|---|---|---|---|---|---|
| DMSO | 100 | LX-201 | 337 | LX-202 | 327 | LX-305 | 227 | LX-307 | 305 |
| LX-309 | 254 | LX-401 | 277 | LX-402 | 340 | LX-403 | 320 | LX-405 | 310 |
| LX-406 | 315 | LX-407 | 339 | LX-411 | 335 | LX-412 | 327 | LX-413 | 322 |
| LX-415 | 297 | LX-416 | 315 | LX-417 | 347 | LX-418 | 286 | LX-419 | 313 |
| LX-420 | 307 | LX-421 | 328 | LX-422 | 336 | LX-431 | 283 | LX-432 | 270 |
| LX-435 | 263 | LX-436 | 278 | LX-437 | 301 | LX-501 | 233 | LX-502 | 321 |
| LX-503 | 333 | LX-505 | 317 | LX-506 | 169 | LX-601 | 318 | LX-602 | 248 |
| LX-603 | 332 | LX-701 | 297 | LX-702 | 305 | LX-801 | 329 | LX-802 | 329 |
| LX-901 | 255 | LX-902 | 234 | LX-903 | 239 | LX-905 | 217 | LX-907 | 264 |
| LX-909 | 240 | LX-910 | 238 | LX-911 | 206 | LX-912 | 244 | LX-913 | 240 |
| LX-915 | 229 | LX-916 | 198 | LX-917 | 223 | LX-918 | 200 | LX-919 | 215 |
| LX-920 | 197 | LX-921 | 175 | LX-923 | 219 | LX-1001 | 183 | LX-1002 | 296 |
| LX-1101 | 183 | LX-1102 | 324 | LX-1103 | 158 | LX-1201 | 289 | LX-1202 | 301 |
| LX-1203 | 298 | LX-1205 | 279 | LX-1203 | 299 | LX-1207 | 318 | LX-1301 | 318 |
| LX-1302 | 147 | LX-1401 | 287 | LX-1402 | 248 | LX-1403 | 306 | LX-1405 | 187 |
| LX-1407 | 175 | LX-1601 | 257 | LX-1602 | 197 | LX-1603 | 163 | LX-1605 | 338 |
| LX-1607 | 311 | LX-1608 | 317 | LX-1701 | 191 | LX-1703 | 206 | LX-1801 | 295 |
| LX-1901 | 213 | LX-1902 | 271 | LX-1903 | 267 | LX-1905 | 201 | LX-2001 | 328 |
| LX-2002 | 314 | LX-2003 | 218 | LX-2005 | 252 | LX-2007 | 192 | LX-2101 | 297 |
| LX-2102 | 233 | LX-2201 | 326 | LX-2301 | 274 | LX-2302 | 313 | LX-2305 | 269 |
| LX-2306 | 296 | LX-2307 | 279 | LX-2308 | 287 | LX-2309 | 322 | LX-2310 | 319 |
| LX-2311 | 276 | LX-2312 | 339 | LX-2315 | 247 | LX-2316 | 263 | LX-2317 | 282 |
| LX-2318 | 278 | LX-2319 | 310 | LX-2401 | 284 | LX-2402 | 286 | LX-2403 | 267 |
| LX-2405 | 249 | LX-2406 | 283 | LX-2407 | 316 | LX-2408 | 329 | LX-2409 | 281 |
| LX-2501 | 333 | LX-2502 | 297 | LX-2503 | 285 | LX-2505 | 308 | LX-2506 | 267 |
| LX-2507 | 248 | LX-2508 | 283 | LX-2509 | 305 | LX-2510 | 317 | LX-2511 | 296 |
| LX-2512 | 274 | LX-2513 | 311 | LX-2517 | 318 | LX-2601 | 193 | LX-2602 | 283 |
| LX-2603 | 245 | LX-2701 | 179 | LX-2702 | 237 | LX-2703 | 202 | LX-2705 | 160 |
| LX-2707 | 288 | LX-2801 | 315 | LX-2802 | 279 | LX-2803 | 195 | LX-2805 | 158 |
| LX-2807 | 293 | LX-2809 | 297 | LX-2901 | 189 | LX-2902 | 271 | LX-3001 | 261 |
| LX-3002 | 314 | LX-3101 | 334 | LX-3102 | 327 | LX-3103 | 254 | LX-3105 | 202 |
| LX-3106 | 236 | LX-3107 | 267 | LX-3109 | 247 | LX-3200 | 305 | LX-3201 | 309 |
| LX-3203 | 293 | LX-3206 | 215 | LX-3207 | 222 | LX-3209 | 199 | LX-3301 | 229 |
| LX-3302 | 232 | LX-3303 | 239 | LX-3307 | 295 | LX-3309 | 247 | LX-3401 | 284 |

As a result, the amount of HMGN1 secreted into aged cancer cells increased when cells died with treatment with test compounds. In addition, as shown in the results obtained by being treated with LX-112 in Example 10, caspase inhibitors did not effectively inhibit the death of senescent cells by the test compounds. Despite the presence of caspase inhibitors, the test compounds still could remove senescent cells by inducing cell death. From this, it was confirmed that the test compounds induce the death of aged cancer cells through a different mechanism from ABT-263.

Furthermore, the degree of death of aged cancer cells was analyzed based on the weight of HMGN1 in the same manner as in Example 1. Cancer cells in which aging was induced by treatment with doxorubicin were treated with test compounds at designated concentrations and measured using the CellTiter-Glo Luminescent Cell Viability Assay (purchased from Promega). The cell viability results are shown in Table 67 below.

**[Table 67]**

| Survival (%) | 0 µM | 1.1 µM | 3.3 µM | 10 µM | 30 µM | 90 µM | 270 µM |
|---|---|---|---|---|---|---|---|
| LX-201 | 100 (±3.4) | 72.5 (±2.7) | 15.2 (±1.7) | | | | |
| LX-202 | 100 (±8.3) | 96.7 (±8.7) | 71.6 (±4.9) | 37.3 (±3.3) | | | |
| LX-305 | 100 (±11.1) | 99.2 (±6.7) | 93.8 (±8.4) | 63.1 (±1.8) | 41 (±5.7) | 13.3 (±1.1) | |
| LX-307 | 100 (±1) | 100.6 (±0.8) | 96.8 (±4.2) | 72.5 (±9.6) | 7.6 (±0.7) | | |
| LX-309 | 100 (±3.5) | 100.5 (±1.4) | 96.9 (±2.9) | 92.3 (±9.7) | 28.1 (±1.7) | | |
| LX-401 | 100 (±6.4) | 89.7 (±8.1) | 68.6 (±7.8) | 42.1 (±4.7) | 16.8 (±1.1) | | |
| LX-402 | 100 (±2.7) | 71.6 (±9.7) | 20.3 (±1.1) | | | | |
| LX-403 | 100 (±8.8) | 40.2 (±2.8) | 33.7 (±2.1) | 3.5 (±0.8) | | | |
| LX-405 | 100 (±8.1) | 23.8 (±1.7) | | | | | |
| LX-406 | 100 (±5.4) | 64.2 (±3.3) | 33.9 (±1.8) | 10.2 (±0.2) | | | |
| LX-407 | 100 (±6.6) | 29.4 (±1.8) | | | | | |
| LX-411 | 100 (±4.6) | 1.8 (±0.0) | | | | | |
| LX-412 | 100 (±3.3) | 4.8 (±0.0) | | | | | |
| LX-413 | 100 (±5.4) | 10.1 (±1) | | | | | |
| LX-415 | 100 (±4.7) | 15.6 (±1.3) | | | | | |
| LX-416 | 100 (±2.8) | 91.4 (±2) | 36.1 (±1.1) | | | | |
| LX-417 | 100 (±3.5) | 43.5 (±3.3) | 2.2 (±0.1) | | | | |
| LX-418 | 100 (±2.6) | 17.7 (±0.8) | | | | | |
| LX-419 | 100 (±3.1) | 25.1 (±3.2) | | | | | |
| LX-420 | 100 (±2.5) | 10 (±1) | | | | | |
| LX-421 | 100 (±3.8) | 3.8 (±0.5) | | | | | |
| LX-422 | 100 (±4.1) | 4.8 (±0.4) | | | | | |
| LX-431 | 100 (±5.8) | 94.6 (±5.8) | 32.8 (±3.1) | | | | |
| LX-432 | 100 (±9.6) | 97.1 (±12.1) | 28.6 (±1.6) | | | | |
| LX-435 | 100 (±8.8) | 92.1 (±5.6) | 31.1 (±3.7) | | | | |
| LX-436 | 100 (±7) | 99.5 (±2) | 26.7 (±2.3) | | | | |
| LX-437 | 100 (±8.3) | 93.6 (±3.4) | 18.9 (±1.5) | | | | |
| LX-501 | 100 (±6.9) | 99.8 (±1.8) | 99.9 (±6.1) | 102.9 (±2.8) | 99.5 (±2.2) | 13.1 (±1.5) | |
| LX-502 | 100 (±3.8) | 78.4 (±1.9) | 33.3 (±1.7) | | | | |
| LX-503 | 100 (±2.7) | 34.4 (±2.1) | | | | | |
| LX-505 | 100 (±6.4) | 51.1 (±3.6) | 1.7 (±0.0) | | | | |
| LX-506 | 100 (±4.4) | 109.2 (±4.2) | 101.5 (±2.7) | 93.8 (±6.5) | 91.4 (±4.8) | 44.2 (±2.3) | |
| LX-601 | 100 (±5.7) | 13.8 (±3.4) | | | | | |
| LX-602 | 100 (±2.9) | 102.1 (±3.5) | 103 (±7.4) | 99 (±5.9) | 41.8 (±4.7) | 2.7 (±0.1) | |
| LX-603 | 100 (±5.1) | 94.1 (±6.3) | 83.9 (±3.4) | 53.6 (±1.7) | 17.5 (±1.9) | | |
| LX-701 | 100 (±3.1) | 99.2 (±4.8) | 98.2 (±7.6) | 91 (±7.7) | 11.3 (±2.4) | | |
| LX-702 | 100 (±4.8) | 87.3 (±7.1) | 50.2 (±3.8) | 33.4 (±1.3) | 3 (±0.2) | | |
| LX-801 | 100 (±5.9) | 78.1 (±7.3) | 58.5 (±2.1) | 12.7 (±1.5) | | | |
| LX-802 | 100 (±7) | 62.7 (±4.8) | 7.8 (±2.1) | | | | |
| LX-901 | 100 (±9.6) | 97.8 (±2.7) | 85.7 (±4.8) | 82.7 (±6.8) | 34.8 (±2.2) | 11.7 (±3.6) | |
| LX-902 | 100 (±11.1) | 94.6 (±4.6) | 91.1 (±3.3) | 88.2 (±6.1) | 62.7 (±2.5) | 25.8 (±1.7) | |
| LX-903 | 100 (±8.9) | 98.9 (±3.9) | 92.5 (±3.3) | 81.6 (±2.9) | 42.7 (±2.4) | 12.3 (±0.8) | |
| LX-905 | 100 (±9.3) | 100.8 (±2.3) | 97.5 (±3) | 83.1 (±1.5) | 79.5 (±3.3) | 22.7 (±1.1) | |
| LX-907 | 100 (±8.1) | 90.1 (±2.8) | 89.5 (±14.2) | 78.5 (±5) | 53.7 (±3.6) | 5.9 (±0.4) | |
| LX-909 | 100 (±7.3) | 96.7 (±3.3) | 87.5 (±8.8) | 85.6 (±4.3) | 77.2 (±1.3) | 17.5 (±2.1) | |
| LX-910 | 100 (±4.2) | 95.8 (±4.9) | 91.6 (±4.9) | 76.9 (±3.5) | 47.5 (±7) | 2.7 (±0.1) | |
| LX-911 | 100 (±5.2) | 98.4 (±5.3) | 89.2 (±5.4) | 82.9 (±7) | 78.6 (±3.4) | 27.4 (±2.1) | |
| LX-912 | 100 (±4.3) | 92.4 (±4.6) | 85.7 (±2.7) | 83.9 (±4.4) | 32.4 (±1.5) | 6.7 (±0.7) | |
| LX-913 | 100 (±6.9) | 95.1 (±4.2) | 90.2 (±2.6) | 82.6 (±6.8) | 45.7 (±3.2) | 1.9 (±0.6) | |
| LX-915 | 100 (±8.7) | 94.7 (±7.1) | 85.5 (±4.8) | 86.9 (±4) | 84.9 (±4.9) | 6.8 (±0.7) | |
| LX-916 | 100 (±6.4) | 103.9 (±4.1) | 91.4 (±2.9) | 71.7 (±3.8) | 24.4 (±0.8) | 1.7 (±0.1) | |
| LX-917 | 100 (±9.1) | 101.5 (±4.1) | 93.5 (±4) | 65.7 (±5.2) | 17.1 (±1.7) | 3.5 (±0.1) | |
| LX-918 | 100 (±7.4) | 103.8 (±3.8) | 89.5 (±3.6) | 86.7 (±4.1) | 82.9 (±3.8) | 31.8 (±2.8) | |
| LX-919 | 100 (±8.2) | 95.7 (±2.2) | 91.2 (±3.6) | 93.7 (±4.6) | 91.7 (±4.5) | 27.6 (±1.4) | |
| LX-920 | 100 (±4.7) | 98.4 (±2.3) | 97.6 (±8.3) | 95.3 (±3.6) | 93.9 (±2.8) | 25.1 (±1.9) | |
| LX-921 | 100 (±6.9) | 99.5 (±2.7) | 97.5 (±5.2) | 91.2 (±3.4) | 90.9 (±4) | 39.7 (±2.8) | |
| LX-923 | 100 (±7.1) | 96.7 (±2.9) | 92.5 (±2.8) | 89.4 (±3.8) | 81.7 (±3.6) | 15.7 (±0.8) | |
| LX-1001 | 100 (±3.9) | 95.5 (±7.5) | 96.6 (±7.7) | 92.2 (±4.2) | 71.8 (±2.9) | 25.3 (±0.7) | |
| LX-1002 | 100 (±4.1) | 47.6 (±2.9) | 20 (±2.7) | | | | |
| LX-1101 | 100 (±5.1) | 95.4 (±3.3) | 97.6 (±4.7) | 92.7 (±2.7) | 88.3 (±3.6) | 23.7 (±2.1) | |
| LX-1102 | 100 (±4.5) | 99.5 (±2.9) | 87.7 (±2.6) | 17.7 (±2.4) | | | |
| LX-1103 | 100 (±3.2) | 96.6 (±2.4) | 96.4 (±1.7) | 95.1 (±8.2) | 91 (±2.6) | 91.1 (±4.4) | 63.7 (±6.8) |
| LX-1201 | 100 (±6.7) | 47.3 (±1.2) | 26 (±1.1) | | | | |
| LX-1202 | 100 (±4.2) | 53.4 (±5.5) | 30.5 (±4.5) | | | | |
| LX-1203 | 100 (±0.2) | 88.7 (±6.4) | 9 (±0.9) | | | | |
| LX-1205 | 100 (±6.4) | 34.2 (±3) | 0.1 (±0.0) | | | | |
| LX-1203 | 100 (±5.3) | 96.5 (±2.9) | 78.3 (±3.7) | 15.5 (±1.2) | | | |
| LX-1207 | 100 (±3.9) | 59.8 (±3.7) | 5.7 (±0.2) | | | | |
| LX-1301 | 100 (±6.4) | 105.7 (±5.8) | 108.5 (±3.2) | 101 (±4.7) | 17.5 (±2) | | |
| LX-1302 | 100 (±4.2) | 95.5 (±4.6) | 94.8 (±4.7) | 102.2 (±6.2) | 101.7 (±3.2) | 69.6 (±3.3) | 19.8 (±1.4) |
| LX-1401 | 100 (±9.3) | 99 (±4.4) | 90.7 (±3.7) | 49.9 (±4.1) | 22.8 (±2.8) | | |
| LX-1402 | 100 (±4.1) | 97.6 (±3.9) | 87.5 (±4.8) | 81.1 (±3.6) | 29.5 (±3.2) | | |
| LX-1403 | 100 (±4.7) | 97.6 (±6.5) | 83.7 (±4.4) | 70 (±3.6) | 8.4 (±0.5) | | |
| LX-1405 | 100 (±9.2) | 97.1 (±4.8) | 94.9 (±1.2) | 82.8 (±1.7) | 37.4 (±3.2) | | |
| LX-1407 | 100 (±8.2) | 101.5 (±6.9) | 89.7 (±4.2) | 71.7 (±8.8) | 39.4 (±4.1) | | |
| LX-1601 | 100 (±3.9) | 114 (±4.7) | 115 (±8.3) | 117.5 (±6.2) | 74.2 (±4.2) | 3.1 (±0.1) | |
| LX-1602 | 100 (±7.6) | 94.3 (±2.9) | 95.7 (±3.3) | 96.5 (±5.4) | 39.8 (±2.1) | | |
| LX-1603 | 100 (±5.7) | 99.6 (±2.8) | 94.3 (±4.1) | 95.7 (±5.5) | 96.5 (±4.2) | 49.8 (±2.7) | |
| LX-1605 | 100 (±3.8) | 91.7 (±3.1) | 71.8 (±2.7) | 41.8 (±1.9) | 6.4 (±0.7) | | |
| LX-1607 | 100 (±5.2) | 95.7 (±4.9) | 99.1 (±3.2) | 62.7 (±5.7) | 15.1 (±1.2) | | |
| LX-1608 | 100 (±3.9) | 59.3 (±4.2) | 51.7 (±5.1) | 24.7 (±2.8) | 2.6 (±0.0) | | |
| LX-1701 | 100 (±6.3) | 108.9 (±4.4) | 98.6 (±1.6) | 91.3 (±3.7) | 90.5 (±3.3) | 87.01 (±3.5) | 29.8 (±1.5) |
| LX-1703 | 100 (±4.1) | 98.7 (±5.8) | 95.9 (±2.9) | 101.6 (±2.1) | 87.5 (±2.8) | 73.6 (±4.1) | 17.5 (±1.1) |
| LX-1801 | 100 (±3.3) | 60.6 (±3.2) | 43.9 (±3.4) | 25.1 (±1.7) | | | |
| LX-1901 | 100 (±4.1) | 63.8 (±4.6) | 56.9 (±3.7) | 51.1 (±3.8) | | | |
| LX-1902 | 100 (±11.4) | 66.2 (±6.6) | 40.1 (±2.7) | 4.2 (±0.2) | | | |
| LX-1903 | 100 (±9.8) | 93.1 (±6.1) | 56.2 (±4.8) | 12.3 (±1) | | | |
| LX-1905 | 100 (±5.5) | 100.2 (±3.5) | 92.3 (±3.1) | 89.1 (±4.3) | 53.9 (±3.8) | | |
| LX-2001 | 100 (±3.7) | 98.3 (±3.2) | 77.7 (±4.6) | 5.1 (±0.0) | | | |
| LX-2002 | 100 (±8.7) | 99.6 (±6.4) | 104 (±5.6) | 10.4 (±0.8) | | | |
| LX-2003 | 100 (±6.7) | 97.5 (±5.2) | 94.8 (±4.8) | 90.6 (±3.2) | 52.7 (±4.2) | 18.3 (±1.1) | |
| LX-2005 | 100 (±7.9) | 101.5 (±4.1) | 92.6 (±3.7) | 95.7 (±2.9) | 82.9 (±6.6) | 57.2 (±2.3) | 5.3 (±0.2) |
| LX-2007 | 100 (±2.8) | 111.3 (±2.9) | 103.9 (±2.4) | 96.3 (±7.5) | 87.9 (±2.9) | 62.8 (±2.4) | 32.4 (±2.2) |
| LX-2101 | 100 (±4.1) | 71.6 (±3.8) | 18.7 (±1.2) | | | | |
| LX-2102 | 100 (±6.1) | 88.4 (±4.4) | 15.8 (±0.8) | | | | |
| LX-2201 | 100 (±5.2) | 28.4 (±1.9) | | | | | |
| LX-2301 | 100 (±2.8) | 21.5 (±1.7) | | | | | |
| LX-2302 | 100 (±7.1) | 95.9 (±2.8) | 59.5 (±2.3) | 5.4 (±0.5) | | | |
| LX-2305 | 100 (±2.9) | 33.6 (±2.5) | | | | | |
| LX-2306 | 100 (±8.2) | 101.5 (±5.9) | 52.7 (±1.6) | 11.5 (±0.4) | | | |
| LX-2307 | 100 (±4.1) | 4.8 (±0.1) | | | | | |
| LX-2308 | 100 (±9.1) | 96.6 (±3.1) | 66.5 (±2.2) | 23.5 (±0.9) | | | |
| LX-2309 | 100 (±1.2) | 111.5 (±5.4) | 75.2 (±5.5) | 14.9 (±0.8) | | | |
| LX-2310 | 100 (±7.2) | 100.5 (±3.9) | 47.5 (±2) | 3.5 (±0.3) | | | |
| LX-2311 | 100 (±3.6) | 97.5 (±4.4) | 67.2 (±4.7) | 8.9 (±1.1) | | | |
| LX-2312 | 100 (±5.8) | 96.3 (±6.5) | 56.8 (±4) | 7.8 (±0.8) | | | |
| LX-2315 | 100 (±7.1) | 98.2 (±3.1) | 85.6 (±1.1) | 39.2 (±1.7) | | | |
| LX-2316 | 100 (±9.9) | 106.2 (±2.1) | 77.4 (±4.2) | 25.4 (±1.9) | | | |
| LX-2317 | 100 (±5.2) | 93.5 (±8.9) | 52.1 (±2.7) | 29.7 (±2) | | | |
| LX-2318 | 100 (±3.7) | 94.1 (±4.6) | 71.8 (±4.4) | 31.1 (±2.7) | | | |
| LX-2319 | 100 (±3.2) | 96.5 (±8.5) | 59.6 (±5.7) | 21.3 (±1.1) | | | |
| LX-2401 | 100 (±3.6) | 107.5 (±4.3) | 60.1 (±1.8) | 15.9 (±0.8) | | | |
| LX-2402 | 100 (±3) | 99.5 (±3.8) | 55.6 (±4.1) | 5.4 (±0.1) | | | |
| LX-2403 | 100 (±6.5) | 100.5 (±6.2) | 57.5 (±2.6) | 3.2 (±0.6) | | | |
| LX-2405 | 100 (±8.1) | 95.6 (±6.1) | 71.2 (±5.5) | 39.1 (±1.8) | | | |
| LX-2406 | 100 (±4.2) | 97.1 (±4.4) | 49.9 (±3.8) | 23.8 (±1.1) | | | |
| LX-2407 | 100 (±4.4) | 89.6 (±7.6) | 52.5 (±1.5) | 41.8 (±2.9) | | | |
| LX-2408 | 100 (±7.6) | 96.5 (±7.2) | 71.7 (±4.8) | 15.9 (±1.7) | | | |
| LX-2409 | 100 (±5.9) | 88.5 (±5.2) | 62.5 (±2.3) | 26.4 (±2.1) | | | |
| LX-2501 | 100 (±4.5) | 14.4 (±0.8) | | | | | |
| LX-2502 | 100 (±6.6) | 97.2 (±4.3) | 62.6 (±2.1) | 5.9 (±1.3) | | | |
| LX-2503 | 100 (±5.7) | 105.4 (±0.6) | 80.1 (±2.1) | 31.2 (±0.9) | | | |
| LX-2505 | 100 (±9.9) | 100.1 (±4.5) | 92.6 (±4.3) | 18.6 (±1.2) | | | |
| LX-2506 | 100 (±9.2) | 98.3 (±1.2) | 85.6 (±3.7) | 26.4 (±1.5) | | | |
| LX-2507 | 100 (±2.6) | 97.5 (±2.8) | 79.5 (±4.9) | 35.7 (±3.7) | | | |
| LX-2508 | 100 (±2.8) | 93.5 (±4.1) | 92.3 (±4.6) | 43.9 (±4) | | | |
| LX-2509 | 100 (±6.9) | 105.6 (±1.1) | 97.3 (±3.6) | 21.8 (±5) | | | |
| LX-2510 | 100 (±4.8) | 95.7 (±2.2) | 69.5 (±4.3) | 2.9 (±0.1) | | | |
| LX-2511 | 100 (±5.8) | 97.2 (±1.1) | 47.4 (±3.1) | 2.1 (±0.4) | | | |
| LX-2512 | 100 (±7.2) | 88.5 (±1.1) | 78.2 (±5.4) | 11.7 (±0.8) | | | |
| LX-2513 | 100 (±9.7) | 105.3 (±3.6) | 66.4 (±4.2) | 6.9 (±0.1) | | | |
| LX-2517 | 100 (±2.1) | 8.4 (±0.4) | | | | | |
| LX-2601 | 100 (±3.3) | 98.6 (±2.8) | 86 (±3.9) | 59.7 (±3.4) | 50.7 (±2.8) | | |
| LX-2602 | 100 (±4.1) | 102.7 (±4.9) | 107.4 (±3.7) | 96 (±3.9) | 32.5 (±1.1) | | |
| LX-2603 | 100 (±5.2) | 98.7 (±3.7) | 94.8 (±3.1) | 89.1 (±2.8) | 41.5 (±1.5) | 1.8 (±0.1) | |
| LX-2701 | 100 (±3.8) | 101.7 (±3.7) | 96.1 (±4.1) | 97.1 (±2.9) | 93.8 (±5.2) | 42.9 (±2.8) | |
| LX-2702 | 100 (±4.5) | 96.7 (±3.5) | 100.2 (±7.6) | 94.4 (±8.2) | 55.1 (±5.1) | 9.5 (±1.2) | |
| LX-2703 | 100 (±2.8) | 101.4 (±3.1) | 97.8 (±3.4) | 99.6 (±3.3) | 71.5 (±3.3) | 28.3 (±2.3) | |
| LX-2705 | 100 (±5.4) | 101.1 (±3.9) | 103.1 (±8.3) | 96.8 (±3.6) | 100.2 (±1.2) | 61.2 (±2.3) | |
| LX-2707 | 100 (±8.2) | 95.1 (±6.3) | 90.4 (±6.8) | 64.8 (±4.5) | 29 (±4.8) | | |
| LX-2801 | 100 (±6.9) | 89.6 (±3.1) | 88.4 (±3.3) | 58.6 (±3.8) | 14.2 (±1.1) | | |
| LX-2802 | 100 (±3.2) | 100.9 (±3.7) | 100.2 (±4.8) | 74.1 (±4.2) | 35.7 (±2.2) | | |
| LX-2803 | 100 (±4.7) | 91.2 (±2.5) | 82.6 (±4.9) | 84.9 (±3.4) | 54.4 (±2.7) | | |
| LX-2805 | 100 (±6.9) | 105.4 (±2.3) | 95.7 (±4.2) | 96.7 (±2.8) | 82.9 (±3.9) | 73.3 (±3.3) | 49.3 (±5.8) |
| LX-2807 | 100 (±5.5) | 65.9 (±3.5) | 33.5 (±2.1) | | | | |
| LX-2809 | 100 (±2.8) | 89.6 (±3.1) | 78.6 (±2.7) | 23.1 (±1.2) | | | |
| LX-2901 | 100 (±4.2) | 89 (±3.7) | 87.8 (±2.1) | 90.4 (±2.8) | 92.3 (±4.9) | 37.2 (±1.4) | |
| LX-2902 | 100 (±6.4) | 95.7 (±9.1) | 84.4 (±8.4) | 72.4 (±4.5) | 31.2 (±2.2) | | |
| LX-3001 | 100 (±4.8) | 101.6 (±3.3) | 95.6 (±3.8) | 22.6 (±1.1) | | | |
| LX-3002 | 100 (±3.3) | 90.2 (±3.3) | 9.9 (±0.7) | | | | |
| LX-3101 | 100 (±2.5) | 89.2 (±4.5) | 63.6 (±6.2) | 7.1 (±0.3) | | | |
| LX-3102 | 100 (±3.9) | 58.9 (±4) | 21.4 (±2.4) | | | | |
| LX-3103 | 100 (±2.8) | 68.7 (±6.1) | 51.1 (±3.3) | 21.1 (±1.4) | | | |
| LX-3105 | 100 (±2.9) | 84 (±2) | 79.4 (±2.5) | 69.9 (±9.1) | 59.3 (±1.7) | 39.6 (±0.0) | |
| LX-3106 | 100 (±7.2) | 108.5 (±8) | 98.1 (±5.2) | 91.7 (±8.8) | 76.8 (±5.9) | 4.7 (±0.0) | |
| LX-3107 | 100 (±2.3) | 96.5 (±1.7) | 88.7 (±4.8) | 77.8 (±2.5) | 4.4 (±0.2) | | |
| LX-3109 | 100 (±3.1) | 107.5 (±5.8) | 94.5 (±5.5) | 85.7 (±7.2) | 68.2 (±5.1) | 43.4 (±1.5) | 0.1 (±0.0) |
| LX-3200 | 100 (±3.5) | 94.9 (±4.2) | 91.7 (±7.1) | 61.6 (±2.7) | 1.6 (±0.0) | | |
| LX-3201 | 100 (±0.4) | 96.5 (±3.8) | 82.7 (±4.4) | 68.1 (±6.1) | 12.8 (±0.5) | | |
| LX-3203 | 100 (±0.9) | 105.4 (±4.7) | 72.9 (±1.4) | 21.6 (±0.6) | | | |
| LX-3206 | 100 (±2.6) | 92.6 (±4.7) | 91.4 (±6.3) | 51.9 (±3.9) | 5.7 (±0.0) | | |
| LX-3207 | 100 (±1.1) | 100.6 (±2.6) | 96.8 (±3.7) | 76.4 (±3.2) | 18.9 (±0.2) | | |
| LX-3209 | 100 (±2.3) | 95.4 (±4.4) | 104.4 (±5.8) | 55.7 (±5.6) | 2.2 (±0.1) | | |
| LX-3301 | 100 (±3.6) | 100.1 (±2.7) | 95.2 (±3.9) | 85.9 (±7.5) | 51.9 (±3.8) | 4.2 (±0.2) | |
| LX-3302 | 100 (±9.7) | 99.5 (±3.6) | 88.2 (±4.7) | 67.9 (±8.8) | 16.4 (±0.2) | | |
| LX-3303 | 100 (±3.2) | 100.6 (±3.6) | 93 (±1.7) | 53.8 (±5.9) | 11.7 (±0.5) | | |
| LX-3307 | 100 (±8.9) | 99.8 (±6) | 92.1 (±10.1) | 51.9 (±6.7) | 20 (±2.4) | | |
| LX-3309 | 100 (±3.9) | 103.6 (±10.8) | 88.9 (±2.3) | 49.6 (±1.9) | 35.2 (±2.4) | | |
| LX-3401 | 100 (±4.3) | 110.8 (±3.7) | 87.2 (±3.2) | 58.3 (±3.9) | 5.8 (±0.8) | | |

As a result, the higher the concentration of test compounds in senescence-induced cancer cells, the faster the cell survival rate decreased. From this, it was reconfirmed that the test compounds of the present invention can effectively eliminate aged cancer cells by inducing the death of aged cancer cells.

In this way, the cell survival rate decreases in aged cancer cells and the total number of cells decreases sharply. However, when cancer cells in which senescence was not induced by treatment with the control group (DMSO) were treated with test compounds, the total number of cells continued to increase with growth. From this, it was confirmed again that the test compounds can selectively remove aged cancer cells by inducing the death of aged cancer cells only.

Therefore, the compounds of the present invention can be usefully used in prosenescence anti-cancer therapy to senescent cancer cells and subsequently remove the senescent cancer cells. That is, the compounds of the present invention can be used for the treatment or prevention of proliferative diseases such as cancer.

### Example 13. Removal of senescent human cells by secreted fluid from cells treated with compounds of the present invention

In the same manner as in Example 1, senescence-induced foreskin cells treated with doxorubicin or control (DMSO) were treated with LX-401, LX-402, or LX-405 at a concentration of 30 µM, respectively. After 3 days, cell viability was measured using CellTiter-Glo Luminescent Cell Viability Assay (Promega) or light microscopy. As a result of the measurement, it was confirmed that when the test compounds were treated at high concentrations, they induced the death of most cells in both normally proliferating and senescent cells.

After treating cells with the test compound at a high concentration that induces the death of most cells in both non-senescence-induced and senescence-induced cells, the cells were washed three times with PBS to remove the test compound, and replaced with fresh medium and cultured for an additional 3 hours at the time when the compound has entered into the cell but has not yet induced cell death (approximately 1 to 3 hours after compound treatment). During this additional culture, by checking with an optical microscope, etc., the medium supernatants containing secreted fluid from normally proliferating and senescent cells in which cell death was induced were obtained. The obtained medium supernatant was replaced with the medium of normally proliferating and senescent cells, respectively, and cell survival rate was measured after 3 days. The results are shown in Table 68 below.

**[Table 68]**

| | Supernatant from Death-Induced Cells | Supernatant-Treated Cells | Survival (%) |
|---|---|---|---|
| LX-401 | Proliferating Cells | Proliferating Cells | 88.1 (±4.2) |
| | | Senescent Cells | 48.2 (±4.2) |
| | Senescent Cells | Proliferating Cells | 95.1 (±5.7) |
| | | Senescent Cells | 42.2 (±4) |
| | No Cells | Proliferating Cells | 97.9 (±7.2) |
| | | Senescent Cells | 101.1 (±5.5) |
| LX-402 | Proliferating Cells | Proliferating Cells | 78.1 (±3.8) |
| | | Senescent Cells | 31 (±1.2) |
| | Senescent Cells | Proliferating Cells | 101.1 (±2.2) |
| | | Senescent Cells | 48.3 (±2.1) |
| | No Cells | Proliferating Cells | 97.1 (±5.9) |
| | | Senescent Cells | 98 (±3.6) |
| LX-405 | Proliferating Cells | Proliferating Cells | 104 (±3) |
| | | Senescent Cells | 41.3 (±3.6) |
| | Senescent Cells | Proliferating Cells | 100.2 (±5.6) |
| | | Senescent Cells | 52.7 (±0.1) |
| | No Cells | Proliferating Cells | 99.4 (±2.4) |
| | | Senescent Cells | 102.8 (±4.6) |

As a result, both medium supernatants obtained from normally proliferating and senescent cells whose cell death was induced by the test compounds selectively induced the death of senescent cells. However, both media failed to induce death of normally proliferating cells. In this way, both medium supernatants obtained from normally proliferating and senescent cells whose cell death was induced by LX-112 selectively induced the death of senescent cells.

In addition, the medium supernatant obtained through the same process as above from the medium treated with test compounds without culturing cells (No Cells) did not induce the death of both normally proliferating and senescent cells. From this, it was confirmed that the medium supernatant comprising substances secreted from cells whose death was induced by treatment with test compounds can induce the death of senescent cells and effectively remove senescent cells.

Furthermore, other test compounds were used in the same manner in Example 11 at high concentrations of 30 µM, 90 µM, and 270 µM, respectively, and the culture medium supernatant comprising secreted fluid from dying senescent cells were obtained to measure the degree of death of senescent cells. The cell viability results are shown in Table 69 below.

**[Table 69]**

| | Survival | | Survival | | Survival | | Survival | | Survival |
|---|---|---|---|---|---|---|---|---|---|
| LX-201 | 62.9 (±3.5) | LX-202 | 45.7 (±3.7) | LX-305 | 89.5 (±5.2) | LX-307 | 51.8 (±4) | LX-309 | 58.4 (±2.8) |
| LX-401 | 42.2 (±4) | LX-402 | 48.3 (±2.1) | LX-403 | 45.1 (±1.5) | LX-405 | 52.7 (±0.1) | LX-406 | 61.2 (±1.8) |
| LX-407 | 58.7 (±1.5) | LX-411 | 51.8 (±2.1) | LX-412 | 53.3 (±3.4) | LX-413 | 48.1 (±2.8) | LX-415 | 51.1 (±3.7) |
| LX-416 | 54.8 (±3.8) | LX-417 | 53.2 (±5.1) | LX-418 | 49.8 (±3.9) | LX-419 | 57.8 (±6.1) | LX-420 | 53.3 (±2.4) |
| LX-421 | 53.9 (±4.9) | LX-422 | 47.8 (±3.1) | LX-431 | 61.8 (±3.8) | LX-432 | 53.9 (±4.3) | LX-435 | 55.7 (±2.3) |
| LX-436 | 49.2 (±2.8) | LX-437 | 51.9 (±2) | LX-501 | 97.5 (±6.2) | LX-502 | 53.9 (±3.7) | LX-503 | 58.7 (±6.2) |
| LX-505 | 61.7 (±3.5) | LX-506 | 85.5 (±3.5) | LX-601 | 55.4 (±3.8) | LX-602 | 61.2 (±3.3) | LX-603 | 65.5 (±3.1) |
| LX-701 | 56.2 (±2.4) | LX-702 | 59.1 (±3.6) | LX-801 | 52.7 (±3.7) | LX-802 | 48.4 (±1.8) | LX-901 | 61.4 (±2.7) |
| LX-902 | 58.3 (±1.2) | LX-903 | 47.6 (±3.6) | LX-905 | 46.5 (±1) | LX-907 | 52.6 (±3.2) | LX-909 | 49.2 (±3.3) |
| LX-910 | 61.1 (±7.9) | LX-911 | 47.6 (±3.4) | LX-912 | 49.5 (±2.1) | LX-913 | 52.4 (±2.2) | LX-915 | 53.1 (±3.8) |
| LX-916 | 48.7 (±4) | LX-917 | 42.5 (±4.1) | LX-918 | 63.8 (±4.2) | LX-919 | 74.5 (±3.6) | LX-920 | 58.8 (±2.4) |
| LX-921 | 46.7 (±4) | LX-923 | 51.7 (±3.1) | LX-1001 | 64.3 (±3.7) | LX-1002 | 43.3 (±2.6) | LX-1101 | 62.1 (±3.5) |
| LX-1102 | 52.9 (±3.2) | LX-1103 | 101.8 (±4.9) | LX-1201 | 57.8 (±3.3) | LX-1202 | 55.1 (±4.1) | LX-1203 | 47.1 (±2.1) |
| LX-1205 | 53.4 (±3.8) | LX-1203 | 63.5 (±1.8) | LX-1207 | 62.7 (±7.8) | LX-1301 | 58.4 (±5.1) | LX-1302 | 98.5 (±3.5) |
| LX-1401 | 48.2 (±3.6) | LX-1402 | 49.7 (±2.7) | LX-1403 | 59.5 (±4.5) | LX-1405 | 71.8 (±2.8) | LX-1407 | 69.9 (±3.6) |
| LX-1601 | 81.4 (±3.4) | LX-1602 | 64.3 (±3.9) | LX-1603 | 97.5 (±6.1) | LX-1605 | 52.4 (±6.5) | LX-1607 | 73.5 (±5.3) |
| LX-1608 | 62.4 (±2.8) | LX-1701 | 104.5 (±5) | LX-1703 | 100.9 (±5.8) | LX-1801 | 41.6 (±2.7) | LX-1901 | 62.8 (±4.4) |
| LX-1902 | 53.4 (±4.4) | LX-1903 | 62.8 (±6.1) | LX-1905 | 61.6 (±4.6) | LX-2001 | 57.8 (±3.1) | LX-2002 | 58.3 (±2.5) |
| LX-2003 | 64.2 (±3.9) | LX-2005 | 91.6 (±2.5) | LX-2007 | 95.7 (±3.7) | LX-2101 | 39.7 (±2.5) | LX-2102 | 46.3 (±3.4) |
| LX-2201 | 57.7 (±8.2) | LX-2301 | 51.4 (±3.6) | LX-2302 | 62.8 (±5.1) | LX-2305 | 52.2 (±7.2) | LX-2306 | 47.2 (±5.6) |
| LX-2307 | 61.1 (±1.9) | LX-2308 | 43.9 (±3.3) | LX-2309 | 51.7 (±3.2) | LX-2310 | 56.8 (±2.4) | LX-2311 | 48.6 (±2.4) |
| LX-2312 | 59.4 (±2.4) | LX-2315 | 41.7 (±2.1) | LX-2316 | 53.9 (±2.3) | LX-2317 | 47.5 (±2.5) | LX-2318 | 46.2 (±2.5) |
| LX-2319 | 44.1 (±2.4) | LX-2401 | 48.3 (±1.8) | LX-2402 | 47.8 (±1.9) | LX-2403 | 46.9 (±1.7) | LX-2405 | 48.2 (±2.9) |
| LX-2406 | 51.3 (±3.4) | LX-2407 | 54.3 (±5.1) | LX-2408 | 55.2 (±2.8) | LX-2409 | 43.4 (±1.8) | LX-2501 | 49.7 (±3.6) |
| LX-2502 | 50.1 (±4) | LX-2503 | 47.3 (±4.6) | LX-2505 | 45.9 (±3.4) | LX-2506 | 53.9 (±5) | LX-2507 | 46.2 (±0.6) |
| LX-2508 | 47.9 (±4.2) | LX-2509 | 53.6 (±5.3) | LX-2510 | 46.3 (±5) | LX-2511 | 47.2 (±6.8) | LX-2512 | 59.6 (±3.3) |
| LX-2513 | 39.6 (±3.6) | LX-2517 | 52.7 (±3.7) | LX-2601 | 67.3 (±4.2) | LX-2602 | 69.5 (±6.2) | LX-2603 | 73.5 (±5.5) |
| LX-2701 | 71.5 (±2.5) | LX-2702 | 68.6 (±3.1) | LX-2703 | 65.5 (±1.3) | LX-2705 | 72.6 (±6.3) | LX-2707 | 68.6 (±3.2) |
| LX-2801 | 61.1 (±2.6) | LX-2802 | 64.3 (±1.5) | LX-2803 | 43.8 (±2.2) | LX-2805 | 102.4 (±3.9) | LX-2807 | 45.3 (±4.7) |
| LX-2809 | 55.5 (±2.1) | LX-2901 | 97.5 (±5.6) | LX-2902 | 61.8 (±3.6) | LX-3001 | 58.7 (±3.6) | LX-3002 | 46.8 (±4.1) |
| LX-3101 | 52.8 (±2.9) | LX-3102 | 54.6 (±2.5) | LX-3103 | 64.3 (±3.5) | LX-3105 | 69.1 (±4) | LX-3106 | 72.6 (±1.3) |
| LX-3107 | 62.1 (±5.3) | LX-3109 | 78.2 (±5.1) | LX-3200 | 54.9 (±0.7) | LX-3201 | 55.2 (±1.7) | LX-3203 | 57.1 (±4.1) |
| LX-3206 | 51.9 (±2.4) | LX-3207 | 61.2 (±3.6) | LX-3209 | 58.8 (±2.6) | LX-3301 | 74.6 (±1.2) | LX-3302 | 47.8 (±3.4) |
| LX-3303 | 53.5 (±5.6) | LX-3307 | 52.8 (±2.8) | LX-3309 | 47.1 (±3.4) | LX-3401 | 66.8 (±3.3) | | |

As a result, it was confirmed again that the medium supernatant comprising secreted fluid from cells whose death was induced by treatment with test compounds can effectively remove senescent cells by inducing their death. The same results were confirmed in LX-112.

### Example 14. Removal of senescent cancer cells by secreted fluid from cells treated with the compounds of the present invention

In the same way as Examples 12 and 13, senescence of cancer (A549) cells was induced by treatment with doxorubicin or the control group (DMSO), and the cells were cultured. The cells were treated with LX-405 at a concentration of 30 µM. 3 days later, cell viability was measured using CellTiter-Glo Luminescent Cell Viability Assay (Promega) or an optical microscope. In the same manner as in Example 13, the medium supernatants comprising secreted fluid from normally proliferating and senescent cancer cells was obtained. Normally proliferating and senescent-induced cancer cells were treated with the medium supernatants. Afterwards, the survival rate of cells was measured and the results are shown in Table 70 below.

**[Table 70]**

| | Cells | Supernatant from Death-Induced Cells | Supernatant-Treated Cells | Survival (%) |
|---|---|---|---|---|
| LX-405 | A549 | Proliferating Cells | Proliferating Cells | 98.6 (±4.8) |
| | | | Senescent Cells | 78.8 (±7.7) |
| | | Senescent Cells | Proliferating Cells | 105.2 (±4.9) |
| | | | Senescent Cells | 72.5 (±5.7) |
| | | No Cells | Proliferating Cells | 101.4 (±1.3) |
| | | | Senescent Cells | 98.2 (±2.8) |

As a result, it was confirmed that the medium supernatant comprising secreted fluid from cancer cells whose death was induced by treatment with test compounds could more effectively remove aged cancer cells compared to normally proliferating cancer cells.

Furthermore, other test compounds were used in the same manner in Example 12 above at high concentrations of 30 µM, 90 µM, and 270 µM, respectively, and the cultured medium supernatant comprising secreted fluid from aged cancer cells that were killed were obtained. As a result of measuring the degree of death of aged cancer cells, it was confirmed that aged cancer cells can be effectively removed, as shown in Example 12 and Table 65.

### Example 15. Removal of senescent cells and related anti-aging activity by the compounds of the present invention in aging mice

In the same manner as in Example 7-1, naturally aged mice were administered LX-201 (5% DMSO in PBS solution (purchased from GenDEPOT), 15 mg/kg concentration, intraperitoneal administration), LX-307 (5% DMSO in PBS solution (purchased from GenDEPOT), 12 mg/kg concentration, intraperitoneal administration), LX-405 (5% DMSO in PBS solution (purchased from GenDEPOT), 10 mg/kg concentration, intraperitoneal administration), LX-411 (0.5% CMC-Na solution (purchased from Sigma), 10 mg/kg concentration, intraperitoneal administration), LX-502 (0.5% CMC-Na solution (purchased from Sigma), 100 mg/kg concentration, oral administration), LX-701 (PBS (purchased from GenDEPOT), 100 mg/kg concentration, intraperitoneal administration), LX-801 (PBS (purchased from GenDEPOT), 100 mg/kg concentration, intraperitoneal administration), LX-1002 (5% DMSO in PBS solution (purchased from GenDEPOT), 100 mg/kg concentration, intraperitoneal administration), LX-1102 (0.5% CMC-Na solution (purchased from Sigma), 100 mg/kg concentration, oral administration), LX-1202 (PBS (purchased from GenDEPOT), 5 mg/kg concentration, intraperitoneal administration), LX-1401 (PBS (purchased from GenDEPOT), 50 mg/kg concentration, oral administration), LX-1801 (PBS (purchased from GenDEPOT), 50 mg/kg concentration, intraperitoneal administration), LX-2101 (0.5% CMC-Na solution (purchased from Sigma), 10 mg/kg concentration, intraperitoneal administration), and LX-3403 (mouse anti-PD-1 (purchased from BioXcell), 100 µg concentration, intraperitoneal administration) for 5 days under the following conditions. The control group was administered only the solution in the same volume. After drug administration was completed, the method described in each Example below was performed. The mouse was sacrificed in the same manner as Example 7-2 and 7-6. Total RNA was extracted from liver tissue, and qRT-PCR was performed and analyzed using synthesized cDNA. Table 71 shows the mRNA amounts of p16, a aging-related marker, and MMP3 and IL6, which are aging-related inflammation and fibrosis secretory factors (SASP) in mice in which natural aging was induced and without or with test compound treatments. The mRNA amounts of p16, MMP3, and IL6 after natural aging were induced were calculated as 100% and compared them with the mRNA amounts of p16, MMP3, and IL6 after treatment with test compounds. Furthermore, an open field test was performed to analyze aging-related frailty in mice using the same method as Example 7-7. Table 71 shows the quantitative results of activity evaluation, which is a criterion for determining frailty, under conditions in which natural aging-induced mice were not treated with and treated with the test compounds. The amount of activity evaluated after inducing aging was calculated as 100% and compared it with the amount of activity evaluated after treatment with the test compounds.

**[Table 71]**

| | p16 mRNA (*%*) | MMP3 mRNA (%) | IL6 mRNA (%) | Open Field Test Time in Center (%) |
|---|---|---|---|---|
| LX-201 | 85 (±8.2) | 77 (±9.6) | 65.7 (±5.1) | 226.4 (±27.6) |
| LX-307 | 68.7 (±14.8) | 71.7 (±7.5) | 71.4 (±4.7) | 169.3 (±16.1) |
| LX-405 | 76.9 (±6.8) | 64.7 (±11.5) | 73.6 (±7.4) | 201.5 (±18.3) |
| LX-411 | 78.8 (±7.5) | 73.7 (±9.4) | 76 (±5.8) | 180.1 (±12.6) |
| LX-502 | 81.4 (±6.8) | 79.4 (±7.7) | 72.8 (±4.8) | 167.7 (±20.2) |
| LX-701 | 72.3 (±8.2) | 81.1 (±7.5) | 75 (±9.6) | 215.1 (±38.1) |
| LX-801 | 69.4 (±6.9) | 76.5 (±4.2) | 71.1 (±1.8) | 241.8 (±17.9) |
| LX-1002 | 76.1 (±12.6) | 68.4 (±16.9) | 78.5 (±7.3) | 210.4 (±15.2) |
| LX-1102 | 75.2 (±5.5) | 89.7 (±12.5) | 84.5 (±8.9) | 163 (±7.9) |
| LX-1202 | 74.2 (±10.4) | 40.2 (±22.7) | 74.1 (±6.1) | 231 (±23) |
| LX-1401 | 80.8 (±8.9) | 70.6 (±11.1) | 78.6 (±8.5) | 213.1 (±31.4) |
| LX-1801 | 77.4 (±5.2) | 47.1 (±15.6) | 56.8 (±19.3) | 252.9 (±28) |
| LX-2101 | 62.5 (±13.1) | 53.6 (±14.3) | 64.1 (±8.8) | 215.3 (±30.8) |
| LX-3403 | 78.1 (±5.1) | 70.4 (±3.7) | 77.7 (±5.9) | 178.2 (±7.3) |

As a result, as shown in Example 7-2 and Table 21, Example 7-6 and Table 31, Example 7-5, and Table 28, when mice naturally aged, the amounts of p16, MMP3, and IL6 increased in liver tissue. On the other side, when treated with the test compounds, the mRNA amounts of p16, MMP3, and IL6 decreased. From this, it was confirmed that the test compounds effectively reduce the amount of p16, an aging marker, and MMP3 and IL6, aging-related inflammation and fibrosis secretory factors (SASP), by eliminating senescent cells that increase in mice due to natural aging.

Furthermore, as shown in Example 7-7 and Table 32, when mice naturally aged, activity decreased, but when treated with the test compounds, activity increased. From this, it was confirmed that the test compounds improved frailty of mice by removing senescent cells that increase as mice age.

### Example 16. Removal of senescent cells and related anti-aging activity by senescent cells killed by the compounds of the present invention in aging mice

To obtain preadipocytes, groin fat tissue was collected from an 8-week-old female C57BL6J mouse (purchased from KRIBB Laboratory Animal Resource Center). The fat tissue was cut into small pieces and treated with collagenase to decompose the fat tissue, then centrifuged to collect the stromal vascular fraction (SVF) containing preadipocytes. It was washed with a washing medium (HBSS, purchased from GenDEPOT; 3.5% Albumin Fraction V, purchased from GenDEPOT; 100 U/ml Penicillin & Streptomycin, purchased from Invitrogen), mixed again, and extracted by filtering through a 100 µm nylon mesh. The isolated preadipocytes were cultured in the designated medium (DMEM, purchased from GenDEPOT; 20% FBS, purchased from GenDEPOT; 100 U/ml Penicillin & Streptomycin, purchased from Invitrogen). In the same manner as Example 1, cells were treated with doxorubicin at a concentration of 250 nM for 24 hours and cultured for an additional 21 days to induce cell senescence. Cells were treated with doxorubicin at a concentration of 250 nM for 24 hours and cultured for an additional 21 days to induce cell senescence. By treating the test compounds in the same manner as in Example 13, it was confirmed using an optical microscope that the death of most senescent cells was induced. The killed preadipocytes were washed using PBS (purchased from Welgene) and then mixed with PBS at a concentration of 5×10⁵ cells/200 µl. It was administered subcutaneously to 8-week-old female C57BL6J mice (purchased from KRIBB Laboratory Animal Resource Center). At 7 and 10 days after administration of the killed senescent cells, 2 million senescent preadipocytes were mixed in 200 µl of PBS and administered into the abdominal cavity of mice. After administration of killed senescent cells, obesity was induced in mice with a high-fat diet (DIO) for 8 weeks in the same manner as in Example 9-1. Afterwards, the mouse was sacrificed in the same manner as in Example 7-2, total RNA was extracted from the groin fat tissue, and qRT-PCR was performed and analyzed using the synthesized cDNA. Table 72 shows the mRNA amounts of p16, an aging marker, and MMP3 and IL6, which are aging-related inflammation and fibrosis secretory factors (SASP), under conditions of not administering and administering cells whose death was induced by the test compounds. The mRNA amount of p16, MMP3, and IL6 without administration of senescent cells whose death was induced by the test compounds was calculated as 100%, and the amount of p16, MMP3, and IL6 after administration of the senescent cells whose death was induced by the test compounds was compared with them. Furthermore, an open field test was performed to analyze aging-related frailty in mice using the same method as Example 7-7. Table 72 shows the results quantitatively showing the activity, which is a criterion for determining frailty, under the conditions of not administering and administering senescent cells whose death was induced by test compounds. The amount of activity evaluation without administration of senescent cells whose death was induced by the test compounds was calculated as 100% and compared it with the amount of activity evaluation after administration of senescent cells whose death was induced by the test compounds.

**[Table 72]**

| | p16 mRNA (%) | MMP3 mRNA (%) | IL6 mRNA (%) | Open Field Test Time in Center (%) |
|---|---|---|---|---|
| LX-201 | 79.5 (±7.6) | 67.1 (±9.2) | 76.8 (±6.4) | 176 (±22.6) |
| LX-307 | 73.1 (±8.5) | 76.7 (±7.7) | 87.2 (±10.5) | 159 (±9.9) |
| LX-405 | 72.4 (±3.9) | 80.5 (±2.8) | 68.7 (±9.1) | 174.2 (±12.5) |
| LX-411 | 79.7 (±6.6) | 92.7 (±8.1) | 71.9 (±7.2) | 163 (±9) |
| LX-502 | 81.7 (±7.8) | 77.4 (±8) | 83.3 (±5.1) | 126 (±20.4) |
| LX-701 | 81.9 (±6.8) | 72.5 (±9.2) | 68.2 (±14.3) | 181.8 (±27.1) |
| LX-801 | 79.8 (±3.4) | 81.7 (±2.9) | 79.4 (±4.6) | 216.8 (±12.8) |
| LX-1002 | 77.4 (±8.6) | 68.1 (±13.8) | 74.4 (±8.5) | 141.1 (±18.3) |
| LX-1102 | 82.9 (±4.8) | 95.4 (±9.6) | 76.2 (±7.1) | 146.2 (±17.1) |
| LX-1202 | 67.2 (±12.1) | 70.7 (±7.5) | 62.8 (±8.9) | 175.9 (±21.5) |
| LX-1401 | 83.4 (±6.9) | 75.1 (±9.4) | 72.8 (±10.4) | 166.7 (±14.8) |
| LX-1801 | 62.5 (±9.4) | 67.8 (±13.1) | 56.4 (±12.4) | 173.4 (±18.6) |
| LX-2101 | 73.8 (±9.7) | 68.3 (±16.4) | 75.9 (±6.7) | 144.9 (±26.6) |

As a result, as shown in Example 9-2 and Table 53 or Example 9-3 and Table 55, when mice aged due to obesity, the mRNA amounts of p16, MMP3, and IL6 increased in adipose tissue, etc. However, the mRNA amounts of p16, MMP3, and IL6 decreased when senescent cells whose death was induced by the test compounds were administered to mice and senescence was induced by obesity. From this, it was confirmed that when senescent cells induced to die by the test compounds are administered to mice, they can effectively reduce the amount of p16, an aging marker, and MMP3 and IL6, aging-related inflammation and fibrosis secretory factors (SASP) by removing senescent cells that increase in mice due to aging such as obesity.

Furthermore, as shown in Example 9-4 and Table 57, activity decreased when mice aged due to obesity. However, when senescent cells induced to die by the test compounds were administered to mice and aging was induced, activity increased in comparison. From this, it was confirmed that administering senescent cells whose death was induced by the test compounds to mice improved frailty in mice by eliminating senescent cells that increase in mice due to aging such as obesity.

In this way, the effect of administration of senescent cells induced to die by the test compounds, which improve frailty by removing senescent cells and reducing p16 and inflammation and fibrosis secretory factor (SASP), was not observed in the Rag-2 gene knockout C57BL6J mice (purchased from KRIBB Laboratory Animal Resource Center). Therefore, it was confirmed that the immune system in the body is involved in the effect of removing and reducing senescent cells using senescent cells whose death was induced by the test compounds. Ultimately, combined administration of immune checkpoint inhibitors, cytokines (interferons, interleukins, etc.), and polysaccharide-K, which are known to contribute to this immune response process in the body, can further increase the effect of eliminating senescent cells. In this way, the combined administration of immune checkpoint inhibitors, cytokines, etc. along with HMGN1, which binds to the immune response receptor (Toll-like receptor), can synergistically activate the immune response. In this regard, in the above experiment in Example 16, it further reduced the amount of p16 mRNA by about 27% to intraperitoneally administering mouse anti-PD-1 (purchased from BioXcell), LX-3403, at a concentration of 100 µg each time twice a week for 8 weeks after 7 days of administration of senescent cells whose death was induced by the test compounds such as LX-405. From this, it was confirmed that senescent cells whose death was induced by the test compounds could effectively remove increased senescent cells in mice with induced aging either alone or in combination with an immune response activator. Through this in vivo verification, the removal of senescent cells can be induced by contacting dying senescent cells with immune cells (dendritic cells, lymphocytes, macrophages, monocytes, neutrophils, epithelial cells, innate lymphoid cells, mast cells, eosinophils, basophils, natural killer cells, etc.) or immune tissues *ex vivo.*

## Claims

1. A composition for removing senescent cells, comprising a compound of Table 1 or 2 or its pharmaceutically acceptable salt; secreted fluid from cells killed by treatment with such a compound or its pharmaceutically acceptable salt; or cells killed by treatment with such a compound or its pharmaceutically acceptable salt.

2. The composition for removing senescent cells according to claim 1, wherein the compound of Table 1 or 2 or its pharmaceutically acceptable salt increases the secretion of HMGN1 (High Mobility Group Nucleosome Binding Domain 1) protein (HMG14) in senescent cells.

3. The composition for removing senescent cells according to claim 2, wherein the composition comprises a compound of Table 1 or its pharmaceutically acceptable salt; secreted fluid from cells killed by treatment with a compound of Table 1 or its pharmaceutically acceptable salt; or cells killed by treatment with a compound a compound of Table 1 or its pharmaceutically acceptable salt.

4. The composition for removing senescent cells according to any one of claims 1 to 3, wherein the composition improves, treats or prevents diseases and disorders related to senescent cells.

5. The composition for removing senescent cells according to claim 4, wherein the diseases and disorders related to senescent cells is at least one selected from the group consisting of proliferative diseases and disorders, inflammatory or autoimmune diseases and disorders, neurological diseases and disorders, metabolic diseases and disorders, cardiovascular diseases and disorders, lung diseases and disorders, kidney diseases and disorders, liver diseases and disorders, eye diseases and disorders, skin diseases and disorders, regenerative maintenance-related diseases and disorders, transplantation-related diseases and disorders, fibrosis diseases and disorders, sclerosis diseases and disorders, and β-galactosidase-related diseases and disorders, geriatric diseases and disorders, side effects or aftereffects due to stress and aging-related diseases and disorders resulting therefrom, and rare aging-related diseases and disorders.

6. The composition for removing senescent cells according to claim 4, wherein the diseases and disorders related to senescent cells is cancer, inflammation, fibrosis, sclerosis, frailty, cognitive impairment, sarcopenia, osteoporosis, lipoatrophy, liver damage, kidney damage, skin aging, or side effects of anticancer drugs or obesity.

7. The composition for removing senescent cells according to claim 6, wherein the frailty is decreased activity, decreased endurance, decreased muscle strength, or decreased grip strength.

8. The composition for removing senescent cells according to claim 6, wherein the skin aging is wrinkles, loss of elasticity, hair loss, deterioration of hair skin, freckles, blemishes, skin rash, skin spots, skin discoloration, pigmentation, warts, hives, worsening of pores, or delayed wound healing.

9. The composition for removing senescent cells according to claim 6, wherein the side effects of anticancer drugs or obesity is fatigue, weakness, weight loss, anxiety, depression, lethargy, or fatty liver.

10. A method of removing senescent cells, comprising contacting with senescent cells or administering or applying to a subject in need of improvement, treatment or prevention of diseases and disorders related to senescent cells a therapeutically effective amount of the composition according to any one of claims 1 to 9.

11. The method according to claim 10, wherein the contact with the senescent cells is performed *in vitro* or *ex vivo.*

12. The method according to claim 10, wherein the removal of senescent cells improves, treats or prevents diseases and disorders related to senescent cells.

13. The method according to any one of claims 10 to 12, wherein the method further comprises contacting with senescent cells or administering or applying to a subject in need of improvement, treatment or prevention of diseases and disorders related to senescent cells an immune checkpoint inhibitor.

14. The method according to claim 13, wherein the immune checkpoint inhibitor is any one or more of a CTLA4 inhibitor, a PD-1 inhibitor, a PD-L1 inhibitor, and a PD-L2 inhibitor.

15. A kit or preparation for removing senescent cells, comprising
a composition according to any one of claims 1 to 9, and
an immune checkpoint inhibitor.
